(19) Europäisches Patentamt / European Patent Office / Office européen des brevets

(11) **EP 3 052 933 B1**

(12) **EUROPEAN PATENT SPECIFICATION**

(45) Date of publication and mention of the grant of the patent:
**04.09.2019 Bulletin 2019/36**

(21) Application number: **14850341.0**

(22) Date of filing: **02.10.2014**

(51) Int Cl.:
***G01N 33/48*** *(2006.01)*

(86) International application number:
**PCT/IL2014/050870**

(87) International publication number:
**WO 2015/049688 (09.04.2015 Gazette 2015/14)**

(54) **PATIENT-SPECIFIC IMMUNOTHERAPY FOR TREATING HETEROGENEOUS TUMORS**

PATIENTENSPEZIFISCHE IMMUNTHERAPIE ZUR BEHANDLUNG HETEROGENER TUMORE

IMMUNOTHÉRAPIE SPÉCIFIQUE DU PATIENT POUR LE TRAITEMENT DE TUMEURS HÉTÉROGÈNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2013 US 201361885511 P**

(43) Date of publication of application:
**10.08.2016 Bulletin 2016/32**

(73) Proprietor: **Suri Technologies Ltd.**
**4593000 Ramot-HaShavim (IL)**

(72) Inventors:
• **SARIEL, Aviram**
**45930 Ramot-HaShavim (IL)**
• **HOCHMAN, Ilan**
**44813 Oranit (IL)**

(74) Representative: **Mathys & Squire**
**The Shard**
**32 London Bridge Street**
**London SE1 9SG (GB)**

(56) References cited:
WO-A1-2008/117226     WO-A1-2014/135669
WO-A2-02/054171       WO-A2-2013/011479
US-A1- 2003 097 220   US-A1- 2007 111 257
US-A1- 2009 117 111   US-A1- 2011 130 850
US-A1- 2011 300 156

• M. F. OCHS ET AL: "Detection of Treatment-Induced Changes in Signaling Pathways in Gastrointestinal Stromal Tumors Using Transcriptomic Data", CANCER RESEARCH, vol. 69, no. 23, 1 December 2009 (2009-12-01), pages 9125-9132, XP055039908, ISSN: 0008-5472, DOI: 10.1158/0008-5472.CAN-09-1709
• Jian-Hua Yu ET AL: "Individualized leukemia cell-population profiles in common B-cell acute lymphoblastic leukemia patients", AIZHENG - CHINESE JOURNAL OF CANCER, vol. 32, no. 4, 5 April 2013 (2013-04-05), pages 213-223, XP55433528, CN ISSN: 1000-467X, DOI: 10.5732/cjc.012.10041

## Description

FIELD AND BACKGROUND OF THE INVENTION

The present invention, in some embodiments thereof, relates to the field of cancer therapy, and more particularly, to patient-specific immune therapy for cancer.

The immune system comprises two distinct portions: the innate system and the adaptive system. The innate system includes the cells and mechanisms that provide the host with an immediate generic defense against infections. The adaptive system is activated by the innate system, and composed of highly specialized cells that provide the host with the ability to recognize specific pathogens, and retain or memorize this critical information for future attacks, mainly through the use of memory B and T cells.

B cells are lymphocytes involved in the production of antibodies (the humoral immunity). Monoclonal antibodies (mAbs) specific to tumor cell antigens are potentially useful therapeutics; either as targeting agents, or as inhibitors of critical biological interactions. Cytotoxic T cells are a sub-group of T lymphocyte that recognize and attack virus infected cells or tumor cells by direct cell-cell interaction and destroy them. The *ex vivo* expansion of these cells is the basis of individual T cell therapy.

Attempts at harnessing the immune system to eliminate tumors have been described to include vaccination to increase the frequency of tumor specific T-cells, or adoptive transfer of effector T cells to promote tumor regression (Dudley et al., 2003; Finn *et al.*, 2003). However, despite considerable success in preclinical studies, transfer of immunotherapy to clinical practice has encountered difficulties in achieving success.

Conventionally, therapeutic antibodies are developed against very specific targets characteristic to the common denominators of a wide range of malignant cells. Those denominators are spread over time and across the population, thus obscuring details of the specific illness event. For example, herceptin is a humanized murine monoclonal Ab that was approved as a single agent for the treatment of patients with metastatic breast cancer whose tumors over-express the HER2 protein and who have received one or more chemotherapy regimens for their metastatic disease. Also for example, rituximab is a humanized monoclonal antibody that selectively binds to CD20, a protein found on the surface of normal and malignant B cells, and used to reduce the numbers of circulating B cells in patients who have B cell non-Hodgkin's lymphoma.

Failure points of conventional immune-therapeutic treatment derived from at least two issues. First, person-to-person tumor polymorphism results in a varied antibody sensitivity and specificity that contributes to reduced therapeutic efficiency. Second, by targeting a single tumor antigen, cancer cells with other antigen variants are selectively preferred. Most cancers are not caused by a constant genetic mutation, but by a series of genetic variants, either qualitatively or quantitatively different from their normal counterparts.

Isolation of mAbs that recognize tumor cell-specific antigens is typically performed by mouse immunization with a target human cell line, followed by screening of numerous hybridomas. Although this process generates cell-reactive antibodies, it is suboptimal for the discovery of tissue or disease-specific human antigens, because many widely expressed human antigens are recognized as foreign by the mouse immune system. The human immune repertoire is likely to be distinct from that of the mouse; also the human immune system is tolerant to most normal human antigens. For this reason, it has been suggested that disease-specific cell surface antigens can be identified more readily by screening with human mAbs generated *in vivo.* Previous studies have demonstrated an immune response in cancer patients, and tumor reactive mAbs have been identified by the isolation of mature B-cells from blood serum, tumor-draining lymph nodes, and the tumor tissue itself (Aoki *et al.*, 2005; Imahayashi *et al.*, 2000; Rothe *et al.*, 2004; Brandlein *et al.*, 2002).

Such studies show that cancer patients naturally develop immune responses directed against antigens expressed by their tumors and yet, the cancers continue to grow. A reason for this stems from the tumor microenvironmental ability to restrict the effectiveness of anti-tumor responses by displaying a variety of immunosuppressive strategies (Drake *et al.*, 2006). There are many mechanisms by which tumors evade immune destruction; factors which should be overcome to generate a therapeutic tumor immunity (Rabinovich *et al.*, 2007; Disis *et al.*, 2005).

A number of studies were directed to the isolation and use of natural human antibodies for experimental, diagnostic and therapeutic purposes, including antibodies isolated from cancer patients (Vollmers and Brändlein, 2002; Vollmers and Brändlein, 2007; Vollmers and Brändlein, 2008; Vollmers and Brändlein, 2009; Schwartz-Albiez *et al.*, 2008, Schwartz-Albiez *et al.*, 2009). Isolated antibodies were used to identify tumor-derived antigens (targets) that would later become key in the development of new generalized anti-cancer drugs, and were characterized as belonging to the IgM class and being directed to carbohydrates on post-translationally modified cell surface receptors.

EP 0222360, assigned to Biotherapeutics Inc., is directed to a method of producing a patient-specific cytotoxic reagent for the treatment of a malignant tumor, comprising: processing and expanding cells from the tumor to provide a tumor cell preparation, immunizing B-lymphocytes against said tumor cell preparation to produce antibodies selective for the tumor cells, fusing said immune B-lymphocytes with fusion partner cells to form a plurality of hybridomas, testing each of said plurality of hybridomas for production of an antibody selective for said tumor cells, cloning a plurality of said hybridomas to isolate those which generate said selective antibodies, combining said monoclonal antibodies to produce a monoclonal antibody cocktail that will react with substantially all of said tumor cells, and linking each said monoclonal antibody in said monoclonal antibody cocktail with a substance which is toxic to the tumor cells to generate an immunoconjugate reagent for treating the tumor. A method of biologically treating a cancerous tumor further comprising the steps of testing said immunoconjugate solution for localization and effectiveness against the tumor cells and applying said immunoconjugate solution in therapeutic doses against the tumor is also disclosed.

Attempts of Biotherapeutics Inc. to produce patient specific immunotherapy were described as unsuccessful, as patients selected for therapy either passed away and/or did not manifest a reduction in tumor size or other significant clinical responses (McIntosh, 1990, Marantz Henig, 1986). It was further disclosed that this treatment may not be suitable for most cancer patients afflicted with aggressive cancer due to the length of the process, reported to take about a year for developing and producing the antibodies (Marantz Henig, 1986).

U.S. Patent No. 6,180,357, assigned to Arius Research Inc., is directed to a method for the selection of individually customized anti-cancer antibodies which are useful in treating a cancerous disease consisting of: obtaining a cancerous tissue sample from an individual patient; manufacturing antibodies directed against cells of said tissue sample; directly subjecting said manufactured antibodies to a cytotoxicity assay designed to identify a subset of antibodies which express an enhanced degree of cytotoxicity directed toward cancerous cells while simultaneously being relatively nontoxic to non-cancerous cells of said individual's tissue sample; whereby said subset of antibodies defines a group of individually customized anti-cancer antibodies characterized as being cytotoxic to said cancerous cells and essentially benign toward normal cells.

International Patent Publication No. WO 2013/011479 A2 relates to methods for inferring activity of one or more cellular signaling pathway(s) in tissue of a medical subject based at least on the expression level(s) of one or more target gene(s) of the cellular signally pathway(s) measured in an extracted sample of the tissue of the medical subject. This is discussed in the context of various cancers being known to be associated with specific combinations of genomic mutations/variations and/or high or low expression levels for specific genes, which play a role in growth and evolution of cncer, *e.g.,* cell proliferation and metastasis.

International Patent Publication No. WO 2014/13569 A1 relates to assessing status of a solid tumor cancer in a subject involving detection of tumor-associated mutations, and identifying a candidate non-small cancer cell lung cancer patient for a targeted drug therapy. Correlation between mutation status and treatment outcome is discussed.A publication entitled "Individualized leukemia cell-population profiles in common B-cell acute lymphoblastic leukemia patients" authored by Jian-Hua Yu et al. (Chinese Journal of Cancer, vol. 32, no. 4, 5 April 2013 pp. 213-223) describes a protocol to explore the immunophenotypic profiles of common acute lymphoblastic leukemia (ALL) based on the expression levels of the antigens associated with B lymphoid development; concluding that the profiles of patient specific composite leukemia cell populations could provide detailed information helpful for the diagnosis, therapeutic monitoring, and individualized therapies for common ALL.

SUMMARY OF THE INVENTION

There is provided, in accordance with some embodiments of the present invention, a method of adapting a pharmaceutical composition comprising immunocompound pharmaceutical components for the treatment of a cancerous disease in a patient in need thereof, comprising: determining a model including: (1) a current disease state including as factors determinations of cell count prevalences of a plurality of clonal cell types in the cancerous disease of the patient, and (2) immunocompound pharmaceutical components having treatment effects on the disease state based on the measured partitioning of the immunocompound pharmaceutical components among the clonal cell types; calculating a composition of said immunocompound pharmaceutical components acting on the current disease state according to their measured partitioning to produce a target disease state model including as factors determinations of cell count prevalences of the plurality of clonal cell types; formulating a treatment composition for said patient based on the calculated composition; receiving results providing information about cell count prevalences of the plurality of clonal cell types in the patient after the treatment composition was administered; producing a new disease state model based on the received results; adjusting the model based on differences between the target disease state model and the new disease state model of the patient; and repeating said calculating and formulating.

In some embodiments, the immunocompound pharmaceutical components comprise immunocompounds; and wherein their measured partitioning is determined based on their binding activities associated with binding to respective clonal cell types of the plurality of clonal cell types.

In some embodiments, the repeating comprises repeating the calculating and formulating for at least four iterations of formulation and subsequent treatment with the formulation.

In some embodiments, the repeating comprises repeating the adjusting.

In some embodiments, the calculating takes into account a tradeoff between a change in patient health state and a change in disease state caused by treatment with the composition.

In some embodiments, the current disease state is stored as a vector, and wherein the calculating comprises matrix-vector or matrix-matrix multiplications on the stored vector.

In some embodiments, the calculating uses a mimo model with multiple inputs generating multiple interdependent outputs.

In some embodiments, the calculating comprises search a state-space for a state at least approaching the target state.

In some embodiments, the adjusting comprises at least one of the group consisting of: adjusting a model of the interaction between disease and treatment, and changing a disease state portion of the model.

In some embodiments, the method comprises determining the susceptibility of the cancer; the determining of susceptibility comprising assaying identified preparations of the immunocompounds to select preparations of immunocompounds that localize to the clonal cell types in a cancerous tumor of the patient in vivo.

In some embodiments, the formulating comprises providing a pharmaceutical composition comprising identified immunocompound preparations at predetermined doses, wherein the dose ratio of the different preparations in the composition is correlated to relative vital states of the clonal cell types.

In some embodiments, the model comprises an adaptively controlled model wherein: the calculating a composition comprises calculating control to exercise on the adaptively controlled model to produce a state at least approaching the target disease state, based on which control component the calculated composition is determined; the adjusting the model based on differences between the target disease state model and the new disease state model of the patient adjusts modelling of the interaction between disease and treatment; the adjusting the model further comprises adjusting the current disease state of the model to correspond to the new disease state of the patient; and the repeating of the calculating calculates a new composition which is different from the composition based on the adjusting of the model.

In some embodiments, the model comprises an adaptive linearly controlled model, wherein the calculating a composition comprises calculating a linear combination of the modeled treatment effects of the immunocompound pharmaceutical components on the disease state.

In some embodiments, the results providing information identify a distribution of a clonal cell type in the patient from diagnostic images of a distribution of at least one of the immunocompounds in vivo.

There is provided, in accordance with some embodiments of the present invention. a system consisting of a computer, configured by programming to implement processing to perform the determining, calculating, receiving, producing, and adjusting.

There is provided, in accordance with some embodiments of the present invention, a method of adapting a pharmaceutical composition comprising immunocompound pharmaceutical components for the treatment of a cancerous disease in a patient in need thereof, comprising: receiving results providing information about cell count prevalences of a plurality of clonal cell types in the patient after a pharmaceutical composition was administered; producing a disease state model based on the received results, wherein the disease state includes as factors determinations of cell count prevalences of the plurality of clonal cell types in the cancerous disease of the patient; adjusting a model including: (1) the disease state, and (2) immunocompound pharmaceutical components having treatment effects on the disease state based on the measured partitioning of the immunocompound pharmaceutical components among the clonal cell types, wherein the model is adjusted based on differences between disease states of the patient prior to and after administration of the pharmaceutical composition;calculating a composition of said immunocompound pharmaceutical components acting on the adjusted disease state model according to their measured partitioning to produce a target disease state model; and formulating a treatment composition for said patient based on the calculated composition.

According to principles of some embodiments of the present disclosure, a repertoire of the patient's tumor specific antibodies is identified, and antibodies are expanded and mass-produced to administer back into the same patient.

In some embodiments of the present disclosure, therapeutic modalities are provided, in which the composition and dosage of the administered anti-tumor antibodies are determined and dynamically adjusted to the individual patient's condition, disease and treatment progression. To potential advantage, embodiments of the present disclosure include the use of mathematical algorithms and/or computer programs to design and adjust the nature and dosage of each component of the administered treatment, thus allowing effective patient-specific and disease-specific cancer treatment targeting the various tumor clones afflicting the patient at each stage of the disease.

In some embodiments, a therapeutic method is provided for use clinically and/or on an industrial scale to prepare an appropriate cancer treatment to a patient within a shortened period of time and with increased tumor specificity and selectivity. Particular embodiments of the present disclosure include methods for providing clones of immortalized antibody-producing cells secreting antibodies suitable for the preparation of medicaments that are usable in the methods of the present disclosure.

There is provided in accordance with some embodiments of the present disclosure a method of adaptively producing a pharmaceutical composition for the treatment of a disease in a patient in need thereof, comprising:

determining a model including: a current disease state corresponding to the disease of the patient, and pharmaceutical components having treatment effects on the disease state based on their measured partitioning among factors of the disease state;

calculating a composition of said pharmaceutical components acting on the current disease state to produce a target disease state;

formulating a treatment composition for said patient based on the calculated composition; and

adjusting the model based on differences between the target disease state and a new disease state of the patient after administration of said formulation; and

repeating said calculating and formulating.

In some exemplary embodiments of the present disclosure, said repeating comprises repeating said calculating and formulating for at least four iterations of formulation and subsequent treatment with the formulation.

In some exemplary embodiments of the present disclosure, said adjusting comprises improving a biological correctness of a reaction of said disease to said components.

In some exemplary embodiments of the present disclosure, said disease state includes a plurality of components and wherein an accuracy of said model with respect to a reaction of disease components to pharmaceutical components is at least 20% inaccurate.

In some exemplary embodiments of the present disclosure, said adjusting comprises adjusting a number of disease components in said model.

In some exemplary embodiments of the present disclosure, said repeating comprises repeating said adjusting.

In some exemplary embodiments of the present disclosure, the method comprises providing said treatment composition to said patient.

In some exemplary embodiments of the present disclosure, said calculating comprises calculating a therapy component other than a formulation of a composition.

In some exemplary embodiments of the present disclosure, said calculating takes into account a change in patient health caused by treatment with said composition.

In some exemplary embodiments of the present disclosure, the method comprises selecting by a user a tradeoff between a health state and a disease state of said patient in response to treatment and wherein said calculating uses said selected tradeoff.

In some exemplary embodiments of the present disclosure, said calculating takes into account an interaction between patient state, disease state and said treatment.

In some exemplary embodiments of the present disclosure, said calculating comprises matrix-vector or matrix-matrix multiplications.

In some exemplary embodiments of the present disclosure, said disease state is stored as a vector.

In some exemplary embodiments of the present disclosure, said calculating uses a mimo model with multiple inputs generating multiple interdependent outputs.

In some exemplary embodiments of the present disclosure, said calculating comprises predicting a future state of said disease after a reaction thereof to said treatment in addition to cell death or inhibition caused by said treatment.

In some exemplary embodiments of the present disclosure, said calculating comprises search a state-space for a state at least approaching said target state.

In some exemplary embodiments of the present disclosure, said adjusting comprises adjusting a model of the interaction between disease and treatment.

In some exemplary embodiments of the present disclosure, said adjusting comprises changing a disease state portion of the model.

In some exemplary embodiments of the present disclosure, said pharmaceutical components include immunocompounds for treatment of cancer. Optionally, said model assumes a plurality of different cell type populations in said cancer, with different susceptibilities to treatment. Optionally or alternatively, said model includes fewer than 20 different populations types. Optionally or alternatively, said model includes at least 4 different populations types.

In some exemplary embodiments of the present disclosure, said immunocompounds have binding selectivities corresponding to binding selectivities of antibodies produced by B cells obtained from said patient. Optionally, said immunocompounds are produced by a plurality of immortal antibody-producing cell clones. Optionally, the method comprises screening the plurality of antibody-producing cell clones to identify clones producing antibodies which selectively bind tumor cells obtained from the patient while not substantially binding non-malignant human cells or tissue in a selective manner. Optionally, the method comprises combining the individual antibody-producing cell clones to obtain pooled clones, each pooled clone consisting of one or more individual antibody-producing cell clones that are substantially similar to the antibody-producing cells within the pooled clone with respect to at least one parameter selected from the group consisting of genetic homology in the immunoglobulin variable domains, and antigen specificity of the antibodies secreted from said antibody-producing cell clones. Optionally, each said individual antibody-producing cell clone is substantially similar to the antibody-producing cells within the pooled clone with respect to at least one parameter selected from the group consisting of binding sensitivity and binding selectivity of the antibodies secreted from said antibody-producing cell clones to their respective antigens. Optionally or alternatively, each said individual antibody-producing cell clone is substantially similar to the antibody-producing cells within the pooled clone with respect to at least one parameter selected from the group consisting of the isotype of the antibodies secreted from said antibody-producing cell clones, the growth rate of the antibody-producing cell clones and the antibody secretion rate of said antibody-producing cell clones.

In some exemplary embodiments of the present disclosure, the method comprises:

producing antibodies from each pooled clone separately, to obtain a plurality of antibody preparations corresponding to the plurality of pooled clones; and
assaying the plurality of antibody preparations to identify preparations of antibodies that do not substantially bind non-malignant human cells or tissue in a selective manner while selectively binding to tumor cells obtained from said patient.

In some exemplary embodiments of the present disclosure, the method comprises determining the susceptibility of said cancer comprises assaying identified preparations of said immunocompounds to select preparations of immunocompounds that localize to the tumor of said patient in vivo. Optionally, said determining of susceptibility comprises determining the relative tumor-localizing fraction of said identified preparations of said immunocompounds. Optionally or alternatively, said determining of a current vital state comprises evaluating the relative amount and optionally malignancy of individual tumor clones in said patient corresponding to said identified preparations of immunocompounds.

In some exemplary embodiments of the present disclosure, said selecting comprises providing a pharmaceutical composition comprising identified immunocompound preparations at predetermined doses, wherein the dose ratio of the different preparations in the composition is correlated to relative vital states of said cell type populations. Optionally, said cell type populations comprise populations of individual tumor clones in said patient. Optionally, said vital states are relative numbers of said individual tumor clones. Optionally, said vital states are relative malignancies of each tumor clone.

In an exemplary embodiment of the present disclosure, the dose ratio is inversely correlated to the relative tumor-localizing fraction of each immunocompound preparation.

In some exemplary embodiments of the present disclosure, said cell type populations comprise cancerous cell types.

In some exemplary embodiments of the present disclosure, said cell type populations comprise non-cancerous cell types found at the site of a cancer tumor.

In some exemplary embodiments of the present disclosure, said selecting a composition comprises determining a dosage ratio among a plurality of said immuno compounds.

In some exemplary embodiments of the present disclosure, the target disease state is selected to prevent a vital state of a first cell type of said plurality of cell types from being reduced below a determined level relative to a vital state of a second cell type of said plurality of cell types.

In some exemplary embodiments of the present disclosure, the immunocompounds comprise antibodies conjugated to an anti-cancer substance prior to administration to said patient.

There is provided in accordance with some embodiments of the present disclosure a method of correcting a treatment composition to a treatment target comprising a plurality of cell types, comprising:

determining that treatment of a first cell type of the treatment target is impaired when a second cell type of the treatment target reduces;
adjusting an component of said treatment composition to reduce the reduction of said second cell type, relative to said first cell type. Optionally, the method comprises administering the composition to the patient before a repeating of said determining. Optionally or alternatively, said determining and said adjusting comprises determining for more than one type of first cell type and/or more than one type of second cell type.

There is provided in accordance with some embodiments of the present disclosure a pharmaceutical composition produced by the methods as described herein.

There is provided in accordance with some embodiments of the present disclosure a computer readable medium having stored thereon a treatment protocol including multiple administrations each using a pharmaceutical composition produced as described herein.

There is provided in accordance with some embodiments of the present disclosure apparatus configured to perform said calculating as described herein, for use in carrying out any of the methods as described herein.

There is provided in accordance with some embodiments of the present disclosure a method of adaptively producing a pharmaceutical composition for the treatment of cancer in a patient in need thereof comprising:

determining, for each of a plurality of cell type populations of the patient:

a current vital state, and
a susceptibility to each of a plurality of immunocompounds derived from the immune system of the patient;

selecting a composition of the immunocompounds calculated, based on said determining, to change the current vital states to corresponding target vital states, upon administration to the patient; and
iteratively:

administering the selected composition to the patient,
re-determining the vital states,

adjusting the determined susceptibilities based on changes of the vital states after the administering, and
re-selecting the composition of immunocompounds, the re-selecting comprising calculating the composition-based on said re-determining and adjusting-to change the re-determined vital states to corresponding target vital states, upon administration to the patient. Optionally, said plurality is between 4 and 10 cell types. Optionally or alternatively, said re-selection comprises estimating an effect of changing cell type population properties on a further treatment and/or a future health or disease state.

There is provided in accordance with some embodiments of the present disclosure a method of adaptively producing a pharmaceutical composition for the treatment of cancer in a patient in need thereof, comprising:

adjusting predictions of a dynamic model based on one or both of a changing health state and a changing disease state of the patient;
wherein the predictions of the model are in the form of a pharmaceutical composition description, and one or both of a new health state and a new disease state that the pharmaceutical composition produces upon administration to the patient; and
wherein the model is also adjusted as though the described pharmaceutical composition is administered to produce one or both of said changing health state and said changing disease state.

There is provided in accordance with some embodiments of the present disclosure a method of adaptively augmenting antibody production by an immune system, comprising:

determining a modification in the availability of antibodies specific for an antigen;
supplying antibodies specific to said antigen, based on said determining. Optionally, the method comprises supplying antibodies for said antigen in an amount of at least 100 times said availability in response to said determining. Optionally or alternatively, the method comprises supplying antibodies for a plurality of antigens in response to said determining. Optionally or alternatively, said supplying comprises compensating for an increased reaction time of said immune system.

There is provided in accordance with some embodiments of the present disclosure a method for producing a pharmaceutical composition for the treatment of cancer in a patient in need thereof, comprising:

(a) obtaining a sample of tumor cells from the patient;
(b) obtaining B-cells from said patient;
(c) providing a plurality of immortal antibody-producing cell clones, wherein the produced antibodies have binding selectivities corresponding to binding selectivities of antibodies produced by the B cells obtained from said patient;
(d) screening the plurality of antibody-producing cell clones to identify clones producing antibodies which selectively bind the tumor cells obtained from said patient while not substantially binding non-malignant human cells or tissue in a selective manner;
(e) combining the individual antibody-producing cell clones identified in (d) to obtain pooled clones, each pooled clone consisting of one or more individual antibody-producing cell clones that are substantially similar to the antibody-producing cells within the pooled clone with respect to at least one of the parameters of group (i) and optionally further with respect to at least one of the parameters of groups (ii) and (iii);
(f) producing antibodies from each pooled clone separately, to obtain a plurality of antibody preparations corresponding to the plurality of pooled clones;

(g) assaying the plurality of antibody preparations to identify preparations of antibodies that do not substantially bind non-malignant human cells or tissue in a selective manner while selectively binding to tumor cells obtained from said patient;

(h) assaying the antibody preparations identified in (g) to select preparations of antibodies that localize to the tumor in said patient in vivo, and optionally to determine the relative tumor-localizing fraction of each antibody preparation;

(i) evaluating the relative amount and optionally malignancy of individual tumor clones in said patient corresponding to each antibody preparation; and

(j) providing a pharmaceutical composition comprising the antibody preparations identified in (h) at predetermined doses, wherein the dose ratio of the different preparations in the composition is correlated to the relative amounts of the individual tumor clones in said patient, optionally further correlated to the relative malignancy of each tumor clone, and optionally further inversely correlated to the relative tumor-localizing fraction of each antibody preparation;

wherein the antibodies are optionally conjugated to an anti-cancer substance prior to administration to said patient, and wherein:

the parameters of group (i) are selected from the group consisting of: genetic homology in the immunoglobulin variable domains, and antigen specificity of the antibodies secreted from said antibody-producing cell clones,

the parameters of group (ii) are selected from the group consisting of: binding sensitivity and binding selectivity of the antibodies secreted from said antibody-producing cell clones to their respective antigens, and

the parameters of group (iii) are selected from the group consisting of: the isotype of the antibodies secreted from said antibody-producing cell clones, the growth rate of the antibody-producing cell clones and the antibody secretion rate of said antibody-producing cell clones. Optionally, said immortal antibody-producing cells are hybridoma cells. Optionally or alternatively, (c) is effected by fusing the B-cells obtained in (b) with immortal cells to produce a plurality of hybridomas.

In some exemplary embodiments of the present disclosure, the duration of (a) to (j) is up to about three months.

In some exemplary embodiments of the present disclosure, the B-cells obtained in (b) are incubated with the tumor cells obtained in (a) prior to (c), under conditions enabling said B-cells to be immunologically sensitized to said tumor cells.

In some exemplary embodiments of the present disclosure, the tumor cells are not propagated in culture for more than one week prior to (d) and/or (g).

In an exemplary embodiment of the present disclosure, there is provided method for treating cancer in a patient in need thereof, comprising producing a pharmaceutical composition for the treatment of cancer as described herein, and further comprising:

(k) administering said composition to said patient;

(1) determining the amount and optionally malignancy of said individual tumor clones in said patient; and

(m) repeating at least (k) to (1) at predetermined intervals, to adjust the administered dose of each antibody preparation according to a change in the amount and/or malignancy of said individual tumor clones. Optionally, dose adjustment is performed using an adaptive control algorithm. Optionally or alternatively, the predetermined intervals are between 2 to 5 weeks. Optionally or alternatively, (1) is performed subsequently to (k). Optionally or alternatively, (1) is performed 2 to 5 weeks subsequently to (k).

There is provided in accordance with some embodiments of the present disclosure a method of providing immortal antibody-producing cell clones secreting antibodies suitable for the preparation of a medicament for the treatment of cancer, the method comprising:

(a) screening a plurality of immortal antibody-producing cell clones to identify individual antibody-producing cell clones producing antibodies which selectively bind tumor cells while not substantially binding non-malignant human cells or tissue in a selective manner;

(b) combining the individual antibody-producing cell clones identified in (a) to obtain pooled clones, each pooled clone consisting of one or more individual antibody-producing cell clones that are substantially similar to the antibody-producing cells within the pooled clone with respect to at least one of the parameters of group (i), optionally further with respect to at least one of the parameters of group (ii) and optionally further with respect to at least one of the parameters of group (iii). Optionally, the individual antibody-producing cell clones within each pooled clone are not substantially similar to antibody-producing cells of other pooled clones with respect to the parameters of group (i) and optionally further with respect to at least one of the parameters of group (ii). Optionally or alternatively, the individual immortal antibody-producing cell clones within each pooled clone are not substantially similar to antibody-producing cells of other pooled clones further with respect to the parameters of group (iii). Optionally or alternatively, said immortal antibody-producing cells are hybridoma cells.

There is provided in accordance with some embodiments of the present disclosure a method for the preparation of a pharmaceutical composition for the treatment of cancer, comprising providing immortal antibody-producing cell clones according to claim 68, and further comprising:

(c) producing antibodies from each pooled clone separately, to obtain a plurality of antibody preparations corresponding to the plurality of pooled clones; and
(d) providing a pharmaceutical composition comprising the antibody preparations at predetermined doses, wherein the dose ratio of the different preparations in the composition corresponds to the relative proportions of tumor clones corresponding to each antibody preparation in a patient in need thereof, and optionally further to the relative malignancy of each tumor clone.

There is provided in accordance with some embodiments of the present disclosure a pharmaceutical composition produced by any of the methods described herein.
There is provided in accordance with some embodiments of the present disclosure computer readable media having stored thereon a treatment protocol for using a pharmaceutical composition produced by any of the methods described herein.
According to an aspect of some embodiments of the present disclosure, there is provided a method for producing a pharmaceutical composition for the treatment of cancer in a patient in need thereof, comprising: (a) obtaining a sample of tumor cells from the patient; (b) obtaining B-cells from the patient; (c) providing a plurality of immortal antibody-producing cell clones, wherein the produced antibodies have binding selectivities corresponding to binding selectivities of antibodies produced by the B cells obtained from the patient; (d) screening the plurality of antibody-producing cell clones to identify clones producing antibodies which selectively bind the tumor cells obtained from the patient while not substantially binding non-malignant human cells or tissue in a selective manner; (e) combining the individual antibody-producing cell clones identified in (d) to obtain pooled clones, each pooled clone consisting of one or more individual antibody-producing cell clones that are substantially similar to the antibody-producing cells within the pooled clone with respect to at least one of the parameters of group (i) and optionally further with respect to at least one of the parameters of groups (ii) and (iii); (f) producing antibodies from each pooled clone separately, to obtain a plurality of antibody preparations corresponding to the plurality of pooled clones; (g) assaying the plurality of antibody preparations to identify preparations of antibodies that do not substantially bind non-malignant human cells or tissue in a selective manner while selectively binding to tumor cells obtained from the patient; (h) assaying the antibody preparations identified in (g) to select preparations of antibodies that localize to the tumor in the patient in vivo, and optionally to determine the relative tumor-localizing fraction of each antibody preparation; (i) evaluating the relative amount and optionally malignancy of individual tumor clones in the patient corresponding to each antibody preparation; and (j) providing a pharmaceutical composition comprising the antibody preparations identified in (h) at predetermined doses, wherein the dose ratio of the different preparations in the composition is correlated to the relative amounts of the individual tumor clones in the patient, optionally further correlated to the relative malignancy of each tumor clone, and optionally further inversely correlated to the relative tumor-localizing fraction of each antibody preparation; wherein the antibodies are optionally conjugated to an anti-cancer substance prior to administration to the patient, and wherein: the parameters of group (i) are selected from the group consisting of: genetic homology in the immunoglobulin variable domains, and antigen specificity of the antibodies secreted from the antibody-producing cell clones, the parameters of group (ii) are selected from the group consisting of: binding sensitivity and binding selectivity of the antibodies secreted from the antibody-producing cell clones to their respective antigens, and the parameters of group (iii) are selected from the group consisting of: the isotype of the antibodies secreted from the antibody-producing cell clones, the growth rate of the antibody-producing cell clones and the antibody secretion rate of the antibody-producing cell clones.
According to some embodiments of the present disclosure, the immortal antibody-producing cells are hybridoma cells.
According to some embodiments of the present disclosure, step (c) is effected by fusing the B-cells obtained in (b) with immortal cells to produce a plurality of hybridomas.
According to some embodiments of the present disclosure, the duration of steps (a) to (j) is up to about three months.
According to some embodiments of the present disclosure, the B-cells obtained in (b) are incubated with the tumor cells obtained in (a) prior to step (c), under conditions enabling the B-cells to be immunologically sensitized to the tumor cells.
According to some embodiments of the present disclosure, the tumor cells are not propagated in culture for more than one week prior to steps (d) and/or (g).

According to an aspect of some embodiments of the present disclosure, there is provided a method for treating cancer in a patient in need thereof, comprising producing a pharmaceutical composition for the treatment of cancer as defined in claim 1, and further comprising the steps of: (k) administering the composition to the patient; (1) determining the amount and optionally malignancy of the individual tumor clones in the patient; and (m) repeating at least steps (k) to (1) at predetermined intervals, to adjust the administered dose of each antibody preparation according to a change in the amount and/or malignancy of the individual tumor clones.

According to some embodiments of the present disclosure, dose adjustment is performed using an adaptive control algorithm.

According to some embodiments of the present disclosure, the predetermined intervals are between 2 to 5 weeks.

According to some embodiments of the present disclosure, step (1) is performed subsequently to step (k).

According to some embodiments of the present disclosure, step (1) is performed 2 to 5 weeks subsequently to step (k).

According to an aspect of some embodiments of the present disclosure, there is provided a method of providing immortal antibody-producing cell clones secreting antibodies suitable for the preparation of a medicament for the treatment of cancer, the method comprising: (a) screening a plurality of immortal antibody-producing cell clones to identify individual antibody-producing cell clones producing antibodies which selectively bind tumor cells while not substantially binding non-malignant human cells or tissue in a selective manner; (b) combining the individual antibody-producing cell clones identified in (a) to obtain pooled clones, each pooled clone consisting of one or more individual antibody-producing cell clones that are substantially similar to the antibody-producing cells within the pooled clone with respect to at least one of the parameters of group (i), optionally further with respect to at least one of the parameters of group (ii) and optionally further with respect to at least one of the parameters of group (iii).

According to some embodiments of the present disclosure, the individual antibody-producing cell clones within each pooled clone are not substantially similar to antibody-producing cells of other pooled clones with respect to the parameters of group (i) and optionally further with respect to at least one of the parameters of group (ii).

According to some embodiments of the present disclosure, the individual immortal antibody-producing cell clones within each pooled clone are not substantially similar to antibody-producing cells of other pooled clones further with respect to the parameters of group (iii).

According to some embodiments of the present disclosure, the immortal antibody-producing cells are hybridoma cells.

According to an aspect of some embodiments of the present disclosure, there is provided a method for the preparation of a pharmaceutical composition for the treatment of cancer, comprising providing immortal antibody-producing cell clones according to claim 12, and further comprising: (c) producing antibodies from each pooled clone separately, to obtain a plurality of antibody preparations corresponding to the plurality of pooled clones; and (d) providing a pharmaceutical composition comprising the antibody preparations at predetermined doses, wherein the dose ratio of the different preparations in the composition corresponds to the relative proportions of tumor clones corresponding to each antibody preparation in a patient in need thereof, and optionally further to the relative malignancy of each tumor clone.

According to an aspect of some embodiments of the present disclosure, there is provided a pharmaceutical composition produced by the method and 16.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the present disclosure, exemplary methods and/or materials are described below. In case of conflict, the patent specification, including definitions, will control. In addition, the materials, methods, and examples are illustrative only and are not intended to be necessarily limiting.

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer readable program code embodied thereon. Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system.

In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of the present invention may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The present invention and embodiments thereof are set out in the appended claims.

BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example, and for purposes of illustrative discussion of embodiments of the invention. In this regard, the description taken with the drawings makes apparent to those skilled in the art how embodiments of the invention may be practiced.

In the drawings:

FIG. 1A schematically illustrates cyclic development, tuning, and application of an adaptive, patient-specific treatment regime, according to some exemplary embodiments of the present disclosure;

FIG. 1B schematically illustrates sub-loops within the cyclic development, adjustment, and application of an adaptive, patient-specific treatment regime, according to some exemplary embodiments of the present disclosure;

FIG. 2 schematically relates mathematical terms to corresponding laboratory operations in the initial determination of a treatment composition, according to some exemplary embodiments of the present disclosure;

FIG. 3 schematically illustrates the effects of different branches of a treatment composition on different disease cell lines, support cell types, and/or other patient cells, according to some exemplary embodiments of the present disclosure;

FIGs. 4A-4B schematically relate model term estimates to available laboratory data in the initial determination of a treatment composition, according to some exemplary embodiments of the present disclosure;

FIG. 5 is a schematic flowchart of interaction between data inputs-including treatment effectiveness/adverse effect measurements, disease state, patient performance status-and treatment determination/adjustment, according to some exemplary embodiments of the present disclosure;

FIG. 6 schematically illustrates treatment, disease, and patient events and states during an exemplary time-course of iteratively controlled treatment, according to some exemplary embodiments of the present disclosure;

FIG. 7 schematically illustrates adjustments and additions of treatment components in response to clonal cell-type population prevalence during an exemplary time-course of iteratively controlled treatment, according to some exemplary embodiments of the present disclosure;

FIG. 8 schematically illustrates adjustments of treatment components to take advantage of clonal cell-type mutual suppression during an exemplary time-course of iteratively controlled treatment, according to some exemplary embodiments of the present disclosure; and

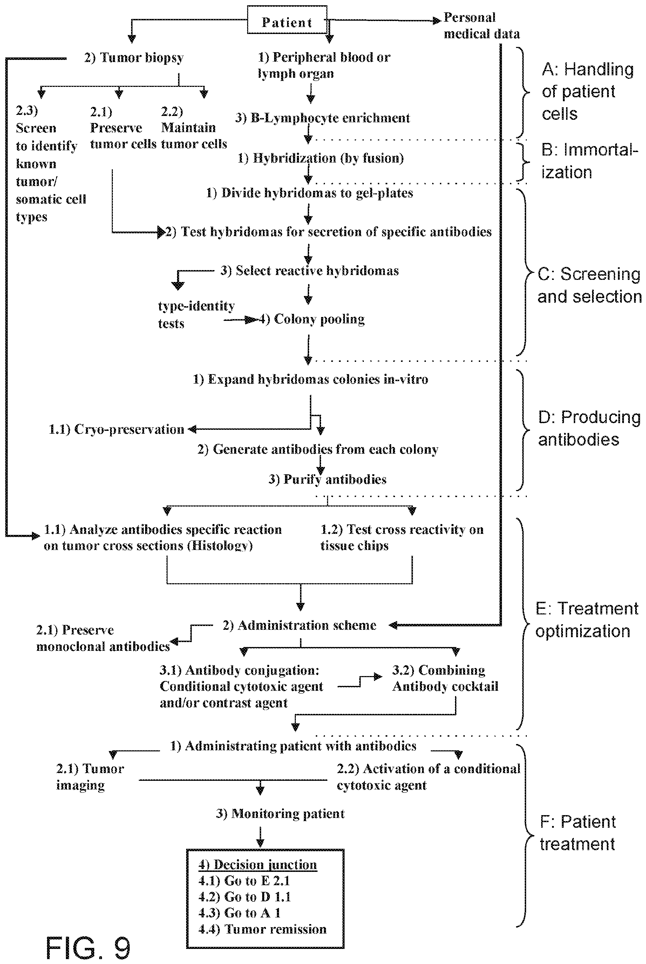

FIG. 9 is a schematic flowchart example of adaptively controlled determination and administration of treatment components comprising antibodies selected for specific binding to cancer cell lines, according to some exemplary embodiments of the present disclosure.

## DESCRIPTION OF SPECIFIC EMBODIMENTS OF THE INVENTION

The present invention, in some embodiments thereof, relates to the field of cancer therapy, and more particularly, to patient-specific immune therapy for cancer.

**Overview**

There apparently remains an unmet medical need for providing immunotherapy for cancer, which is specifically adapted to an individual patient's tumor characteristics and condition in sufficient time to prove effective. In some embodiments of the invention, this need is provided by a dynamic therapy, allowing accurate and efficient treatment readjustment during some or all of the entire course of therapy; for example in the treatment of heterogeneous tumors comprising multiple tumor clones.

A broad aspect of some embodiments of the invention relates to the integration of aspects of disease treatment planning and delivery-including one or more of development, composition, and/or dosage-under control logic (e.g., feedback loop) by which treatment is dynamically tuned. In some embodiments, adjustment is according to the patient-dependent and/or disease-particular effects, specificities, and/or progression of the treatment.

In some embodiments, the effect of dynamic adjustment is to augment and/or replace by external means responsive properties like those of the immune system, to achieve therapeutic results. In some embodiments, elements of the immune system (for example, but not only, patient B cells and/or IgM antibodies) are harnessed and/or modified as part of this augmentation and/or replacement. In some embodiments, the immune system of the same patient that is being treated is extended by the addition of methods and/or devices comprising control logic that allows selective amplification of the immune system's existing specificities. Amplification comprises, for example, increasing the concentration (dosage) of an available antibody specificity, and/or increasing the therapeutic effects associated with that specificity, for example by conjugation of antibody specificity to an additional treatment agent. Optionally, selective amplification allows "forcing" of immunoreactivity which found, but suppressed within the native immune system - for example, by its own normal regulation, and/or due to immunoevasive properties of the disease (cancer, in particular) itself.

It is a particular feature of some embodiments of the invention that the external means includes a controller which calculates a desired immune augmentation.

Potentially, this approach is well suited to the treatment of cancers, and in particular polyclonal cancers. In some embodiments, each clonal line of a polyclonal cancer comprises a distinct treatment target. Optionally, treatment adjustment comprises selecting one or more treatment targets for focused treatment, for treatment at a particular level relative to the target specificity of an available treatment option, and/or for a particular level of treatment relative to other target treatments.

Potentially, augmentation of an immune system allows deliberate control of treatment supply—of antibodies, or of any other treatment, and particularly treatments having a determinable cost/benefit tradeoff-to suit the situation, and particularly to suit the life-threatening exigencies of malignant disease. Such control potentially confers advantages such as the ability to speed treatment production, react more quickly to changes during treatment progression, and/or make informed selection of risk/benefit trade-offs.

In some embodiments, drug specificities (for example, immunospecificities) are tested outside the body of the patient on targets derived from the patient - including cells of the disease (cancerous and/or cancer-supporting), to which targeting is aimed, and/or somatic cells to which targeting is generally to be reduced and/or avoided. *Ex vivo* screening potentially speeds treatment by parallelizing exploratory portions of the treatment program of the patient. Selected patient/disease conditions are duplicated in culture, allowing pre-enactment of scenarios which an actually delivered treatment seeks to duplicate, combine, and/or build on. It is potentially significant that the conditions duplicated are specific to the patient/disease system, and in particular, specific to the current state of that system. This enables, in some embodiments, targeting of epitopes subject to change in an evolving cancer cell population, and/or in the evolution of the cells themselves. The problem of unstable epitopes is potentially met by a dynamically adjusting treatment and/or treatment profile.

Potentially, an *ex vivo* approach to treatment screening allows simultaneous characterization of a wide range of immunospecificities or other treatments. Optionally, this includes screening to characterize interactions among specificities which, in effect, create (for example, by pooling of individual immunospecificities) new specificities. Potentially, this allows the controlled introduction of non-linear interactions among treatment components aimed at particular disease targets, the interactions being chosen in particular for amplification of effects on treatment targets.

The option to dynamically determine treatment specificity, on one hand, and to select non-linear combinations of specificity, on the other, potentially reduces a requirement for the cancer itself to provide one or two "good targets" (and/or for such targets to be found)-a requirement, for example, of stability, very high specificity, and/or target ubiquity. In some embodiments of the invention, a sufficient number of targets for which specific binding activities can be identified-even if the targets change over time and/or under the selective pressures of treatment-potentially allows effective treatment by controlled application of a dynamically adapted spectrum of therapeutic specificities.

It is also a potential advantage, particularly in the case of a weakened patient, for the energy-demanding functions of the immune system to be taken up at least in part by an external system. In some embodiments, a patient's native immune system is itself directly damaged by elements of combination therapy (for example, chemotherapies and radiation therapies); it is a potential advantage to artificially supplement these damaged capabilities, and/or change the acceptable balance of treatment side-effects and benefits.

A broad aspect of some embodiments of the invention relates to methods and systems for calculating the composition of a multicomponent treatment.

In some embodiments, *ex vivo* determinations of specificities (of treatment compounds to targets and/or non-targets), of sensitivities (of cell types, and/or of the patient's whole organism), and/or of interactions among them comprise a set of data from which calculated selection is made in order to provide a treatment potentially likely to be effective.

In some embodiments, calculations are performed based on a model of the disease/patient system. In some embodiments, modeling comprises assignment of weights, functions, and/or dynamics to model terms which explicitly correspond to elements of the actual disease/patient system. For example, dynamics of the cancer (for example, its tendency to grow), of the patient (for example, the ability to fight cancer growth), the state of the cancer (for example, its distribution of cell types), and/or the effects of treatment components (for example, which cell types they are specific for, in what concentrations, and with what delivery dynamics) are each assigned specific terms of the model. In some embodiments, a model comprises associations determined without dependence on such term mappings; for example, by the use of machine learning techniques. Optionally, machine learning techniques are used to find the associations among treatments delivered/assayed and results achieved without explicit description of the parts of the system within which treatment operations occur.

In some embodiments, a process of treatment bootstrapping occurs. Optionally, some model parameters are initially determined by data gathered before treatment. For example, analysis of a cancer biopsy potentially allows construction a cancer-describing vector, the vector comprising a plurality of vector components corresponding to aspects of a plurality of cell types found in the cancer. Sensitivities of each cancer cell type-optionally, also of somatic cell types related or unrelated to the cancer-are determined *ex vivo,* in some embodiments, over the course of isolation and screening of treatment components (in particular, of immunocompounds found to bind selectively to tumor targets and/or which have other effects on the immune system).

Other data is developed over one or more rounds of treatment. For example, though assays may allow determination of preferable relative concentrations of immunocompounds, the relationship between a treatment dose delivered and effective dose received (at the tumor and/or in non-targeted tissue) is potentially undetermined (and/or modified by numerous unknown factors). Optionally, a model estimates this factor initially, for example, based on general knowledge of effective dosages by similarly compounded treatments. As there are potential costs to both over-administration (in the form of adverse effects) and under-administration (in the form of lost time, lost opportunity, and/or lack of measurable response) of a treatment, early calculations are optionally directed toward quickly but safely "locating" actual treatment responses relative to the known data. For example, one or more initial treatments are provided using immunocompounds without and/or with reduced levels of toxic conjugates, with reduced dosage overall, and/or with the immunocompounds being preferentially labeled with markers (for example, metallic and/or radioactive markers) allowing their identification in diagnostic imaging. Optionally, *in vitro* binding data determined over a range of concentrations can then be matched to the *in vivo* findings, allowing a scaling factor to be quickly determined.

Another type of information which is optionally refined over the course of treatments is the dynamics of the system surrounding the disease to be treated. Determined *in vitro* specificities, and/or determined *in vitro* toxicity, potentially map to effects on reduction of the cancer through factors which include the growth state of the cells actually in the cancer (their vitality, for example), and/or the state of the patient's organism. In some embodiments, a delivered treatment is optionally treated as a vector applied to the vector of a cancer through a matrix of such cancer and patient dynamics, with the weights of the vector determined from *in vitro* data, and the dynamics initially estimated. An output of this result is optionally taken to be a new cancer state, taken from new measurements (by biopsy, imaging, or otherwise determined). The error between the result anticipated (or estimated) and the result achieved, is itself a vector.

The meaning of the error vector, in some embodiments, itself is subject to treatment by further calculations. Optionally, the appearance and/or increased growth of selected components of the vector (other components having reacted generally as anticipated), indicates the arising of a new cancer cell subtype. In some embodiments, isolation and screening efforts are directed toward the identification of treatments selective for this subtype, as performed for the initial stage of treatment. Optionally, an explanation for large deviations from the expected change in several components together is sought from other data relating to the patient state. For example, an obvious degradation in patient performance status potentially explains a failure to reduce the cancer as expected. Alternatively, there is a change in the conditions of the cancer which allows acceleration of growth; however, this potentially masks the arising of a subtype change.

Additionally or alternatively, and particularly at the beginning of a series of treatment cycles, the explanation for the change is potentially one of initial under- or over-estimation of dosage efficacy, patient dynamics, and/or cancer dynamics. Optionally, one or more of these terms is correspondingly corrected to increase the accuracy of treatment effect predictions in further rounds of treatment.

Increasingly accurate prediction of treatment effects over the course of a plurality of treatment cycles provides a potential advantage for early detection of significant change in the clinical situation. The more confidence that can be placed in predictions of treatment effects, the more meaningful is any surprise in the results of the treatment-since that surprise is more likely to underlie an actual change in the cancer/patient system. In some embodiments of the invention, particularly relevant changes detected by deviation from calculated results include, for example, rising levels of a cancer cell subtype, and/or unexpectedly high levels of an adverse effect. It should be emphasized that such changes can occur even against a background such that the absolute effect is neutral, or even improving. Thus, in some embodiments of the invention, a clinical situation which seems overall to be under good control, can, upon inspection of the difference between exception and reality, be understood to be at risk of future deterioration. Optionally, treatment options are corrected to adjust for this prediction, without it being necessary to wait for an absolutely adverse trend to appear.

In some embodiments of the invention, this allows a reduced quality determination, possible an estimation or a prediction, possibly based on a reduced accuracy measurement (e.g., at least 20% or 40% inaccurate), of an effect of treatment on a patient, at least for some treatment cycles.

A particular feature of some embodiments of the invention is that adaptation of the model compensates for lack of precision thereof at a start of treatment.

A particular feature of some embodiments of the invention is that the cancer model need not be precise, for example, erring by at least 10%, 20%, 40% or intermediate or greater percentages with respect to a correct reaction (e.g., time, amplitude) of a cell type to treatment. Rather the use of iteration allows the process of treatment as a whole to converge. Optionally, this allows a faster determination of susceptibilities as they may be imprecise and/or determination of susceptibilities of a pool of differing cells as a single susceptibility.

In some embodiments of the invention, calculation comprises determination of non-linear effects of treatment. In some embodiments, the calculation is "wet" calculation, performed in the course of screening. Clones (for example, hybridoma clones) producing antibodies which are found to be selective for a particular cancer cell clone lineage are optionally merged into a single treatment option. Since pooling is driven by target selectivity, a potential advantage of this is the discovery that the N merged types of treatment clones can have a super-linear effect on the target that they have in common. For example, a plurality of different drug selectivities combine to drive a cell type below a viable threshold, where either one alone has a sub-threshold effect. Non-targets which are at least partially independent of the epitope(s) bound by the pooled selectivities potentially experience a lowered non-linear effect, with the result that a greater effective selectivity is achieved. Insofar as the treatment calculations treat this pooled treatment as a single treatment, the non-linear effects are implicit in calculations of dosage. In some embodiments, however, at least two treatments which combine for non-linear effects on target and/or non-target tissue are separately maintained and calculated for dosage. For example, the non-linear effects are potentially unobserved initially, and discovered in the course of treatment. Discovery of such effects in the course of treatment could comprise, for example, an observation that a commonly targeted cell subtype is reduced more than expected. Optionally, such an observation elicits a new sequence of *in vitro* screening, to determine whether an interaction exists that can be taken advantage of.

In another example of the use of non-linear effects, in some embodiments, treatments comprising competing specificities of somewhat non-overlapping selectivity are provided with different toxicity levels, for example, by use of a conjugating agent on one and not the other. Optionally, the two treatments are provided in competition, such that the lowered-toxicity treatment competes effectively with the higher-toxicity treatment at non-target sites, lowering the net occurrence of adverse effects from those sites. Optionally, competition at the target sight favors the higher-toxicity treatment. Potentially, this allows increased concentration of the more toxic specificity for binding at treatment sites, by increasing the effective specificity difference between target and non-target sites.

An aspect of some embodiments of the invention relates to systems and methods for the creation and iterative updating of a treatment determined according to the specificities of a plurality of treatment components, and the dynamically changing treatment targets presented by the disease itself. In some embodiments, the plurality of treatments are provided according to a treatment profile, the profile comprising a determination, for each of the plurality of treatment components, of the effect of the treatment component on each of a plurality of potential treatment targets and/or adverse effect recipients. In some embodiments, the profile is iteratively changed and/or regenerated, according to changing conditions of the disease and the specific susceptibilities of the targets it presents.

In outlining features of some embodiments of the invention described herein, reference is made to cancer, cancer treatments, tumors, and the like both as examples of disease for which some embodiments of the invention are potentially advantageous, and more generally as examples of concepts and principles embedded in the invention. Nevertheless, it should be understood that, in some embodiments, the treatment is of another disease to which an immune response may be mounted, such as influenza and/or HIV infection or auto-immune diseases and/or diseases which adversely affect the immune system. In particular, embodiments of the invention are potentially beneficial in relation to diseases where epitopes/specificity targets are variable (over time within a patient and/or within a population). In some exemplary embodiments of the invention, the method potentially provides particular value by enabling the use of a source of specificities already directly exposed to the specific targets of the disease variant or variants in play.

In some embodiments, a treatment option profile is initially generated based on samples and/or tests which provide measures of individual treatment component properties. For example, patient samples (including healthy and/or cancerous cells) are exposed to candidate treatment components, and the effects of the exposure measured. In some embodiments, the profile incorporates treatment characteristics otherwise known and/or determined; for example, based on known effects on a population of previously treated patients. Treatment components comprise, for example, antibodies sourced from the patient, and/or from another antibody library, with or without conjugation treatment. In some embodiments, non-immunocompound treatments are also included in the treatment option profile. In some embodiments, non-immunocompound treatments used include, for example, radiations therapies, antibiotics, hormones and/or hormone agonists or antagonists, chemotherapeutic agents, antiviral drugs and/or a therapies comprising another treatment type.

In some embodiments of the invention, generation of a profile comprises generation of the treatment components themselves. In some embodiments, antibodies are sourced from the patient; for example from B cells of the patient. In some embodiments of the invention, T cells are used as a source antibodies and/or antibody specificities. Antibodies are obtained, for example, from hybridomas produced based on the antibody producing cell population. In some embodiments, produced antibodies are evaluated for disease-targeting effects, and propagated to a stage of evaluation for treatment efficacy/adverse effects. In some embodiments, evaluation is guided by the initial status of the disease. For example, hybridomas and/or antibodies specific to the most prominent cell types/clones of a cancer are preferentially propagated to a further stage of evaluation. In some embodiments, antibody treatment components are developed which are specific to a cell type which is not cancerous, but is required for cancer growth (support cell).

In some embodiments, repeat iterations of treatment are provided for, by which a first version of a treatment profile is updated according to effects of a treatment supplied to a patient based on the treatment profile. Samples from and/or tests of the patient are obtained/made after a treatment cycle. The results of tests/sampling are compared to the results expected when the last version of the profile was formed. In some embodiments, particularly upon initial application, the results expected are formulated broadly: for example, based on *in vitro* assay results. A relatively broad formulation of expectation comprises, for example, an expectation that a particular clonal population should be reduced by a treatment component, with adverse side-effects anticipated as minimal, though as yet untested *in vivo*. A treatment design including such a component potentially includes it at a measurably effective but conservative level, this expectation being formulated based on binding and/or other properties observed in *in vitro* assays.

Before a subsequent round of treatment administration, in some embodiments, the profile of the treatment is updated to include information about what actually occurred as a result of the previous treatment administration: for example, adverse effects, tumor clonal cell population changes, and/or treatment (antibody) distribution in the patient at and/or away from a tumor site. In some embodiments, the treatment dose itself is updated as a result of this information. A relatively simple situation, for example, comprises raising a dosage of a component which is found partially effective, without observed risk of adverse effects. Optionally, where both a treatment effect and an observed risk of adverse effects are seen, a dosage level is optimized to a balance between treatment effects and adverse effects. Optionally, the balance, and/or a boundary condition of an acceptable balance, is determined based, for example, on the patient performance status.

In some embodiments, dosage (optionally, a dosage balancing adverse and treatment effects) is found through an iterative approach. In some embodiments, *in vivo* antibody distribution data is available (for example, from imaging). Optionally, the relative concentration of antibodies seen in target/non-target tissues is used to locate observed *in vivo* effects within a range of efficacies observed *in vitro.* This locating in turn is optionally used to determine of how much increase in dosage is likely to be permissible and/or increasingly effective. For example, if there are both target- (e.g., tumor cell) and non-target (e.g., partially bound somatic cell) *in vitro* binding curves available, the ratio of distributions seen *in vivo* is optionally compared to a point on the binding curves where a similar ratio is seen. The upper limit of dosage administration is optionally set so that no more than some predetermined fraction of predicted maximum binding occurs in non-target tissues- for example, no more than 5%, 10%, 15%, or another greater, lesser, or intermediate value. Additionally or alternatively, a threshold is set for a target tissue, for example, to reduce the risk of overdosing, and/or to control the rate at which target cells are removed. Optionally, the threshold relative to maximum binding is, for example, 80%, 85%, 90%, or another greater or lesser threshold.

In some embodiments, adjustment of the dosage is according to another rule. For example, a factor rule is applied, which allows increase of dosage between each treatment cycle by a factor of 25%, 50%, 75%, 100%, or another greater, smaller, or intermediate factor, so long as no contraindication is seen. Optionally, treatment increase is stopped when increased binding efficacy to the target (and/or reduction thereof) is no longer observed; or when a dose-dependent increase in efficacy falls below a certain relative level to a previous treatment-for example, less than 20%, 15%, 10%, 5%, or another greater, larger, or intermediate increase factor relative to previous efficacy. Additionally or alternatively, dosage increase continues only for so long as increased distribution of antibody in to tumor tissue is observed to outpace increases in non-tumor tissue. Additionally or alternatively, dosage increase is halted when distribution of a treatment antibody to non-tumor tissue is found to be above a level of minimum sensitivity, initial detection, non-selective distribution, or another chosen level by some factor. Optionally, the factor is, for example, 2x, 4x, 8x, or another greater, smaller, or intermediate factor.

In some embodiments, dosage increase is stopped upon observation of adverse effects, or pre-adverse effects (changes which, though potentially not adverse in themselves, indicate a change in the direction of an adverse effect) in the patient. Halting of dosage increase occurs particularly if such effects are localized at least in part to tissue regions for which the administered treatment is known to be selective.

In some embodiments, treatment efficacy and potential adverse effects on the patient are modeled according to one or more techniques of mathematical modeling (and optionally optimization). For example, treatment efficacy is chosen as a quantity to maximize, according to a model of patient and limiting criteria comprising limits to adverse effects. Additionally or alternatively, patient adverse effects are chosen as a quantity to minimize, according to a model of a patient and limiting criteria comprising a prescribed minimum level of efficacy.

In some embodiments, the system state (e.g., cancer and or patient states) is learned by probing. For example, an initially high level of treatment is reduced rapidly after a probing "pulse" of treatment.

In some cases, learning the system state uses more than a single pulse of treatment. For example a series of treatments and/or interventions may be applied to determine, based on the response thereto, a model of the patient/disease system and/or to determine one or more parameters therefor. Various methods of estimating system (based on various assumptions, such as linearity) are known in the art and may be used, as well as other methods. Optionally, when a series of interventions are tried out, intervention(s) are selected and/or determined based on one or both of patient safety, identification and amount of information missing in the model and/or timeline of disease progression.

In one example, a series of pulses can be used to characterize a system, e.g., $y=A*x$; $A=[y\_1|y\_2|...y\_n]*[x\_1|x\_2|...x\_n]^{-1}$, where $y\_i$ are observation vectors and $x\_i$ are transmission vectors - probing treatment "pulses". Optionally, $\{x\_i\}$ are linearly independent, and the matrix is invertible (though these constraints can be relaxed using other formulations/methods). For example, there may be an orthogonal basis; more specifically, $\{x\_i\}$ may be pulses of various unit impulses, such as $x\_i=(0,0,0,...1,...0)$ (1 in the i-th coordinate). They may also be selected to be other linear independent set elements, such as cosine functions, or any other suitable combination. In some cases, actually orthogonal treatments cannot be selected and/or may delay treatment too long, so non-orthogonal treatments may be selected for "probing".

In another control regime, treatment dosages are adjusted to titrate effects on clonal cell lines to one another. Optionally, titration is calibrated to reduce both cell lines in synchrony, even if it is technically possible to reduce at least one of the cell lines more rapidly. Potentially, this is an advantage for cancers where two cell lines are in competition, with one or both serving to inhibit the other. Synchronous reduction potentially prevents releasing one of the cell lines from the effects of competition.

In some embodiments, treatment and/or adverse effects are modeled linearly. In some embodiments of the invention, non-linear effects are measured-for example by co-assays of treatment types, and/or by analysis of effects resulting *in vivo* when treatment components are varied such that their independent and interacting effects are potentially distinguishable by statistical analysis and/or non-linear modeling.

In some embodiments, the effect determination for each component is normalized to one or more common scales; for example: lethality to cells and/or cell types, effect on a cell population, effect on a physiological measure, and/or effect on patient performance status (well-being and/or quality-of-life). In some embodiments, the common scale expresses relative effectiveness/adverseness according to an arbitrary scale. For example, a treatment for which a tumor reduction rate is available in mm/week, and a treatment for which a cultured cell death rate is available in terms of LD50%, are normalized for comparison to a common "effectiveness" scale according to conversion formulae appropriate to each measure. In some embodiments, such calibrations are adjusted based on their observed accuracy, and the adjusted calibrations applied subsequent re-treatments, allowing appropriate treatment doses to be more accurately determined.

In some embodiments, treatment components (new antibodies, for example) continue to be developed/screened over the course of the adaptive treatment cycle. This is a potential advantage for dealing with the evolution of a cancer, and in particular to meet the exigencies of a cancer evolving in response to highly targeted set of treatment specificities. Potentially the both quantitative and semi-continuous nature of the process of treatment allows early indications of changes in the cancer cell population to be detected, and sufficiently long lead time to develop and amplify effective countermeasures, before the cancer escapes control.

In some embodiments of the invention, a system for performing portions of the methods described herein comprises a computer, configured by programming to implement processing, for example of models and/or algorithms described herein.

In some embodiments, a system implementing aspects of the invention comprises a processing module for receiving immunocompound screening data comprising selectivity information for a plurality of cell and/or tissue types, and converting it to a database (stored as an array, relational database, spreadsheet file or other appropriate format) which relates treatment component selectivity and/or other binding information to cell/tissue types of a patient to be treated. Optionally, the treatment components categorized are immunocompounds. Optionally, the immunocompounds have selectivities corresponding to selectivities of antibodies produced by the patient's own immune cells; for example, B cells, or other antibody producing immune cells.

Screening data comprises, for example, data describing effects of isolated immunocompound preparations and/or immunocompound producing cultures on cultured cells (optionally, cells taken from the patient), distributions of immunocompound binding in histological preparations (optionally histological preparations of tissue, for example, tumor biopsy material, taken from the patient), distributions of immunocompounds determined by *in vivo* imaging of immunocompound distribution, or another form of screening data which characterizes the activity of a treatment components binding and/or toxicity characteristics as a function of target type.

In some embodiments, a system implementing aspects of the invention comprises a treatment selection module. The selection module is configured to accept an initial and/or current configuration of a disease state, and the treatment selectivity database. Optionally, a goal state of the treatment and/or current treatment round is accepted as an input, and/or is specified by the configuration of the module. Optionally, one or more constraints on allowable routes by which the goal state is to be approached are configured and/or accepted by the treatment selection module. Optionally, the treatment selection module accepts and/or implements descriptions or models of non-treatment dynamics, for example, patient dynamics and/or cancer dynamics. In some embodiments, the treatment selection module seeks a state consistent with the current cancer state, available treatments and their effects, constraints, and/or dynamics, and which can be reached by administration of a combination of available treatment components.

In some embodiments, a system implementing aspects of the invention comprises a model adjustment module. The model adjustment model comprises functionalities for calculating differences between an expected (modeled) result of a previously administered treatment plan, and actual results as observed/measured subsequently. In some embodiments, the model adjustment module accepts input describing an updated disease state (optionally, the updated disease state is formatted as for the configuration of a disease state used by the treatment selection module), and accepts also the modeled, measured, and/or assayed data structures available to the other modules of the system. Optionally, adjustments are made to the modeled data structures, for example, to adjust estimates of dynamics, calibration factors, or other information, so as to anneal the modeled disease/patient state more closely to the observable aspects of the clinical situation.

In some embodiments, the model adjustment module is configured to recognize certain forms of discrepancies as potentially signaling an imminent change within the patient/disease system, and to produce an alert accordingly. For example, a rise in a particular cell type which was previously not treated-or treated, but the treatment is found to be unexpectedly ineffective-raises the possibility that the cancer is escaping its current state, potentially requiring additional screening work and/or adjustments to the treatment database to allow adjustments to the treatment which fall outside of the already available range of possibilities.

In some embodiments of the invention, an external processor is provided which implements as disease state predictor. Optionally, the disease state predictor takes data about a current disease state, and produces a new prediction of disease state, based on an assumption that a particular treatment composition will be provided. In some embodiments, the prediction includes a description of the particular treatment composition. In some embodiments, a differential between the predicted disease state and an actually measured disease state comprises a basis for adjusting the parameters of the disease state predictor on which predictions are based.

An aspect of some embodiments of the invention relates to methods and systems for auxiliary support to a native immune system.

In some embodiments of the disclosure, the immune system of a patient is augmented by a supply of immunocompounds and/or immunocells delivered from outside the body. For example, the sensing ability of the immune system is used and immunocompound production provided externally. In another example, sensing is provided externally and the immune system is stimulated to produce immunocompounds as needed. Optionally or alternatively, the reactiveness of an immune system is increased. Optionally or alternatively, the type of reaction of the immune system is augmented using external logic which can, for example, select a desired level and/or type of immunocompounds based on a prediction of a change in disease and/or health state(s) over time and/or in response to treatment.

In some embodiments, a current status of the immune system of a patient is sampled at one or more time points, for example, by drawing of lymph or blood and/or by leukapheresis (or by another method, for example as described hereinbelow in relation to patient cell sampling), and assayed against one or more disease antigens, for example as described hereinbelow for screening of hybridomas, pooled hybridomas, and/or antibodies.

Optionally, one or more dominant antibody specificities present in the sample is produced (in the form of antibodies or another immunocompound) externally to the patient, for example as described in relation to patient cell sampling, formation of antibody producing cells, and antibody production, hereinbelow.

In some embodiments, the externally produced immunocompound is supplied the patient. Optionally, the patient supply dose is selected proportionally to the difference in concentration of the corresponding antibody specificity measured between two of the sampling time points. Optionally, the proportionality is determined by the difference expressed as a rate of rise between at least two time points. Optionally or alternatively, the proportionality is determined using a different function of the points, for example, using proportional-integral-derivative controller (PID controller).

Optionally, the proportionality is determined by another marker of cell population change, for example, cells taking up a tracer, and/or counts of cells in a particular phase of their life cycle (for example, as determined by expression of factors related to the recent completion of cell division). The factor determining the amount of resupply is set, for example, to bring the supplied immunocompound to reach an average blood concentration which is about 50% of the current average blood (or other fluid and/or body region) concentration of the pre-existing corresponding antibody specificity in the blood (or other fluid and/or body region). In some embodiments, the supply concentration ratio is about 10%-20%, 15-30%, 25%-50%, 40%-60%, 50%-80%, 75%-100%, or within another range of concentrations having the same, larger, smaller, and/or intermediate bounds. In some embodiments, this arrangement comprises a form of feedforward control logic for immunocompound concentration. In an exemplary embodiment of the invention, the amount provided is a factor of, for example, 3, 10, 50, 100, 1000, 10,000, 100,000 or more or intermediate factors of an amount of immunocompound (e.g., 1, 2, 4, 5, 10, 20 or intermediate or greater numbers of different immunocompound and/or different targets for which the immunocompound are designated) estimated to be in the patient. In some cases, this provision compensates for an increased reaction time and/or amplitude of the body, for example, compensating for a weakened immune system or reducing an effective reaction time (how soon enough immunocompound are in the body) by, for example, a factor of at least 10, 5, 2 or intermediate or greater factors.

In some embodiments, the native (controlling) antibody population is chemically differentiated from the supplied (input) concentration of immunocompound. For example, the input immunocompound is conjugated to a tag or otherwise comprises a modified form of the native specificity which it augments. Optionally, this differentiation is used as a basis for detection of the natively generated signal state against the background of introduced immunocompound. For example, the introduced immunocompound is potentially blocked at a binding site to which an immunolabeling compound is bound in an assay for determining current levels of immunospecificity.

Potentially, the exogenous supply of antibodies assists the activities of an ongoing immune response by the patient, according to an antibody which is at least partially controlled by the immune response itself. A potential advantage of the exogenous supply is to reduce the energy investment of the patient in the activities of the immune system, since a portion of the response needed is developed externally. Another potential advantage is to supplement the functioning of an immune system weakened by disease and/or treatment. It is a potential advantage to rely on the control signals of the native immune system, to take advantage of the autoregulatory powers of the immune system, while nevertheless augmenting its responsiveness.

Optionally, the feed-forward mechanism is used for control, in addition to and/or in place of reliance on feedback control. In some embodiments, for example, during a relatively acute immune event such as an infection occurring in a weakened patient, an immune cell population is withdrawn from a patient, for example by leukapheresis. Optionally, cells are cultured to produce antibodies outside the body, and the immunoproducts of the cells reintroduced during a period when the patient potentially benefits from the assistance. Optionally, a portion of the withdrawn cell population is restored to the patient as a part of the assistance therapy.

In some embodiments, for example, for a disease having stereotyped antigen presentation, antibodies previously generated from any safe source can be provided. However, it is a potential advantage to provide antibodies which are essentially copies of the patient's own antibodies, to reduce the chances of adverse effects. Personalized feed-forward immunotherapy is potentially most useful for a range of disease time courses wherein effective immunotherapy can begin after the time period required for initial culturing, after an initial antigen binding activity can be isolated.

In some embodiments, augmented supply of immunocompounds is based on autologous leukapheresis. Optionally, temporarily harvested immune cells are cultured externally to the body, and later returned to the body in a ratio determined by a measured change in the population.

In some embodiments of the disclosure, immune response is enhanced by stimulating the immune system, for example, by injecting dead tumor cells and/or killing (or otherwise damaging) them in-vivo. Optionally, which cells to inject and/or kill in-vivo is selected according to methods as described herein, rather than uniform ablation of a tumor.

It is a particular feature of some embodiments of the invention that the number of different tumor cells to be treated simultaneously is assumed to be relatively low. In an exemplary embodiment of the invention, this assumption is based on an assumption that different tumor cell types occupy different sectors of a tumor and are thus geometrically limited to between 2 and 50 types, for example, between 7 and 20, 6 and 15, 5-10 or 10-13, or intermediate or greater numbers (see for example, Anderson AR, Weaver AM, Cummings PT, Quaranta V. Tumor morphology and phenotypic evolution driven by selective pressure from the microenvironment. Cell. 2006 Dec 1;127(5):905-15; Gonzalez-Garcia I, Sole RV, Costa J. Metapopulation dynamics and spatial heterogeneity in cancer; Proc Natl Acad Sci USA. 2002 Oct 1;99(20): 13085-9; Maley CC, Galipeau PC, Finley JC, Wongsurawat VJ, Li X, Sanchez CA, et al. Genetic clonal diversity predicts progression to esophageal adenocarcinoma. Nat Genet. 2006 Apr;38(4):468-73; and/or Shibata D. Clonal diversity in tumor progression. Nat Genet. 2006 Apr;38(4):402-3).

It should be noted that a tumor may contain many types of cells. However, with respect to cells that have significantly different functional, behavioral, membranal and sensitivity (to treatment) properties, the above numbers are optionally used (e.g., for modeling, predicting and/or tracking effect). In an exemplary embodiment of the invention, as the tumor evolves, new cell types may come to the front of treatment.

In an exemplary embodiment of the invention, the above assumption makes it feasible to start with a scalar or small vector assumption about the disease (e.g., 1-3 components) and increase the vector size and treatment is applied and reaction to treatment is assessed.

It should be noted that the state vector (or vectors) can include multiple (e.g., 1, 2-20, 4-15, 6-10, 8-30 or smaller, intermediate or greater numbers) non-disease components, for example, patient health/vitality components, risk components (e.g., accumulating risk of certain happenings such as kidney failure), and/or cost components.

A feature of some embodiments of the invention relates to adapting treatment faster than a tumor phenotype can evolve. For example, the majority cancer types are attacked simultaneously so that none of them can emerge as a majority type. A feature of some embodiments of the invention is that the treatment is adapted faster than the genotype of the tumor can adapt, for example, faster than new cancer cell types can evolve to resist treatment. In an exemplary embodiment of the invention, the timing is determined by analyzing the existing cell types and/or using a model of evolution and/o of the particular patient and/or disease.

In an exemplary embodiment of the invention, what is provided is a process for isolating the antigenic profile of the patient tumor and producing the corresponding oligo-clone treatment, based on that profile. Eradication of heterogeneous tumors is optionally a process of:

a. Deriving the tumor antigenic profile directly from the patient and producing a matching pool of antibodies.

b. Applying the antibodies either at once against all targets or in sequence by applying a smaller antibody group, detecting the new profile, and acting against it, thus generating immune response against the multiple antigens profile.

In this respect it is optionally desirable to evaluate the number of clones that may populate a malignant tumor. Although there are theoretically endless genetic variations, due to micro environment restrictions and evolutionary pressure, in practice only a limited number of clones are generally found (e.g., 10-13).

Ensuring maximum antigenic sensitivity and therapeutic efficiency is optionally provided by performing the "full" drug development cycle on a patient tumor derived antigens and thus producing a custom-designed drug matching the person's tumor event.

In an exemplary embodiment of the disclosure, mature B cells are isolated from peripheral blood in each patient. These cells are then immortalized by fusing to human myeloma cell to form a hybrid cell (hybridoma), cultivated and screened for the secretion of specific human antibodies against the patient's own tumor cells. In some exemplary embodiments of the invention, established hybridoma lines that are found to have a good reaction with cancer cells are cultivated and their secreted antibodies harvested and purified.

In an exemplary embodiment of the invention, the reduction of the number of clones retrieved from a single patient is achieved by combining similar clones into a master clone, and then directed against a specific target.

In some exemplary embodiments of the invention, this master clone includes some inherent variability and may better cover the naturally occurring antigen variance. Optionally, clones are pooled together by their genetic homology, for example, as analyzed from the sequences of the antibodies variable region.

In an exemplary embodiment of the invention, purified monoclonal antibodies specific to the patient's tumor cells, are assessed in one or more of several ways, for example one or more of:

1. Cross reactivity - Antibodies are tested against human tissue chips (An FDA approved protocol).
2. Tumor biopsy - The antibodies' specific reaction is tested against the patient's own tumor biopsy.
3. In vivo - Antibodies are conjugated to magnetic particles or a radioactive label, administered to the patient in low dosage and their bio-distribution and accumulation monitored.

In an exemplary embodiment of the invention, the patient's anti-tumor treatment is a sequential procedure: At a first treatment (e.g., the 1st iteration) an antibody clone cocktail is constructed based upon, for example, the information retrieved as noted above and an initial therapeutic dosage is administered to the patient. Some or all of the following iterations are optionally based on information gathered previously, and may include antibodies conjugated to magnetic nanoparticles, radioactive or cytotoxic molecules.

In an exemplary embodiment of the invention, when searching for a treatment protocol, a best treatment taking into account a desired goal is searched for. In some cases, a best treatment cannot be found and/or cannot reasonably be proven to be best, and a satisfising or "best so far" approach is used. In some embodiments, a plurality (e.g., 2-5 or more) of goals are suggested and the cost and/or ability to reach is one is suggested (e.g., cure with less than 50% fatality vs. 6 month with more than 80% certainty). Various methods of searching mathematical/system spaces are known in eth art and may be used. In an exemplary embodiment of the invention, risk is modeled as one of the components which is affected by treatment, etc.

Before explaining at least one embodiment of the invention in detail, it is to be understood that the invention is not necessarily limited in its application to the details of construction and the arrangement of the components and/or methods set forth in the following description and/or illustrated in the drawings. The invention is capable of other embodiments or of being practiced or carried out in various ways.

### Iterative Treatment Targeting

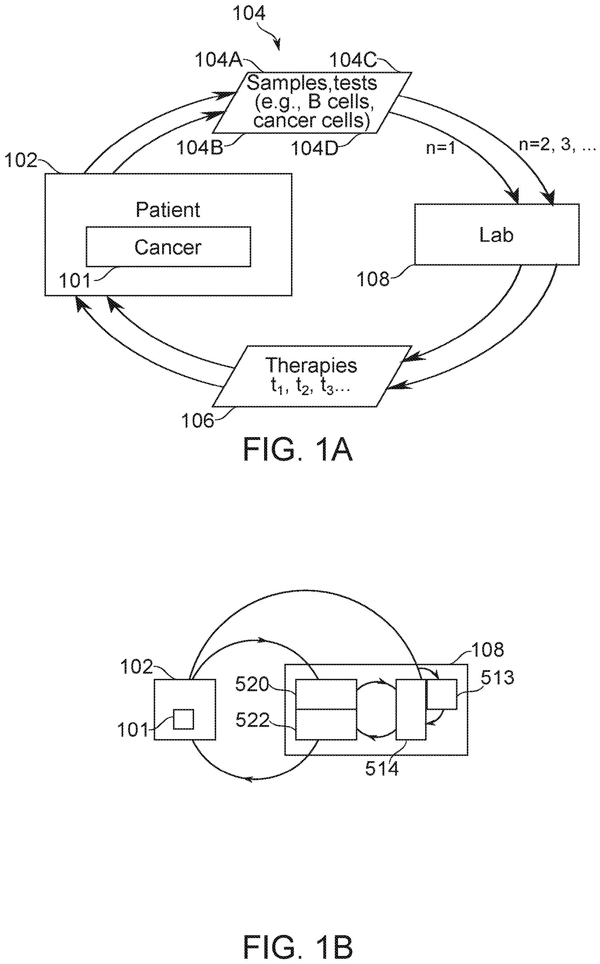

Reference is now made to *Figure 1A,* which schematically illustrates cyclic development, adjustment, and application of an adaptive, patient-specific treatment regime, according to some exemplary embodiments of the invention.

In some embodiments of the invention, a patient **102** presenting with a disease **101** for treatment (for example, a malignant cancer) is subjected to testing and/or sampling.

In some embodiments, samples **104** are obtained for use in laboratory operation. Disease samples **104A**, in some embodiments, are obtained for determining aspects of the disease itself. For example, samples of a tumor are obtained, from which the clonal profile of the tumor is determined. Determination of clonal profile includes, for example, isolation and/or identification of different clonal types making up the tumor. In some embodiments, determination of clonal profile includes determining the prevalences of the various clonal types. Other samples include, for example, samples of patient tissue near the site of a tumor which has been reorganized and/or recruited for the support of the tumor, and/or healthy patient tissue. In some embodiments, samples of tumor, supporting, and/or healthy cells are designated for use in assaying effects of potential treatment candidates.

In some embodiments, treatment source samples **104B** include, additionally or alternatively, cells from which potential treatments are to be derived. For example, samples **104B** comprise immunoglobulin producing cells of a patient, and in particular, B cells. In some embodiments, B cells are designated for use in the production of antibody-producing hybridomas, as a source of binding potential for direct and/or modified use in the delivery of potential treatments.

In some embodiments, status samples **104C** and status tests **104D** include additionally or alternatively other sampling and tests, which may help to determine a current state of the patient and/or the patient's disease. Status samples include, for example, body fluids such as blood, urine, and/or lymph; as well as potentially including samples of tissue biopsies taken also for treatment target evaluation and/or treatment development.

Status tests **104D** also include, for example, test of the samples which relate to overall health, for example, to determine if there is a current deficiency and/or organ malfunction indicated by the samples. In some embodiments, tests comprise tests of patient well-being, such as response to stress tests. In some embodiments, tests comprise findings of patient-reported and/or physician-observed parameters, such as daily activity level, responsiveness, or the like. In some embodiments, particularly after initial development of disease-specific treatment components, tests **104D** comprise tests, for example tag distribution imaging tests, whereby concentrations of antibodies (optionally tagged with a magnetically and/or radiographically detectable agents) which reach the treatment target are determined. Optionally, imaging tests record concentrations of antibodies which reach and bind to non-treatment targets, whereat the antibodies potentially contribute to adverse effects.

While some embodiments of the invention relay on immune assays, other embodiments use other assessment methods, such as (but not limited to) DNA sequencing, protemic analysis and/or mRNA analysis.

In some embodiments, the samples and tests **104** comprise input for processing by laboratory **108**. In some embodiments, laboratory processing comprises distinguishable processing streams-running in parallel and/or sequentially-for treatment production, treatment assessment, and determination of which treatment components are to be delivered to the patient, and in which dosages.

Reference is now made to *Figure 1B,* which schematically illustrates sub-loops within the cyclic development, adjustment, and application of an adaptive, patient-specific treatment regime, according to some exemplary embodiments of the invention.

One stream of laboratory processing, in some embodiments, comprises the development (at block **513**) of potential treatments based on the material received. In some embodiments, this includes conversion/transformation of immune cell sample material (B cells, in particular) to the production of antibodies which are potentially useful for treatment. Potential treatment development optionally includes conjugations of antibodies to other agents, such as labeling agents and/or toxicity agents. Exemplary operations which block **513** comprises are described, for example, in relation to the sections describing *Antibody-Producing Cells* and *Antibodies,* hereinbelow. Exemplary operations which block **513** comprises, in some embodiments, also correspond to those described in relation to (c)-(f) of *Exemplary Operations in Methods of the Invention,* hereinbelow.

A second stream of laboratory processing, in some embodiments, comprises (at block **514**) assaying the binding and/or other efficacy/adverse effect properties of the developed treatments, and/of other available treatments, against the available disease targets and/or treatment-involved tissues of the patient. Operations which block **514** comprises are described, for example, in relation to the section describing *Screening and Clonal Pooling,* hereinbelow. Operations which block **514** comprises, in some embodiments, are also described in relation to (g)-(h) of *Exemplary Operations in Methods of the Invention,* hereinbelow.

A third stream of processing, in some embodiments, comprises (at blocks **520**, **522**) integrating information about patient and disease status with information about available treatments and their efficacies/adverse effects, to determine the current course of treatment therapy: including composition, dosage, delivery means, schedule, and/or other aspects of the therapy to be delivered. These blocks comprise, in some embodiments, operations described in the section *Modeling and Adaptive Control of Specific Cancer Therapy,* hereinbelow. Block **520** comprises, in some embodiments, calculating operations of (i) and (1) of *Exemplary Operations in Methods of the Invention,* hereinbelow. Block **522** comprises, in some embodiments, calculating operations of (j) of *Exemplary Operations in Methods of the Invention,* hereinbelow.

The streams of processing interact with each other, and provide results synchronously or asynchronously, according to the status of treatment. In an initial cycle, the streams are liable to proceed synchronously, as the treatments need to be developed (at block **513**), assessed (at block **514**), and qualities of the treatment to be delivered determined (at block **522**) based on the results of treatment assessments, and assessment of patient/disease state (at block **520**). It should be noted that treatment may include immunocompounds. Optionally or alternatively, other treatment methods are used. Immunocompounds may be formulated provided as described or in other manners, optionally using the iterative and/or modeling methods described herein for treatment.

In subsequent iterations, blocks **520** and **522** are optionally passed through without additional input from the side of the cycle comprising blocks **513** or **514,** or with only such input (for example, from block **514**) as becomes available as new validated treatments and *in vitro* assessments become available. In some embodiments, results of treatment determined at block **520** feed into block **514**. For example, partially undeveloped treatments affecting a clonal line which is observed to be gaining prevalence are subjected to more intensive assaying work in order to judge the concentration at which they should be introduced to the cycle. In some embodiments, new patient B cell lines from which new types of antibodies are potentially sourced are periodically accepted into block **513**. For example, if a new tumor cell line appears, it is a potential advantage to re-screen the B cells of the patient for appropriately selective antibodies.

In some embodiments, block **522** of the third stream of processing comprises in particular determination of predicted effects (including predicted magnitude) of the therapies to be delivered, and, more particularly, predicted effects insofar as expected to occur in a second or further round of therapy delivery, re-sampling and re-testing. In some embodiments, the treatment to be delivered uses the specific, patient-dependent information gathered (on a first or subsequent treatment round) at block **104** to make determinations as to treatment trade-offs.

Treatment trade-offs include, for example, the balancing of beneficial, disease-specific effects of a treatment, against adverse and/or non-specific effects of the treatment. For example, an antibody is found to have a greater selectivity for a cancer clonal line than its greatest selectivity for a type of somatic cell-it is useful, but causes side-effects. Antibody binding (and/or the effect of an agent conjugated to the antibody) kills cancer cells, but binding to the somatic cell type also potentially causes undesirable cell impairment and/or death.

However, a general understanding that a dosage should be given in sufficient quantity to kill the cancer, without unduly harming the patient, is potentially insufficient to allow determination of a correct dosage.

This is potentially true for any agent, since the balance of susceptibilities can be patient specific. However, a well-known agent has a degree of treatment experience guiding its application. A selectively binding agent, even if well-known, is potentially still difficult to correctly titrate to an optimal dosage, since the epitope environment (either targeted or incidentally bound) is not necessarily the same in every patient.

Finally, dosage determination is particularly difficult in a case where there is no prior experience with the treatment: for example, because it is newly derived from the antibody libraries of the very patient that is being treated. In some embodiments of the invention, however, *in vitro* assay results determined at block **514** allow the balance of desired versus adverse effects to be known with sufficient confidence that a dose having an effective concentration can be safely delivered.

In some embodiments, a safe (even if potentially somewhat adverse) initial dose is determinable, but the degree to which this can be exceeded for optimal effect is initially unknown. In some embodiments, a tradeoff can be made that allows an initial dose, having an effect predicted by assays at block **514**, to be provided, potentially with the expectation that the dosage is unlikely to be optimal to treat the disease. In some embodiments, the effects of the dosage are quantitatively monitored (for instance, by measurements from samples/tests **104** of changes in tumor cell population, and/or imaging assays of binding *in vivo),* and the dosage increased or decreased to strike a balance of treatment effects and adverse effects. Such dosage changes carry with them potential risk. Use of methods of the current invention allows adjusting concentrations quickly (for example, in one or two treatment cycles) while providing a potential advantage to reduce this risk.

In an example: binding curves of an antibody to a primary disease target (cell line) and to a most-endangered somatic cell type are known from *in vitro* work carried out during an early phase of treatment preparation, but, initially, the concentration that results in effective delivery is not. Drug half-life and transport rate, for example, are potentially quite different in the *in vivo* environment than in an *in vivo* assay, and potentially are even different between different delivery sites within the body of the patient.

Optionally, an initial presumed-safe dosage is delivered, allowing actual antibody delivery to be measured. Potentially, this in turn allows locating the *in vivo* treatment's effects on the determined *in vitro* binding curves. From this information, larger/more rapid increases in allowable treatment dosage can be determined, and potentially reached in a small number of additional treatment cycles, for example, as little as one additional cycle.

It should be understood that in place of "binding curve", another *in vitro* result can be substituted, for example, LD50%, reduction of metabolite production, or another measure which assays the effect of the treatment.

Moreover, in some embodiments, an adverse effect comprises a danger upon chronic exposure to a treatment, rather than an acute danger. In some embodiments of the invention, an initial dose is provided which is understood to be potentially above a sustainable level, and the dosage then adjusted downward in cycles shortened by the combined application of patient-specific, *in vitro* assays, and *in vivo* findings.

In some embodiments, trade-offs include, for example, balancing of the resource costs of treatment. For example, in some embodiments, treatments are selected for development (comprising, for example, culturing, assaying, and/or delivery) based on initial indications of effectiveness against the targets of greatest apparent importance. For example, a polyclonal tumor comprises 80% of a first cell type, 10% of a second, with the remaining fraction distributed among mixed cell types. It is a potential advantage to concentrate efforts on the first, and optionally on the second cell type, even though there may be antibodies found which show indications selectivity for the lower-population types. This helps, potentially, to manage costs of the laboratory work. Potentially, selective reduction of chosen targets provides advantages as well during testing, which is not only potentially resource intensive, but also potentially onerous to the patient in its own right. For example, tests and/or sampling potentially involve the administration of radioactive substances, and/or otherwise apply stress to a patient who is already at risk from both disease and treatment effects.

More directly relevant to the effective use of the methods of the invention: selectively targeting prevalent populations helps to constrain the number of variables to be evaluated. For example, where each clonal type is targeted by two or three antibodies, it is potentially easier-when clonal type targets are deliberately restricted-to assign the causal agent of adverse effects which arise, allowing adjustment to be made selectively. It should be understood that the reverse is potentially also true: when a plurality of treatments target the same cell line, it is a potential advantage to use only a subset of them, to allow more readily assigning the source of any adverse effects.

Another tradeoff made, in some embodiments of the invention, is to maintain a cell population balance within the tumor and/or the cells supporting the tumor. For example, one of two prevalent clonal types is potentially the less malignant (for example, less likely to metastasize), while serving also to compete with (suppress) the other clonal type helping to keep it in check. In some embodiments, treatment of the suppressive clone is deliberately balanced to be less aggressive than treatment of the more dangerous clone. In some embodiments, suppressive effects are determined, for example, by the results of co-culturing cell types. In some embodiments, the observed effects *in vivo* of treatment applications indicate potential suppressive interactions.

Returning now to *Figure 1A*: therapies **106**, in some embodiments, comprise the various iterations of the therapy composition to be delivered to patient **102**. At suitable times, patient **102** and disease body (cancer) **101** are sampled/tested again, resulting in a new round of samples/tests **104**. In some embodiments, the samples are returned into the processes of the lab **108.** In some embodiments, the samples are used to confirm and/or detect errors in the original model of disease/treatment/patient interactions (for example, at block **520** of *Figure 1B*). Optionally, the model is adjusted correspondingly (for example, at block **522**).

Potentially, the disease process is observed to undergo a qualitative change requiring a more radical adjustment of the treatment. For example, a new dominant clone appears, and/or the patient performance status undergoes a sudden change, possibly unrelated to the treatment itself-possibly related as an unanticipated adverse effect. Upon such an observation, in some embodiments, the laboratory process of developing and/or assaying disease is begun again using material from the new samples.

In some embodiments, initial treatment is designed based on preliminary laboratory assay results and/or treatment production quantity, with subsequent culturing/assaying/production continuing on a slower cycle in parallel with the more rapid iterations of the treatment cycle. In some embodiments, parallel laboratory work results in new treatment options, and/or new results regarding expected efficacy/adverse effects which potentially affect treatment design.

In any of these cases, the results are optionally fed into the design of a new treatment regime based on corrective data and/or treatments which become available.

In some embodiments, the process continues through additional cycles until a suitable endpoint is reached. The endpoint is, for example, total removal of the cancer or a sufficient level of cancer remission. In some embodiments, treatment continues, but is re-tailored to a maintenance level of treatment, wherein cycle times are lengthened, and/or treatment level is lowered.

**Modeling and Adaptive Control of Specific Cancer Therapy**

**Initial Modeling and Adaptive Modeling**

According to some embodiments of the present invention, there is provided a method for modeling cancer clones (and/or other diseases, particular diseases where a plurality and/or evolving set of epitopes are presented to the immune system), and for determining an adaptive treatment protocol based upon such modeling. Optionally, the adaptive treatment augments the native immune system of a patient. The modeling process optionally starts with an analysis of a plurality of characteristics of the cancer cells from the patient. The model optionally starts with a simple linear model of the behavior of the cancer cells; for example with regard to known biomarkers. Biomarkers include, for example, whether the cancer cells are sensitive or insensitive to hormones and other external stimuli, the presence or absence of the activity of various oncogenes, viability, proliferation rate, migration and so forth.

In some embodiments, the model is refined once antibodies to the various clones are available and have been screened. Optionally, the reaction of the cancer cells to these antibodies is considered: for example, whether the antibodies induce apoptosis, rapid cell death or cell quiescence (defined herein as a return to a non-proliferative state). Optionally, the reaction is to antibodies conjugated to an agent configured for induction of a toxic reaction, either natively, or upon application of a triggering stimulus.

The model is then preferably used to select a combination of antibodies for treatment of the subject. As described also hereinbelow: after treatment with the selected, optionally conjugated, antibodies (optionally with one or more additional treatment modalities as well), the efficacy of the treatment on the patient is assessed. After assessment, the treatment is optionally adjusted. Optionally, the method is iteratively repeated, such that adaptation of the model leads to adaptation of treatment and hence, to therapeutic effects more closely adjusted to the particular clinical situation and requirements of the patient.

Adaptation is performed, in some embodiments, according to the principles of what is often referred to as "adaptive control" (or "adaptive signal processing") principles. Adaptive control is commonly defined as a method for controlling a certain dynamic system by constantly coping with and compensating for temporal changes, which potentially involve substantial uncertainty.

Adaptive control is potentially advantageous in the successful, specific treatment of cancer in individual patients. A target state is selected; for example, a healthy state of the patient, or an early state after diagnosis, minus the presence of cancer. In a simplified example, changes from this state resulting from the activity of cancerous cells, should be periodically and repeatedly measured and treated, until, optimally, the patient is back to the initial, healthy state. In some embodiments, changes occurring for other reasons-for example, change in patient performance status, potentially due to the treatment itself-are also measured and accounted for in the adaptive treatment plan.

A typical cancer comprises a multi-faceted clone group of cells-that is, cells which, though related, comprise a plurality of distinguishable cell lines, based on phenotype and/or genotype differences. In some embodiments, this clone group is addressed according to its structure, as expressed, for example, by its population prevalences and dynamics, to determine a suitable cure.

In some embodiments, interactions among cell lines within the clone group are also measured and considered in the cure determination. For example, cancellation of a specific variant potentially leads to expedited growth of different and/or more virulent variants. In some embodiments, such interactions are determined by accurate analysis and determination of the different members of the pertinent cancer group, their prevalence, activity, and/or changes in any of these factors.

Other aspects for which adaptive control and mathematical modeling are performed, in some embodiments, include the patient's own dynamics and the therapeutic impact on these dynamics. In some embodiments, dynamics of the patients are considered as combining dynamics of the patient state overall (for example, corresponding approximately, but not only, to the patient's performance status), and dynamics of otherwise normal patient tissues which are involved in support functions on which cancerous growth depends.

One view of cancer is as a divergent mode of originally normal cell growth dynamics. In cancer, the patient, considered as a dynamic system, has gone from stability to instability. In some embodiments, this dynamic system is represented mathematically, and an optimal (within the framework of the assumptions of the representation) path to a stable solution sought. A system which has grown unstable can potentially be stabilized through additional control mechanisms. Potentially, added control systems even obviate the need to fix the root cause of the instability.

Put in summary: joint analysis of the patient, the cancer and the treatment allows their description, in some embodiments of the invention, as three or more interacting meta-groups. Interactions are analyzed and, and control introduced to restore the system (the patient) to a stable situation.

**Clinical and Laboratory Entities Appearing as Terms of the Model**

In some embodiments, tools used to deal with this complexity comprise matrix algebra and stability analysis. Cancer clones and other disease parameters are optionally described as a state vector, and various dynamics-of the cancer, of the patient, of the treatment-described as one or more state matrices.

In some embodiments, an adaptive, patient-specific model of the patient/illness characteristics is created (linear, or optionally including non-linear terms), and periodically updated (through measurements)-together with periodic administration of antibody and/or other treatments compounded in accordance with the model. The model is adapted until an endpoint of a cure, remission, essential stability, or another chosen endpoint is reached. In some embodiments, there is no formal "endpoint" as such; rather, as stability is observed to increase, sampling/treatment intervals increase to a limit of maintenance and/or monitoring for new disturbances, rather than actively adapted external control.

The modeling process as described above starts in the stage in which the initial model of the cancer clones is constructed as based upon the initial data provided. Time evolution of cancer is expressed, for example, in the following formula:

$$\vec{x}_{n+1} = f\left(\text{patient}_n, \vec{x}_n\right)$$

$$\text{Equation 1}$$

wherein $\vec{x}_n$ is a vector representing the state of cancer clones at time $n$. This state is determined, for example, according to the screening and analysis examples described herein. Initially, these methods, in some embodiments, relate to analysis of the cancer cells in the absence of antibodies specific to each cancer clone. In some embodiments of the invention, there is a corresponding equation for the patient, for example:

$$\text{patient}_{n+1} = g\left(\text{patient}_n, \vec{x}_n\right)$$

$$\text{Equation 1B}$$

Optionally, the malignancy of each tumor clone is determined, for example, based on the ability of cells of each tumor clone to proliferate and/or invade neighboring tissue. Thus, in some embodiments, cells of various tumor clones are isolated and tested for properties including, for example, viability, proliferation rate, migration, and/or other biomarkers, for example as discussed above.

After antibodies become available, in some embodiments, screening and analysis techniques which require antibodies against the clones as tools are used; for example, to determine the bio-distribution and accumulation of labeled antibodies, useful for constructing/adjusting of the vector $\vec{x}_n$, and/or other estimates relating to treatment efficacy, as described hereinbelow.

It is to be understood that each clone in a vector $\vec{x}_n$ can have more than one entry; for example, the clone vector includes {prevalence, malignancy, vigor}. Vigor is defined, for example, as the ratio of necrotic cells of a type to all cells of that type. Other measurable quantities or qualities are possible. For simplicity of illustration, the following discussion assumes a single scalar, representing a clone by its number of cells.

Optionally, the formula is linearized as follows:

$$\vec{x}_{n+1} = A \cdot \vec{x}_n$$

$$\text{Equation 2}$$

wherein $A$ is a matrix representing the patient dynamics, determined, for example, according to parameters such as patient size, weight, age, gender, vitality (e.g. by measuring vital signs such as blood pressure, heart rate, temperature, weight loss/gain), disease severity, and/or the nature and/or dose of other medical treatments that are concurrently administered to or performed on the patient.

In some embodiments, matrix $A$ is descriptive of both patient dynamics and cancer clone dynamics. In the case of the combined dynamics, the matrix may be partitioned into two, for example, $A = P . C$, where $P$ is the patient dynamics and $C$ is the cancer clone dynamics. Optionally or alternatively, $x_{n+1} = A \cdot x_n + P \cdot patient_n$, or another decomposition of dynamic contributions is used. Suitable initial conditions are assumed from a first measurement. Optionally or alternatively, initial conditions may be estimated and/or further refined, as part of the model, for example, based on patient information, disease and/or a baseline.

It should be understood that the specific choice of vector-matrix, matrix-matrix, vector-(any intervening process)-matrix, and/or other term and/or operator forms for expressing these equations are matter of implementation for various embodiments of the invention. Specific choices of model form discussed herein are exemplary of the principles discussed in connection with them, and not intended as limiting. For example, a model with different assumptions and/or with assumptions which allow for easier calculation and/or better match to sensing and/or treating options, may be selected. At least in some embodiments, a non-linear model is preferred as it may be more precise. In other embodiments a linear model may be preferred due to a potentially greater easy in ensuring stability and/or analyzing the model.

In some embodiments, cancer clones are measured, and clear identification of their nature, count and/or the like is provided, for example as described herein. In some embodiments, methods which do not rely upon obtained antibodies are used. However, once antibodies for cancer clones are identified, other estimation methods are possible, such as, for example:

1. Multi-frequency conjugate magnetic beads, allowing MRI imaging.
2. Needle biopsy and estimation of cancer state via standard *ex vivo* techniques.

Monitoring time scales can be estimated by analysis of matrix $A$'s eigenvalues or through other standard control and system theory techniques. Gross estimation is optionally performed according to the following example:

In an exemplary embodiment, 6 months is chosen for a major time scale. Treatment is available, for example, after around 3 months, so as to treat essentially the same cancer clones sampled, and/or before further progress substantially occurs. Iterative treatment cycles, including individual dosing, proceed thereafter in the time scale of one month. Preferably, measurement is performed, by the Nyquist criterion, at least twice as fast as the time scale of the manipulation, so as to essentially completely determine a progress of the pertinent cancer. Therefore, measurement by either method described above should be performed every two weeks. These time scales are given to assist in the understanding of the relationships of scales with one another. Optionally the time scales described are adjusted proportionately according to a major time scale of, for example, 3 months, 4 months, 5 months, 8 months, 10 months, 12 months, or another greater, smaller, or intermediate timescale. In some embodiments, the timescale is adjusted according to a measurement (for example, of a rate of change, a frequency of change, and/or a frequency component of a rate of change). In some embodiments, a model comprises one or more timescale calibration factors which are adjusted by estimation and/or according to measurements.

The spatial sampling resolution requirement, namely the number of samples to be taken in a single sampling session, is defined, in some embodiments, by the status of the tumor. For example, a solid tumor has less variance, and consequently calls for fewer samples. Stopping criteria for the number of samples are obtained, in some embodiments from models of variance in the clone colony, and/or through considerations relating to the lethality of sampled clones. For example, if the patient situation can be mitigated, in a high enough probability, by attacking a small number (such as 2-3) of highly dominant clones, sampling is optionally stopped.

Linear, patient-specific therapy may be formalized as follows:

$$\vec{x}_{n+1} = A \cdot \vec{x}_n - B \cdot \vec{u}_n$$

Equation 3

where $\vec{u}_n$ is the control vector, interpreted here as therapy, namely-antibody cocktail ingredients, with or without conjugation, and $B$ is the response of the patient and/or the cancer to the therapy.

The "minus" sign of matrix $B$ reflects that its components are expected to be positive, and that the dynamics of the cancer clones are expected to be negated by the influence of the cocktail.

**Relationships of Initial Model State to Laboratory Data**

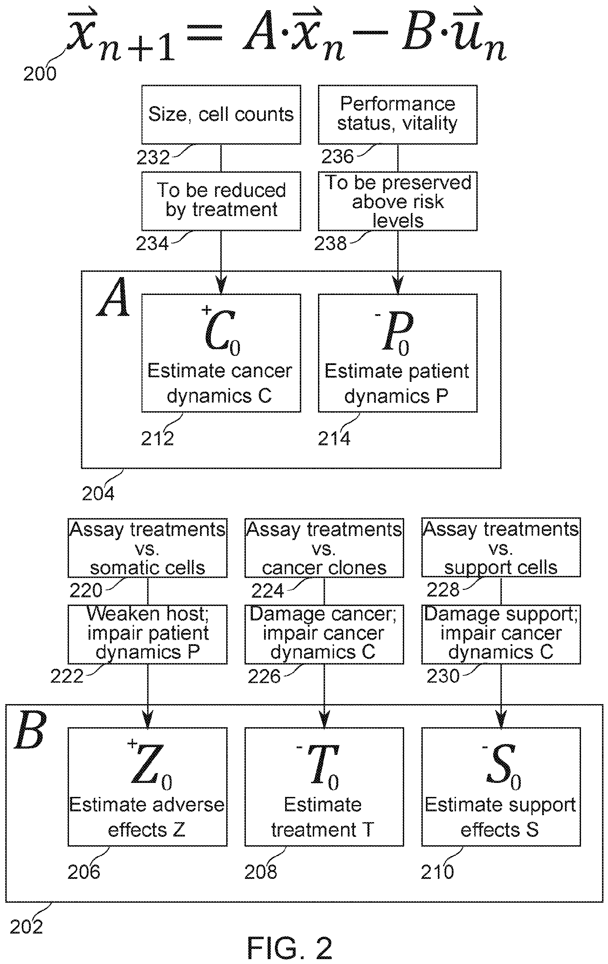

Reference is now made to *Figure 2,* which schematically relates model term estimates to available laboratory data in the initial determination of a treatment composition, according to some exemplary embodiments of the invention.

In some embodiments of the invention, the terms of *Equation 3* (repeated in *Figure 2* as equation **200**) are related to available data as just described, and/or as described in summary in *Figure 2.* It is to be understood that the language and representation of matrix and vector is chosen for convenience of exposition, to describe concepts which more generally underlie modeling of cancer dynamics and control. In some embodiments, other forms of representing cancer state, patient state, treatment effect, and other terms as described hereinbelow are used. For example, a model, in some embodiments, comprises relationships extracted without explicit matrix modeling by techniques of machine learning. In some embodiments, elements of the model are represented and/or manipulated in the form of lists, arrays, database tables, or other structures appropriate to computational manipulation. In some embodiments of the invention, the general case of a matrix is reducible to a more mathematically tractable matrix subtype (for example, a diagonal matrix), a scalar, vector, or another form. Potentially, this allows a more convenient reduction to practice of the general method. Examples of such substitutions and potential justifications for them are provided hereinbelow.

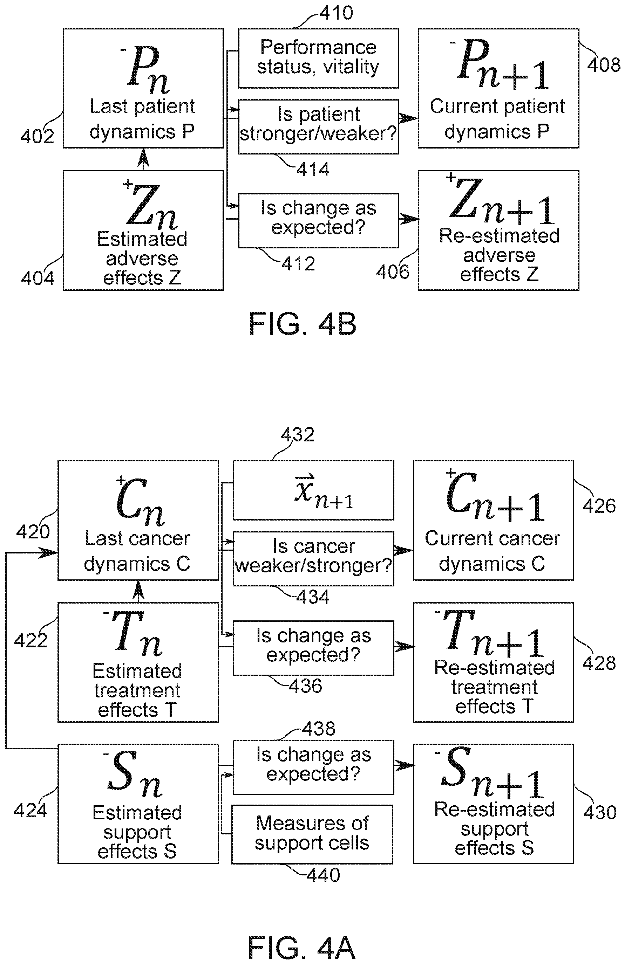

Matrix $A$ **204** can be considered as representing the natural (untreated) evolving tendency of the disease course. Optionally, it is the time-local, linear approximation of the disease dynamics; for example $A_0$ initially, and dynamically changing through subsequent states $A_1 \ldots A_n$. In its initial state, this disease course, as already mentioned, can be itself partitioned, as convenient, into the dynamics of the cancer itself $C_0$ **212**, and the dynamics of the patient $P_0$ **214**. In *Figure 2,* the "+" and "-" prefixed superscripts used with matrix identifiers indicate matrices which tend to contribute to cancer growth, or to inhibit it, respectively. The dynamics of the cancer can be thought of as "the inherent tendency of the cancer to grow", and the dynamics of the patient are "the tendency of the patient's defenses to repress the cancer growth". Each of these comprises several unknowns, and indeed, the line between them potentially arbitrary even if all such unknowns were removed. However, value for making some form of initial partition is found in allowing the useful assignment of later findings of change (as expected, or different than expected) to particular model expectations. This is described, for example, in relation to *Figure 4*, hereinbelow, which describes application of adjustments to matrices between iterations $n$ and $n + 1$.

Some examples of inputs useful in assigning the initial states of these matrices are described hereinabove. Additional examples are described now in relation to the elements shown in *Figure 2.*

For cancer dynamics matrix **212**, the size and cell counts **232** of various cancer cells are one form of input to its estimate. Where there is some lag between cancer detection and treatment onset, the estimate of cancer dynamics can potentially be refined by measuring such parameters at a plurality of pre-treatment time points. Additionally or alternatively, the dynamics are estimated, for example, based on single-time point cancer metrics, plus estimates of relative rate of growth expected for the cancer type in general. While the primary target of treatment, in some embodiments, is the cancer vector $\vec{u}_n$ itself, it is also advantageous for the cancer dynamics **212** to be reduced as described by block **234,** insofar as this allows a greater relative degree of control, for example, by use of treatment matrix *B* **202.** Typically, a shrinking cancer vector $\vec{u}_n$ will correspond to a reduced magnitude of the components of $C_0$ as well.

The initial estimate of the patient dynamics **214** is, in some embodiments, simply by assignment of an arbitrary initial value, such as an identity matrix operating on the cancer dynamics **212.** Using this method of assignment, the whole of the dynamics **204** are initially mathematically attributable to the cancer. Alternatively, some other division is used; for example, a notional patient of normal healthy strength is assigned the identity matrix, while a less-healthy patient is assigned a matrix of weakened magnitude. In any case, the initial assignment of matrix magnitude still potentially sets the relationship between patient dynamics **214,** and patient performance status, vitality, or other patient status parameters **236.** As data distinguishing individual effects of patient dynamics on the components of vector $\vec{u}_n$ are unlikely to be available, particularly at an early stage of treatment, $P_0$ is modeled, in some embodiments of the invention, as a scalar value modifying $C_0$. Despite this simplification, an advantage of considering patient dynamics **214** as a separate term of the model is to provide a focus for criterion **238**: that the patient status be maintained at a sufficiently high level for treatment to have a chance of success.

A therapy responsiveness matrix used in some embodiments of the invention *B* **202** is initially composed, for example, of estimated responsiveness matrices $Z_0$ (adverse effects dynamics **206**), $T_0$ (therapy dynamics **208**), and/or $S_0$ (support cell treatment dynamics **210**). Therapy dynamics **208**, in some embodiments, represents the direct responsiveness of the components of cancer-describing vector $\vec{u}_n$ to application of a therapy cocktail. In some embodiments, this direct responsiveness is estimated, for example, based on assay results for therapy treatments available *in vitro*: a therapy (one or more antibody types, for example, optionally conjugated to one or more conjugation agents) per cancer clonal cell line.

The estimated therapy dynamics **208,** in some embodiments, comprises a version of the assay results **224,** scaled from the measures of the assays to convert to the effect level **226** expected on the cancer metrics used to describe $\vec{x}_n$, and/or on the cancer dynamics *C*.

In some embodiments of the invention, another treatment dynamic matrix component includes adverse effects dynamics **206** ($Z_0$), which describes treatment effects **222** on the somatic cells of the patient uninvolved in specific support of the cancer, as estimated based, for example, on assayed treatment effects **220** on somatic cells. In some embodiments of the invention, another treatment dynamic matrix component includes support cell treatment dynamics **210** ($S_0$), which describes treatment effects **230** on non-cancerous cells recruited into providing support of cancerous cells, as estimated based, for example, on assayed treatment effects **228** on support cell types found within the tumor.

**Control of Patient Vitality**

Herein, some of the description of the modeling of dynamics and control effects concentrates on the design of the control mechanisms for cancer dynamics as such-the aspect of the patient/disease system which has escaped control. However, in some embodiments of the invention, control is considered as being exercised simultaneously on two vectors: disease (cancer) vector $\vec{x}_n$, according to a disease control vector $\vec{u}_n$ (as already outlined); and patient performance status vector $\vec{y}_n$ (comprising vitality and form to the therapy equation of *Equation 3,* may be stated as follows:

$$\vec{y}_{n+1} = A' \cdot \vec{y}_n - B \cdot \vec{u}_n$$

Equation 3B

Optionally, patient status and disease status are both vectorised and on different bases, and there are distinct control vectors and/or dynamics matrices used for *B* and $\vec{u}_n$. In some embodiments, a single vector includes components of both disease state and patient state.

In some embodiments, the main difference between the reduction of *Equations 3* and *3B* to practice is that the goal associated with *Equation 3* is to eventually minimize $\vec{x}_{n+1}$, while the goal associated with *Equation 3B* is to *prevent* minimization of $\vec{y}_{n+1}$, which in the extreme case corresponds to death. For many treatments, there is a necessary through the application of treatments which potentially affect both cancerous and somatic cells. Thus, it is to be expected that $\vec{y}$ will be reduced during treatment; the requirement, however, is that it not be reduced to a level corresponding to irrecoverable and/or intolerable levels of vitality.

It can be seen that patient dynamics matrix *P* and patient performance status $\vec{y}_n$, though related-and potentially, measurable from the same and/or largely overlapping data-serve formally distinct roles in such a dual-goal model. As can be appreciated, in some embodiments, there are more than two goals, for example, also incorporating cost consideration as a goal (and vector components).

Matrix *P* describes the ability of the patient's body to fight cancer; $\vec{y}_n$ describes patient vitality as such. Nevertheless, in some embodiments, control of patient vitality (though maintained vitality itself is obviously critical for the success of therapy), is not separately modeled from how vitality affects the control of the cancer.

Instead, the cancer-control model of *Equation 3,* considered as including the partition $A = P \cdot C,$ or another partition function such as $x_{n+1} = A \cdot x_n + P \cdot \text{patient}_n$, can be considered to implicitly account for patient treatment risk, with the status of the patient dynamics matrix *P* serving as a stand-in for a performance status vector $\vec{y}_n.$ This is justified, for example, by noting that the effectiveness of disease-fighting patient functions such as those of the immune system is gated by the vitality reserves of the patient. Reducing $\vec{x}_n$ will generally entail preserving the effects of *P*; and so long as *P* is effective, $\vec{y}$ can be considered to exceed an acceptable lower limit.

This being the case, the following discussion of the algorithmic basis of cancer dynamics control in some embodiments of the invention focuses on the reduction of the cancer vector. Nevertheless, it should be understood that the same considerations apply in some embodiments, changed as necessary, to the control of patient vitality. For example, a treatment is designed as described hereinbelow, but validated against predicted effects on $\vec{y}_n$ before it is administered.

It should also be understood that, in some embodiments, determination of patient state, for the purposes of risk considerations, is optionally without the use of modeling. Additionally or alternatively, modeled solutions for disease control are validated against judgments of patient state, and/or inputs/parameters of the disease control model are adjusted *ad hoc* (for example, a smaller target magnitude of control vector $\vec{u}$ is set) so that treatment solutions produced are compatible with separately determined judgments of permissible treatment dosages.

In some embodiments of the invention, a treatment strategy comprising dual goals of ensuring patient safety while optimizing for target treatment strength is followed according to the following description.

In an initial round of treatment, a relatively low dose of a treatment component (for example, an antibody or antibody conjugated to a treatment agent) is delivered. The relatively low dose comprises, for example, a dosage which is expected, based on assay information such as preliminary *in vitro* results, to be low enough to avoid severe adverse effects. Preferably, the low dosage is also high enough so that it is expected to produce measurable effects on the cancer state, the patient state/dynamics, or both. Optionally, the low dose is selected to be below predictions of the most effective dose for treatment of the cancer. Optionally, the low dose is selected to be the lowest dose which is predicted to have a measurable effect *in vivo*, based on available assays, such as *in vitro* assays against target cell and/or somatic tissue types.

In some embodiments, adverse effects and/or treatment effects (after treatment administration) are determined based on further sampling/testing-for example, effects on cell counts, patient vitality, or other direct measures of the effects of treatment administration. In some embodiments, distribution of antibody binding is imaged (a portion of delivered antibodies are optionally tagged for this purpose), and the concentrations found in the target cancer and/or non-target somatic regions used as a proxy for likely therapeutic and/or adverse effects.

In some embodiments of the invention, the cycles of treatment optionally enter a positive feedback phase. In the positive feedback phase, dosage is increased-in one or more steps-to dosages which early results allow predicting to be still within safe limits of administration.

**Design of Treatment**

An aspect of some embodiments relates to providing and determining a procedure which uniquely defines $\vec{u}_n$ in each consecutive treatment stage (each n in the formulae). For simplicity of presentation, one example of a design criteria of $\vec{u}_n$ is described here; in the asymptotic limit of a successful treatment, the measurements of cancerous clones, namely $\vec{x}_{n+1}$, should vanish: $\lim_{n \to \infty} \vec{x}_{n+1} = 0$

he algorithm presentation given here is developed from more general-but more difficult to implement-assumptions through a train of simplifications leading to a point at which initially available patient/treatment/cancer information (based, for example, on cell culturing assays and other patient status information) is sufficient to supply the terms of the algorithm, bootstrapping the treatment process. In some embodiments of the information, the algorithm in actual implementation reverses the order of presentation, as increasingly well-defined information about the interactions among treatments, patient, and cancer becomes available.

From the assumed goal condition $\vec{x}_{n+1} \approx 0$:

$$0 = A \cdot \vec{x}_n - B \cdot \vec{u}_n$$

Equation 4

$$B \cdot \vec{u}_n = A \cdot \vec{x}_n$$

Equation 5

$$\vec{u}_n = B^{-1} \cdot A \cdot \vec{x}_n$$

Equation 6

Assuming stability (see below), treating the patient with a cocktail composed as per *Equation 6* the therapy will, in some embodiments, reduce the cancer vector to essentially zero. From a control-theory point of view, this is a naive regulator design. Reasons for elaboration of the naive design, in some embodiments, arise from, for example one or more of:

- the assumption of stability is insufficiently held to in the system;
- some terms of the equation are at least partially not observed initially; requiring, for example, further rounds of treatment and/or observation in order to bring about a targeted level of control;
- stochastic or pseudo-stochastic processes potentially influence one or more of the model terms;
- lack of sufficiently strong treatments for a 1-step approach;
- recognition of the limitations of initially available information;
- need to reduce patient risk (aversion to "minimizing $\vec{y}$"); and/or
- preference to reach or maintain a preferred-for example, a relatively stable-balance of cancer components, rather than risk an "escape" of the cancer into an unfamiliar and/or potentially more virulent mode.

The following discusses a variety of structures of *A* and *B*, according to some embodiments of the invention. It should be understood that the equations presented herein are exemplary, explaining how certain principles of control theory are applicable in general to disease treatment using methods of the present invention. It is also noted that some embodiments of the invention, more generally expressed are not limited to a particular type of modeling or control. Rather, the use of modeling (e.g., even in the form of machine learning without an explicit a priori model) and control in a multicomponent system representing cancer (or other immune related disease) provides a structure on which potentially known types of modeling and/or control may be drafted, as applicable to the problem constraints.

Addition mathematical features found in some embodiments of the invention comprise, for example, stochastic modeling, e.g., with term/component/measurement values considered as being within a range of values comprising associated probabilities. In some embodiments of the invention, adjustment of the model comprises adjusting stochastic parameters, for example, the likely range of a value, likely true value of a measurement, or another feature of the model.

Assuming that $A$ is diagonally dominant, and $B$ is strictly diagonal:

$$A = A_d + \varepsilon \cdot A_0$$

$$B = B_d$$

<div align="center">Equation 7</div>

where $A_d$ is the diagonal matrix, $\varepsilon$ is a small parameter, and $A_0$ are the non-diagonal parts of $A$, properly scaled by $\frac{1}{\varepsilon}$, and $B_d$ is a purely-diagonal matrix (see below).

It should be understood that the form of approximation just given is only one example taken from a range of potential methods of approximation constituting elements of some embodiments of the invention, including, for example small-parameter approximations using $\varepsilon$ in different power, and/or approximations wherein a model comprising terms such as $A = A_d + A_k$ ($A_d$ and $A_k$ being optionally of similar magnitude) is decomposed, for example, according to a transform such as the Fourier transform, a cosine(s) transform, or another variation known from the art of mathematical approximations.

Use of the diagonal matrix assumptions corresponds to assumptions which remove or simplify interactions among the various components, and for example, comprises treating increases/decreases of effect due to such interactions as having secondary and/or separately treatable importance. Thus, the structure of $A_d$ is as follows:

$$A_d = \begin{bmatrix} \alpha_1 & 0 & \dots & 0 \\ 0 & \alpha_2 & \dots & 0 \\ \vdots & \vdots & \ddots & \vdots \\ 0 & 0 & \dots & \alpha_N \end{bmatrix}$$

<div align="center">Equation 8</div>

Where $A_d$ accounts for interaction of every cancer clone with itself alone, namely, a separate, non-interacting phenomena, while $A_0$ accounts for interactions.

Similar concepts can be reflected in the structure of $B$: strict diagonality means that each antibody (also referred to herein as an antibody clone) does not interact with others.

$$B_d = \begin{bmatrix} \beta_1 & 0 & \dots & 0 \\ 0 & \beta_2 & \dots & 0 \\ \vdots & \vdots & \ddots & \vdots \\ 0 & 0 & \dots & \beta_N \end{bmatrix}$$

<div align="center">Equation 9</div>

Plugging *Equations 7-9* into *Equation 6*:

$$\vec{u}_n = B^{-1} \cdot A \cdot \vec{x}_n = B_d^{-1} \cdot (A_d + \varepsilon \cdot A_0) \cdot \vec{x}_n$$

<div align="center">Equation 10</div>

$$\vec{u}_n = B_d^{-1} \cdot A_d \cdot \vec{x}_n + \varepsilon \cdot B_d^{-1} \cdot A_0 \cdot \vec{x}_n$$

Equation 11

Neglecting the $0(\varepsilon)$ terms is similar to stronger linearization requirements, for example, shorter time scales. Assuming these stronger requirements are met, the final result is:

$$\tilde{\vec{u}}_n = -B_d^{-1} \cdot A_d \cdot \vec{x}_n$$

Equation 12

Or, specifically:

$$\tilde{u}_n^j = \frac{\alpha_j}{\beta_j} \cdot x_n^j$$

Equation 13

The last equation is a simple, exemplary design algorithm for the antibody cocktail to be determined: the amount of each component in the cocktail should be proportional to the ratio of the importance of the clone to the effectiveness of the antibodies. The "importance" and "effectiveness" are measured for each component and each clone separately, and hence the cocktail is composed of a distribution which may be *different* from that of the clone colony. A highly lethal clone, treated by less effective antibodies, will lead to significantly higher dosage of the antibodies.

It is to be understood that additional limitations/conditions are optionally imposed in some embodiments of the invention. For example, to ensure preservation of a specified ratio $K_{j,k}$ between two clones $j$ and $k$, the following condition should be met:

$$\frac{\tilde{u}_n^j}{\tilde{u}_n^k} = K_{j,k}$$

Equation 13B

and thus:

$$K_{j,k} = \frac{\alpha_j \beta_k}{\beta_j \alpha_k} \cdot \frac{x_n^j}{x_n^k}$$

Equation 13C

Additionally or alternatively-insofar as somatic-cell (adverse) effects $Z$ of treatment comprise global effects on the patient dynamics $P$-a limiting criterion function can be defined which, for example, ensures that the (suitably calculated) aggregate value of adverse effects does not lower $P_{n+1}$ below a threshold. For example:

$$\|(P_n - Z_n)\| > T$$

Equation 13D

for some suitable definition of the norm (maximum, Euclidean, $L_1$, or other).

It should be understood that the above analysis is exemplary of a more general class of analyses in the time domain (as above) or another appropriate transform domain, for example, via a Fourier, Laplace, or suitable wavelet transform; a singular value decomposition, and/or another transform function. Optionally, a dosage is calculated within a transform-space representation comprising, for example, combinations of cell line clones (and/or other epitope targets, and/or binding targets), and antibodies (and/or immunocompounds more generally, and/or targeted drugs more generally). Considered in a transform space, treatment can be considered as targeted against non-healthy modes of the patient's dynamic system, rather than against specific clones as such. In some embodiments of the invention, the dynamic situation does not reduced to a static ratio, but rather to a form such as oscillations in state as a function of time. For examples of how this may occur, it may be noted that there are likely to be mutually influencing processes in the disease/patient/treatment system which lag and/or lead each other-for example, a native immune response potentially develops and wanes with a lag to the state of the disease, while the disease state itself potentially wanes and develops inversely to the state of the native immune response. Another effect potentially contributing to the dynamism of the control system is the likelihood that treatment delivery is pulsed rather than continuous.

If all clones are assumed equally lethal, and all antibodies assumed equally effective, this relation reduces, further, to direct proportionality:

$$A_{d,s} = \alpha \cdot I$$

$$B_{d,s} = \beta \cdot I$$

Equation 14

where s stands for "scalar", leading to the intuitive result $\tilde{u}_n^j \propto x_n^j$:

$$\tilde{u}_n^j = \frac{\alpha}{\beta} \cdot x_n^j$$

Equation 15

By way of example, consider an even simpler dosing variant: the amount of each component of the antibody cocktail is proportional to the amount of the clone it targets. The cocktail can be of about the same distribution of the clone colony, multiplied by ($\alpha/\beta$).

In an exemplary embodiment of the invention, initial conditions are re-estimated as further information becomes available (e.g., for modeling methods where the initial state is reused for different iterations).

In some embodiments, the initial state is (or is mostly based on) the condition at or near the end of the previous state, so that the initial (t=0) state may be less accurately estimated.

**Effects of Treatment and Updating the Model**

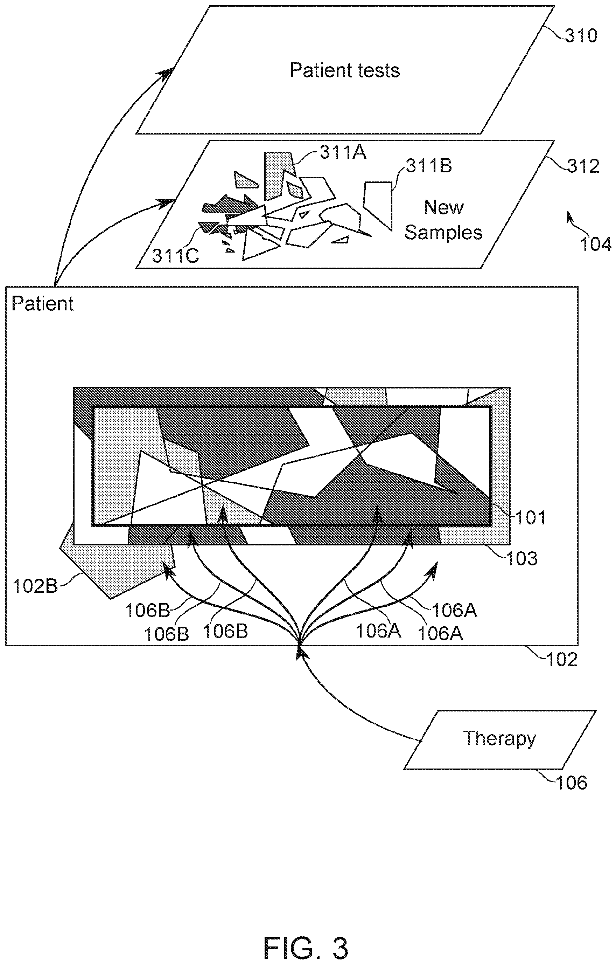

Reference is now made to *Figure 3*, which schematically illustrates the effects of different branches (components having different affected targets) of a treatment composition on different disease cell lines, support cell types, and/or other patient cells, according to some exemplary embodiments of the invention.

At block **106**, in some embodiments, a therapy (for example, an initially determined therapy) is shown for administration to a patient **102**. Upon administration, a plurality of the components of the treatment **106A, 106B** make their way to various points of the body, represented as branching arrows within the schematic representation of the patient. From the standpoint of treatment, the preferred targets comprise cells of the cancer **101** itself, and/or non-cancerous cells **103** which serve direct support functions for the cancer **101**. Shapes found within each compartment suggest variation of cell type and/or cell type quantity; shading found within each compartment suggests differing susceptibility to one or more delivered treatment components. In some embodiments of the invention, adverse effects potentially occur when a treatment component **106B** happens to localize to a somatic cell type, for example, at region **102B.**

The various effects of the treatment are measured by post-treatment tests **310,** for example, imaging tests and/or tests of vital function; and/or post-treatment sampling **312.** In particular, new samples **312** comprise information about the new status of cell populations within the cancer. Highly susceptible population **311C,** for example, may be found to be relatively reduced in quantity compared to insusceptible population **311B;** moderately susceptible population **311A** is intermediately affected.

In some embodiments, based on this information, a new cancer vector $\vec{x}_{n+1}$ is constructed, to form the basis of the next round of treatment decision making. In some embodiments, other terms/representations of the model are adjusted in response to the newly available information.

In general, it is a straightforward process to simply increase/decrease the dosage of a component delivered according to the deviation from expected results. However, the cancer situation is potentially dynamic, and it is a potential advantage, in some embodiments, to abide by rules for maintaining and/or refining the partitioning of effects among the model representations so that changes in this dynamic situation are correctly diagnosed and adjusted to.

For example, changes among the various clones composing $\vec{x}_{n+1}$ may be different than the changes modeled before treatment. There may, at least initially, be insufficient information be fully certain as to whether this is an effect of treatment delivery, cancer dynamic changes, patient dynamic changes, mis-calibrated treatment/adverse effect predictions, or another effect. However, the application of reasonable attribution rules allow the detection of unusual and/or unaccounted-for changes that potentially prompt further investigation and/or treatment development activity.

Reference is now made to *Figures 4A-4B*, which schematically relate model term estimates to available laboratory data in the initial determination of a treatment composition, according to some exemplary embodiments of the invention.

In some embodiments, cancer dynamics **420** represents the cancer dynamics modeled at an iteration *n* to determine a treatment regime, while cancer dynamics **426** represents cancer dynamics modeled at *n* + 1. In some embodiments, the main input to the adjustment is the observed cancer state vector $\vec{x}_n$ **432**. According to whether the cancer is observed to be "stronger" or "weaker" by one or more measures **434** (for example, size, rate of growth, and/or necrotic:viable cell ratio), cancer dynamics **426** are optionally adjusted. The adjustment, in some embodiments, is to an expected (modeled) rate of growth experienced by the cancer in the absence of further treatments, at least for within some interval of time. An optional auxiliary method to calculate treatment-free dynamics more exactly is to make intra-cycle observations without the provision of a new treatment, potentially together with extrapolation of the treatment effect decay-time (treatment effect decay-time can itself be measured directly in some cases; for example, by the imaging of radio label-tagged antibody distributions in target tissue).

In some embodiments, models of direct-cancer treatment dynamic $T_n$ **422** and/or support cell treatment dynamic $S_n$ **424** are expected to have had a certain effect in creating a change in cancer vector (model) $\vec{x}_n$, and/or the state of the cells supporting the cancer (while support cell state is optionally represented as components of $\vec{x}_n$, it is here treated separately for purposes of discussion). At comparison block **436**, insofar as cancer vector effects are similar in quality to those expected, but different in magnitude-particularly if treatment composition has just been adjusted- the difference can be attributed to a calibration issue in treatment efficacy, and corresponding adjustment made to the new treatment dynamic matrix (model) **428.** In some embodiments, similar reasoning potentially applies to measures of support cells **440** matching expectations or not, with adjustment determined at block **438** to the next support cell dynamic matrix **430.**

It should be understood, however, that where relevant data from the history of the treatment happens to be available, it may be apparent that there are interaction effects which allow partitioning the variation from expectation among matrices. For example, if the treatments affecting cancer cells (and thus, the matrix $T_n$ are held steady, but an adjustment made to treatments expected to selectively affect support cells made on the same iteration $S_n$, a corresponding effect on the cancer cell lines is potentially observed. Potentially, this reflects a non-linear interaction of the treatment effects matrix with the support cell reduction (treatment) matrix and/or model.

Additionally or alternatively, in some embodiments, change to the most unaffected population of the cancer serves as a proxy for the "untreated dynamics" of the cancer-though it should be understood that the cancer's inherent dynamics are potentially variable across cell types.

In some embodiments (*Figure 4B*), adjustment is made during a treatment iteration to the modeling of the patient dynamics **402** ($P_n$) and/or the adverse effect dynamics **404** ($Z_n$). For example, in some embodiments, patient performance status and/or other vitality measures **410** are determined to reflect a change in the status of the patient-associated cancer dynamics. In some embodiments, the change, is a potentially expected, or at least, unsurprising change. According to the noted change, for example an increase in performance status, determination (at block **414**) is made to change the patient dynamics at **408** ($P_{n+1}$).

The task of calibrating model change to observed performance change (a task which occurs also in the context of several of the other adjustments described herein), is optionally managed over the course of a plurality of iterations, wherein changes are initially guided by the "sign" of the change (increase/decrease, for example), rules of thumb (for example "change the model value relatively, and inversely according to the relative size of the prediction error"), and/or accumulated experience with similar cases. As treatment progresses, adjustments can be increased or decreased to account for observed effects on control which more immediate experience provides.

In some embodiments, the estimate of adverse effect dynamics **406** ($Z_n$)-originating, for example, in culture assay observations, and/or on observations of somatic cell counts, observed somatic binding levels, or other data-is adjusted, based on dynamics actually observed on the previous course of treatment. For example, if a treatment affects leukocyte count, the adverse effect dynamics **406** ($Z_{n+1}$) are optionally adjusted corresponding to a determination at block **412** that the fractional drop in cell count actually observed is different from that previously predicted.

Other considerations and situations also potentially suggest adjustments to model parameters, and/or to activities at the level of treatment development. In some embodiments, for example, outlier effects of a single treatment component (attributable effects much stronger or weaker than expected, compared to those of the other components) are optionally considered as component-dependent, and adjustment in the model made to the estimated effectiveness of the therapy itself. However, potentially, such a result indicates an undetected change in the clonal population (for example, the arising of a previously undistinguished resistant clone to replace the target clone), leading to a decision for further clone/treatment screening.

To the degree that the unexpected effects of all/most components are correlated, the mis-estimate can be corrected by attribution of the difference to either or both of the cancer dynamics *C* and the patient dynamics *P*. In the absence of previous feedback (for example, after the first round of treatment), the adjustment can be considered as primarily a calibrating adjustment-since it is understood that the initial parameters are based on partial information. Later on in the treatment cycle, a need for readjustment potentially carries with it another potential meaning-that the cancer and/or patient dynamics have actually changed. The ability to sense such state changes by deviations which have not already been explained by the model comprises a potential advantage of the method in general. It allows shifts in strategy based on early detection of quantitative changes, before they convert to qualitative changes such as an externally clear deterioration in the clinical situation.

It should be noted that the use and definitions of A, P and C are only exemplary for some embodiments of the invention and may be changed (or dispensed with), if different modeling is used. For example, A=P+C can be meaningful as a large matrix with P and C on the diagonal and cross terms (e.g., patient-health and cancer development) elsewhere. In this example, the patient vector and the tumor vector are combined into one large vector, and make the goal function and constraints some (different) function of it.

In another example, A=P*C is used to model a case where at least some vector components include a health dimension and a disease dimension. Optionally, the dynamics matrix is decomposable into multiplication of two clearly defined matrices. Optionally, the goal function and the constraints use not only the state vector but also the dynamics matrix.

In another example, A=C1*P*C2, where, for example, C1 is the transpose of C2, or the transpose of its complex conjugate.

## Iteration of the Model

Returning now to the mathematical development of the model, the consecutive levels of simplification in the terms of the model are reflected in reverse by the following flow:

1. Identification and matching of clones and antibodies, corresponds to the linearization, for example of *Equation 3*. The terms are initially approximated with simple assumptions, then with increasing sophistication as more information becomes available. In some exemplary embodiments of the invention, one or both of stochastic modeling and inclusion of measurement error terms, and/or other methods known in the art of modeling, are used.

2. Patient dynamics matrix *A* and antibody effectiveness (treatment) matrix *B* are optionally first determined by a combination of limited available initial data and other assumptions (*Equation 15*), treated by the cocktail design of *Equation 15*, then refined through treatment iterations. This moves up the simplifications process, to *Equations 8* and *9*, treated by the distribution of *Equation 13*, followed by full estimation of *A* and *B*, and the use of *Equation 6* to design the cocktail.

3. As the patient/cancer system is not linear, full, final treatment is essentially not included in the model: mathematically, this is a description of a continuously treated chronic condition.

4. Main system level impact: the model requires constant estimation of the state vector reflecting the cancer. As new data is provided, the model is preferably adjusted.

Overall, the modeling and treatment method may optionally be characterized as comprising parallel loops: in one loop, the clones are identified, antibodies are obtained, treatment is determined and performed with the antibodies, and the state estimation of the state of the patient and of the cancer is performed, after which treatment is optionally performed again, after adjustment of the dosages at which identified treatments have been provided. In an outer (slower), second loop: after treatment is performed the method returns to the start of the first loop, namely identifying the clones and obtaining antibodies. This picture corresponds also in general to the interacting loops of *Figure 1B*, hereinabove.

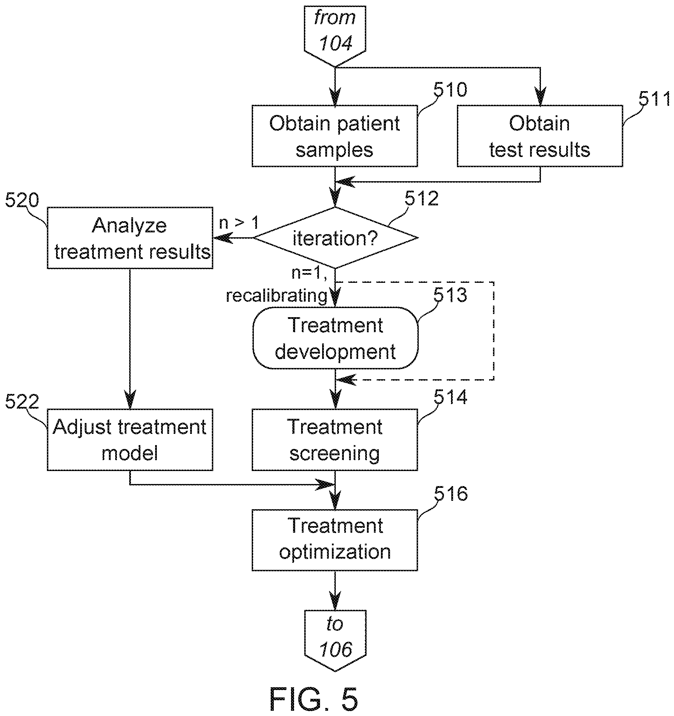

Reference is now made to *Figure 5*, which is a schematic flowchart of interaction between data inputs-including treatment effectiveness/adverse effect measurements, disease state, patient performance status-and treatment determination/adjustment, according to some exemplary embodiments of the invention.

*Figure 5* comprises another view of key portions of the "parallel cycles" concept. The flowchart begins at blocks **510** and **511** with the receipt (for example, from block **104**) of patient samples and/or test results reflecting the treatment status after a most recently completed treatment administration-or, on a first iterations (n=1), the samples/tests provide the state of patient and cancer as initially presented.

At block **512**, in some embodiments, the process branches, or potentially forks, depending on the character of the present treatment cycle. During the first cycle, and at cycle intervals normally greater than 1 cycle thereafter, work is done at block **513** to develop treatment options, for example, based on antibody-producing hybridomas derived from B cell samples of the patient. New development at block **513** is optional after the first cycle. For example, re-development of treatment options is optionally scheduled when it is observed that the clonal cell types in the cancer are changing their previously established responsiveness to the available treatments.

In some embodiments, treatment screening is carried out at block **514**. This can include re-screening of treatments already in use against the current set of cancer clone lines, to verify continued efficacy. Optionally, re-screening is performed to identify which of perhaps several previously developed, but initially unimportant hybridoma lines should be brought into the current treatment mix. This can be because of success in eliminating other cell lines, leaving room to focus on a new "most wanted" candidate. Additionally or alternatively, a previously unimportant cell line begins to multiply, requiring adjustments to control.

For iterations beyond the first (n>1), other activity optionally proceeds in parallel with the work of development and screening. At block **520,** in some embodiments, the test and samples are viewed as treatment results for analysis. Analysis is, according to rules, understandings, and algorithms, for example as described hereinabove. At block **522,** in some embodiments, the analysis is converted into adjustments to the treatment model, such that the treatment model more accurately accounts for the treatment results actually obtained-and, accordingly, will potentially more accurately predict the results of the next treatment round.

At block **516,** in some embodiments, the adjusted model is converted to a new treatment, which is "optimized" in terms of available data, modeled parameters, and assigned goals, and prepared for administration to the patient according to block **106.**

The parallel-stream examples of *Figures 1B* and *Figure 5* illustrate potential advantages of some embodiments of the current invention with respect to rapid treatment development and adaptation. In particular, the development and/or screening streams of the treatment cycles allow a broader approach to treatment adaptation than iterative tuning based on feedback from the patient alone. In effect, part of the process of trial-and-error is parallelized. From the standpoint of algorithm implementation, this allows the limited number of critically informative actual treatment cycles available to be used for the evaluation of treatments already selected and given preliminary evaluation from among many alternative treatments and/or dosages. Another perspective is that the development and/or screening streams can evaluate many options at a coarse level, while actual administration provides information about how promising options should be adjusted for best effect. Naturally, it is also understood that it is a potential advantage to begin with treatments already pre-screened for likely success.

The occurrence of actual treatment delivery in parallel with ongoing treatment delivery also provides the opportunity for validating the hypotheses of the coarse evaluations. More rapid understanding that a promising treatment is not having a predicted effect allows more quickly shifting resources to another treatment option. Understanding the *way* in which a treatment is defying expectations (below-assumed target penetration, above-assumed adverse effects) potentially guides this shift. For example, discovery of below-assumed target penetration potentially focuses attention to dosage level and/or delivery method. Above-assumed adverse effects (or the threat thereof) potentially guide attention to antibodies having lower cross-reactivity. A model which allows both setting and verifying clear initial expectations for these properties (within the rapid portion of the treatment cycle) has the advantage of making deviation from such expectations easier to detect, and to potentially correct by guiding efforts within the slow portion of the treatment cycle.

From another perspective, the process of the method can be considered as comprising artificial augmentation of normal immune processes, including control properties. Specific binding agents (antibodies) are not only developed, amplified, and potentially augmented (for example, by conjugated agents), they are also intelligently screened to avoid amplification of the wrong antibodies-and then, with uncertainty still potentially remaining, the dosage itself continues to be adaptively adjusted, along with individual treatment components entering and leaving the cycle according to need and/or availability. Adaptive control mitigates some of the risks of immunotherapy, by providing control functions which the immune system-while otherwise a highly controlled system in its own right-cannot provide.

**Method Variations**

The method presented above optionally uses an adaptive algorithm, allowing convergence of the estimation, and designed to work under essentially zero-mean measurement noise. Suitable adaptations to the algorithm may allow faster convergence, or a different choice of goal attainment.

Simple convergence/divergence criteria may be selected in order to guide successive iterations of the above loops to a desired goal (namely treatment of the patient so as to result in cessation or at least stasis of the malignant cells): As the vector *u* is a function of *x*, the linear dynamics may be reduced to a function of *x*, as shown in *Equation 11*. It should be understood that non-linear interactions are potentially modeled and/or more complicated convergence/diverge criteria selected according to the totality of information available about the state of the disease. The model given here is an example which illustrates principles by which a model is be guided to a selected goal in some embodiments of the present invention.

Hence, inserting *Equation 11* to *Equation 3*:

$$\vec{x}_{n+1} = (A-R) \cdot \vec{x}_n = (A-R)^2 \cdot \vec{x}_{n-1} = \cdots = (A-R)^n \cdot \vec{x}_1$$

$$\text{Equation 16}$$

Non-divergence criteria:

$$\|A-R\| < 1$$

$$\text{Equation 17}$$

It should be stressed that this example relates to a temporary local model. It may be altered to suit continued evolution, spontaneous appearance of new cancer, etc.

However, if the untreated cancer growth accelerates, $\|A\| > 1$, then for effective therapy, the treatment matrix *R* must counteract this situation. Chemotherapy, for instance, may be viewed as direct reduction of the cancer state vector $\vec{x}_{n+1}$, at the cost of seriously impairing the patient matrix *A*. In this situation, the treatment matrix *R* may be, at times, even counter-productive.

The standard situation, in which a patient with cancer is treated by surgery, chemotherapy, etc., up to a point in which cancer is practically cured, only to restart again from near non-existence, is modeled in *Equation 16*: if $\|A-R\| > 1$, an arbitrarily small perturbation will grow and diverge, leading to deterioration or even death even though the initial treatment succeeded.

The model described above has the general benefit of working well within the toolset of adaptive linear control systems theory, which is an effective way to describe, monitor and control a complex, unexpected system. It is noted that other models, especially non-linear models and models generated by machine learning may be used in some embodiments of the invention.

For example, in some embodiments, there is provided a method for specific cancer therapy, the method comprising:

(a) identifying B cells committed to different antigen types which characterize the pertinent cancer;
(b) producing a plurality of hybridomas from the identified B cells;
(c) producing an antibody cocktail from the hybridomas and administering the cocktail to the patient, wherein an amount of each antibody in the cocktail is proportional to a ratio of the relative quantities of a cancer clone associated with the antibody to an estimated effectiveness of the antibody;
(d) repeating step (a), and

if a B cell committed to a new antigen type is identified, repeating steps (b) and (c);
if no B cell committed to a new antigen type is identified, repeating step (c).

In certain embodiments, a frequency at which step (a) is performed is twice higher, according to the Nyquist criteria, than a frequency at which step (c) is performed.

Another revision to the algorithm presented, present in some embodiments, is to include provision for particular caution with respect to adverse effects. Rather than attempting to wipe out a cancer as quickly as possible, initial treatment is aimed at acquiring the information required to make informed decisions which will take into account measured levels of adverse effects, with the anticipation that this will lead to a positive feedback increase in treatment levels to a point which is determined to optimally balance (according to established criteria) safety and effectiveness.

**Exemplary Treatment Courses**

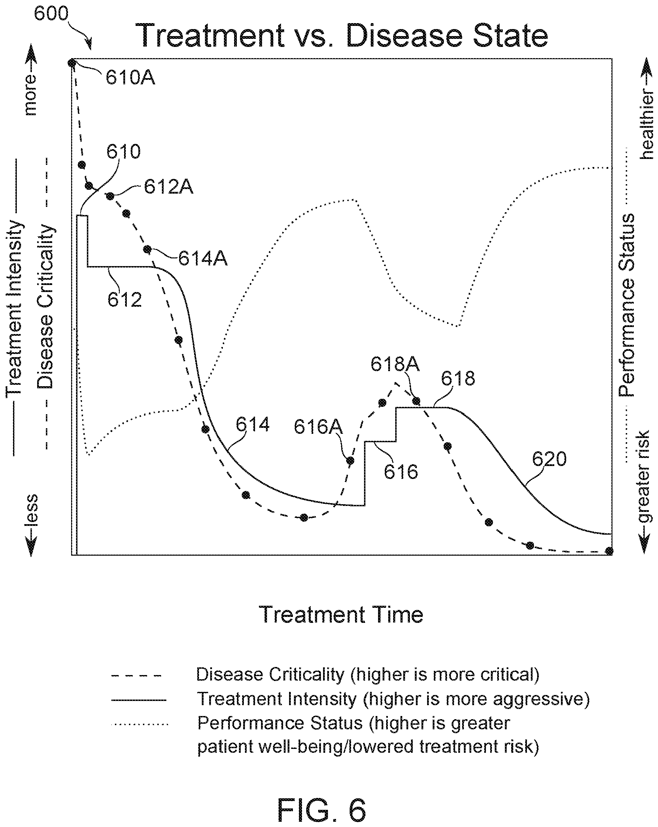

Reference is now made to *Figure 6*, which schematically graphs treatment, disease, and patient events and states during an exemplary time-course of iteratively controlled treatment, according to some exemplary embodiments of the invention.

Graph **600** represents the time-courses of three variables reduced for simplicity to 1-D plots: disease criticality, treatment intensity, and performance status. Black dot represent various assessment points along the time course for which data is available. According to the stage of treatment and/or other factors, the assessment interval is, for example, a few days, a week, two weeks, three weeks, or another greater, lesser, or intermediate period of time. Graph **600** is provided in order to serve as a sequenced example of the operations of various considerations and mechanisms described with the methods of the current invention.

In terms of model embodiments already presented:

- "Disease Criticality" corresponds, for example, to the magnitude of cancer vector $\vec{x}_n$, or to another measure of the vitality/progression of the disease.

- "Treatment Intensity" corresponds, for example, to the magnitude of the treatment vector $\vec{u}_n$. It is assumed for the purposes of this graph that increased treatment intensity generally corresponds to increased levels of adverse effects on the patient status.

- The "Performance Status" graph corresponds, for example, to the notional or actually determined patient status vector $\vec{y}_n$, its "proxy", the patient dynamics $P_n$, or another measure of patient vitality and/or exposure to treatment risk.

Features described with respect to the graph should be understood generally as being features of some embodiments of the invention, including:

- integration of targeted therapy types with other therapeutic modalities;
- balancing of patient performance status with therapy intensiveness, for example by reducing therapy levels according to cancer response;
- monitoring for the appearance of new clonal types;
- development of new therapies in parallel with ongoing modification of exiting therapy dosage; and/or
- entry of therapy into a maintenance mode, or terminating therapy, as therapy reaches a successful stage.

The timeline begins at sampling time **610A,** with disease criticality high, patient performance status intermediate, and treatment not yet begun. In some embodiments, initial treatment occurs during interval **610,** for example, with traditional treatment options such surgery or radiation therapy; the intensity of these treatments is high, and the effects strong on weakening both cancer and patient performance status. Patient-specific (antibody-derived) treatments are in progress at sampling time **612** with initial success being apparent by the drop in disease criticality between sampling times **612A** and **614A,** with disease state continuing to drop. However, there is potential cause for concern in the observation that the performance status of the patient is not returning to a high baseline value.

As a result of treatment changes made after sampling time **614A,** the treatment is graded to become less aggressive. Since it is found that disease criticality continues to decline despite this reduction, further reductions in treatment intensity are scheduled, through interval **614,** with corresponding increases in patient performance status. At sampling time **616A,** however, obtained samples/tests indicate initiation of a rebound of the cancer. This can be for one or more of several reasons, such as, for example: the treatment falling below a level required for suppression, a new cancer clone developing, and/or an existing clone being released from suppression by co-occurring cancer clone lines.

As treatment has been changing only slowly, a suspicion that the change represents a new cell line breakout is investigated by reactivation of hybridoma development and screening efforts. By interval **616,** levels of administration of a currently available antibody have been increased, but it is not clear that the new cancer state is strongly affected. Once again, patient performance status is adversely affected. By sampling time **618A,** a new treatment component has been screened and supplied to the patient. Progress of the breakout clone has been arrested, and follow-up during interval **618** shows a significant drop. To alleviate concern for patient status, the treatment intensity is again lowered, while continued tracking, for example through interval **620,** shows that the cancer continues toward full remission. At the end of the graph, disease criticality has been reduced to almost nothing, while treatment intensity has reached a low level suitable for control of the residual cancer, and/or for eventually wiping out the residual cancer entirely. Patient performance status has returned to a high level.

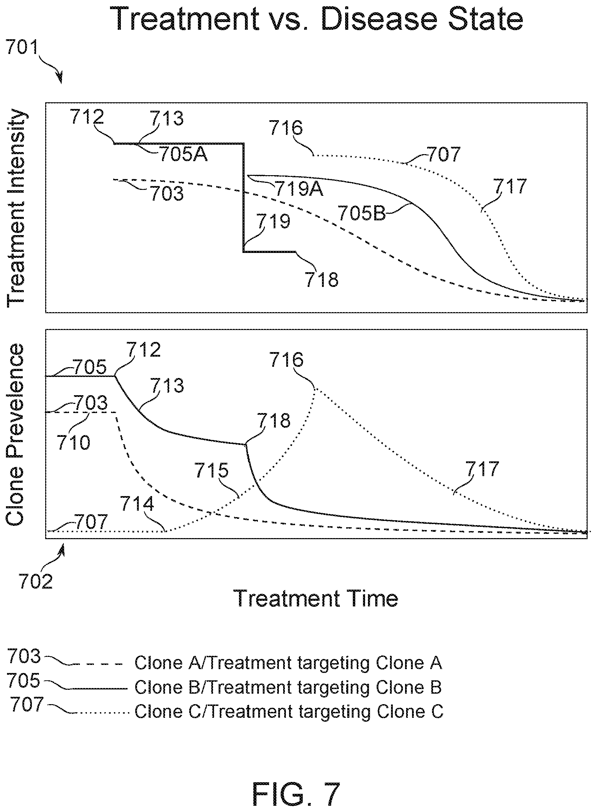

Reference is now made to *Figure 7*, which schematically illustrates adjustments and additions of treatment components in response to clonal cell-type population prevalence during an exemplary time-course of iteratively controlled treatment, according to some exemplary embodiments of the invention.

In some embodiments of the invention, disease control is aimed at one or two most prevalent, or otherwise most important (for example, most malignant) clonal cell lines. Potentially, this saves resources; but it also potentially directly assists the goal of obtaining stable cancer control, since fewer variables are in play at any given time. The potential risk inherent in not attacking all the cells at once is at least partially offset by detailed monitoring of cancer status during treatment, such that if a clonal cell type does happen to "escape", this can be determined and correction made.

Features and concepts described with respect to the graphs should be understood generally as features of some embodiments of the invention, including one or more of:

- targeted choice of clonal populations for therapy;
- dynamic re-prioritizing of targeted populations during therapy;
- dynamic development of new clonally-selective therapies during the course of treatment: for previously ignored cell types, for new cell types, and/or to improve on available therapies for existing cell types;
- deliberate under-delivery of therapy for the sake of maintaining patient well-being; and/or
- therapy transitioning to verify continued treatment effectiveness before discontinuing a previous treatment.

Graph **702** indicates clone prevalence vs. time on arbitrarily scaled axes; graph **701** indicates treatment intensity. In graph **702,** dashed line **703,** solid line **705** and dotted line **707** represent prevalences of clones A, B, and C, respectively. In graph **701,** dashed line **703,** solid lines **705A, 705B** and dotted line **705** represent treatment intensities for antibody-derived treatment components specific for clones A, B, and C, respectively. 705A and **705B** represent two different antibody treatments for clone B.

After a pre-treatment period **710** of treatment development and screening, an initial treatment mix is delivered at time **712,** comprising antibody components directed at clones A and B. During period **713,** treatment of clone A is relatively effective, and the level of treatment **703** is gradually tapered off as levels of clone A drop. Suppression of clone B, however, is only successful in part-perhaps due to weak binding, or alternatively, due to restrained use, for example, in order to reduce adverse effects.

At time **714,** clone C, previously ignored by treatment, begins to rise in prevalence. The rise is due, for example, to release from inhibition by the declining health of the main clone populations, and/or to the appearance of a variant of clone C (C') with increased virulence. During the rise of clone C prevalence **715,** the change in populations is noted, and development of a treatment to meet this threat is begun.

In the meantime, at times **719** and **719A,** treatment begins transitioning to a new treatment option for clone B. At first, treatment **705A** is reduced but not entirely stopped, while the effects of treatment **705B** are judged. Once evidence suggests that treatment **705B** is carrying the weight of continued reduction of clone B, delivery of treatment **705A** is optionally discontinued at time **718.** The effectiveness of treatment **705B** allows it to be tapered off as the prevalence of clone B lowers.

Returning to clone C, a treatment is finally ready for delivery at time **716.** Treatment begins aggressively, for example, due to concerns about the rapid rise of this clone in the population. Treatment is, however, successful, allowing drug delivery to be tapered off significantly by the end of the treatment period described.

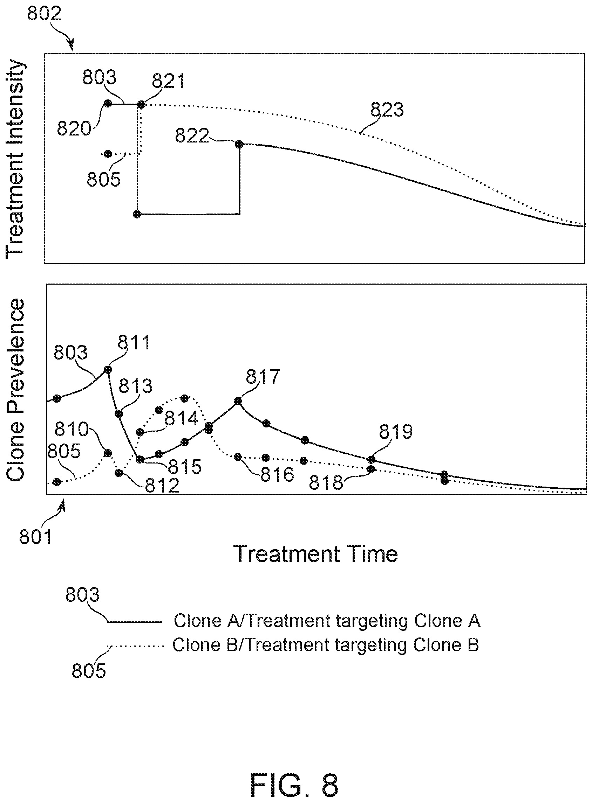

Reference is now made to *Figure 8*, which schematically illustrates adjustments of treatment components to take advantage of clonal cell-type mutual suppression during an exemplary time-course of iteratively controlled treatment, according to some exemplary embodiments of the invention.

A situation which potentially arises during cancer treatment is that an initially minor cancer clone turns out to be held in check by co-existence with other clone types. For example, the tumor microenvironment is potentially limiting in a resource (such as a growth factor or nutrient) for which a suppressed clone type is a poorer competitor. Upon release from competition, the suppressed type itself potentially multiplies to become predominant, and a target for treatment. In some embodiments of the invention, possible situations of competitive inhibition are observed and/or inferred from one or both of *in vitro* and *in vivo* effects, and therapeutic dosage adjusted accordingly to prevent this form of escape from therapeutic control.

Graph **801** of clone prevalence vs. time includes clone A time course (solid line **803**) and clone B time course (dotted line **805**). Corresponding treatment intensity time courses are shown in graph **802.**

Treatment begins with clone A at sample point **811** dominant over clone B at sample point **820.** Optionally reflecting a corresponding difference in treatment priorities, Clone A treatment begins at time **820** with a somewhat higher intensity than treatment of clone B. The strategy seems to be succeeding initially, with clone A at sample point **813** and clone B and sample point **812** both showing initial reductions in prevalence.

Sample point **814** of clone B however, indicates a reversal in this trend, while clone A at sample point **815** has continued to reduce. A possible explanation is that clone B has been released from competition by the significant clearance of clone A from the population. At time **821,** it is decided to alter treatment intensities: now the treatment for clone B is more intense, while treatment of clone A is sharply reduced in intensity. As a result, the population of clone A begins to rise again through a period leading up to sample point **817.** The population of clone B is not immediately reduced, but its rise is slowed, then reversed by sample point **816.**

With parameters of control now better understood, treatment intensity is raised again for clone A. By sample points **818** and **819,** it appears that both clones are under control. Treatment levels are allowed to continue tapering off from time point **823** as the cancer approaches remission.

**Patient Cell Samples**

According to certain embodiments, specimens of the patient's lymphocytes and tumor cells are obtained and used in the methods of the disclosure. In some embodiments, the methods of the disclosure include obtaining B cells from the patient. The methods herein are described in particular with respect to B cells. However it should be understood that aspects of some embodiments of the invention (in particular, aspects of modeling, model updating, and treatment design based on currently modeled state) are applicable also to immunocompound derived from other cells of the immune system, for example, T cells.

In certain embodiments, the B cells obtained from the patient are e.g. mature, memory or plasma cells. According to a particular embodiment, the patient may be sensitized to tumor-specific antigens, e.g. by immunization with inactivated self-tumor cells.

In some embodiments, the B cells may be obtained as different fluid or tissue samples from the patient, e.g. from peripheral blood, tumor tissue, or lymphatic fluid or tissue, using methods well known in the art. According to a particular embodiment, peripheral blood lymphocytes are collected. The obtained B cells may be purified or enriched as known in the art, e.g. by density gradient, fluorescence-activated cell sorting (FACS) or magnetic separation. In some embodiments, B cells are obtained by leukapheresis.

In some embodiments, immune system cells are primed for greater activity by one or more procedures occurring before harvesting. For example, injection of killed tumor cells potentially raises the concentrations of antibodies specific for epitopes of the disease within the blood and/or lymph. Additionally or alternatively, radiation therapy potentially promotes immune system access to cancer cell epitopes, also potentially leading to enhanced activity of tumor cell immunospecificity.

In some embodiments, said B cells are incubated with the tumor cells prior to fusion with immortal cells and hybridoma production, under conditions enabling said B cells to be immunologically sensitized to said tumor cells. Thereby, B cells may be sensitized to said tumor cells to proliferate and/or produce memory or plasma cells secreting tumor-specific antibodies. For example, the B cells may be incubated with tumor cell preparations as detailed herein in the presence of appropriate cytokines, e.g. interleukin-2 (IL-2), IL-4 and/or CpG oligodeeoxynucleotides for *in vitro* immunization. By way of a non-limiting example, B cells may be incubated for 5-10 days in medium containing 10% heat inactivated fetal bovine serum, MDP (10 $\mu$g/ml), IL-2 (10 units/ml), IL-4 (10 ng/ml), 2-mercaptoethanol (20 $\mu$M) and tumor.

According to certain optional embodiments of the invention, droplet compartmentalized selection (DCS) techniques may be used for isolate B cells that produce antibodies with desired binding characteristics, using drop-based microfluidic devices (see, e.g. Köster et al., 2008). In some embodiments, tumor specific B cells may thus be isolated (e.g. following operation (b) of the methods of the invention), which may be used for subsequent cloning or hybridoma formation to produce immortal antibody-producing cells (operation (c)).

In some embodiments, the methods of the disclosure include obtaining a sample of tumor cells from said patient. The sample may be maintained for antibody screening, and optionally at least a portion of the cells may be cryopreserved for subsequent use. According to some embodiments, a tumor sample may be obtained from tissue biopsies or fluid samples (e.g. blood or urine samples) and maintained as known in the art. According to some embodiments, tumor cells may be fixed using formalin, paraformaldehyde or other suitable fixation methods to be used in subsequent screening steps.

In certain embodiments, tumor cells may be obtained from solid or blood borne tumors, from primary tumors or metastatic foci. In various embodiments, the tumor may include, but is not limited to, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), lymphoma (Hodgkin's disease, non-Hodgkin's disease), Waldenstrom's macroglobulinemia, and solid tumors such as sarcomas and carcinomas (e.g., fibrosarcoma, myxosarcoma, liposarcoma, chondrosarcoma, osteogenic sarcoma, chordoma, angiosarcoma, endothelio sarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, papillary carcinoma, sebaceous gland carcinoma, papillary adenocarcinomas, cystadenocarcinoma, medullary carcinoma, bronchogenic carcinoma, renal cell carcinoma, hepatoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma). According to certain particular embodiments, the tumor is a prostate or breast tumor. In another particular embodiment, the tumor is a metastatic tumor.

Advantageously, the methods of the invention utilize in some embodiments fresh tumor cell samples for antibody screening and isolation. According to these embodiments, the tumor cells are not propagated in culture for prolonged periods so as to produce *in vitro* mutated or genetically altered tumor cells, but rather substantially maintain a non-variant tumor antigenic fingerprint characterizing the tumor from which it was obtained. Accordingly, in some embodiments, the tumor cells may be grown in culture for a period of days, e.g. up to two or three days. In some embodiments, the cells are conveniently frozen upon harvesting or shortly after preparing a tumor cell isolate and thawed before the screening assay. In another exemplary embodiment, tumor cells are fixed and the screening is performed using fixed tumor cells rather than live tumor cell cultures. In one embodiment, the cells are not propagated in culture for more than one week prior to screening. Additionally or alternatively, the cells may be kept in culture for a period of one or more weeks.

**Antibody-Producing Cells**

According to some embodiments, the methods of the invention include providing a plurality of immortal antibody-producing cell clones, wherein the produced antibodies have binding selectivities corresponding to antibodies of the B cells obtained from the patient. The resulting cells are screened to identify, isolate and propagate suitable hybridoma clones that produce tumor-specific (or tumor associated) antibodies.

According to some embodiments, the B cells are fused with immortal cells (e.g. myeloma or lymphoma cells) to produce hybridomas, using well known procedures. According to some embodiments, the immortal antibody-producing cells may be produced by sequencing and cloning the immunoglobulin coding sequences into a suitable cell line to produce antibody-producing cells, using well known recombinant technology. According to some embodiments, other methods, such as EBV-induced B-cell immortalization may be used, as known in the art.

The term "hybridoma" as used herein refers to an immortalized cell that produces antibodies. In some embodiments, the term refers to a cell created by fusion of an immortalized cell derived from an immunologic source and an antibody-producing cell. The individual cells used to create the hybridoma can be from any mammalian source, including, but not limited to, mouse, rat, pig, rabbit, sheep, pig, goat, and human. It is to be understood that an immortalized cell used as a fusion partner according to the invention is a cell suitable for producing human antibodies upon fusion with a human B cell. The term also encompasses trioma cell lines, which result when progeny of heterohybrid myeloma fusions, which are the product of a fusion between human cells and a murine myeloma cell line, are subsequently fused with a plasma cell. Furthermore, the term is meant to include any immortalized hybrid cell line that produces antibodies such as, for example, quadromas. According to the principles of the invention, preferred immortalized cells are cells that are particularly effective in producing hybridomas secreting human antibodies when fused with human B cells. According to certain embodiments, human cells are preferred. For example, in certain embodiments, the B cells are fused with human myeloma or lymphoma cells, e.g. MFP-2, Karpas 707H and HAB-1 cells.

For example, B cells may be fused with suitable immortal cells such as myeloma cells using a suitable fusing agent, such as polyethylene glycol, to form a plurality of hybridomas. In some embodiments, additional fusion methods known in the art may be used, e.g. electrofusion.

The hybridoma cells thus prepared are seeded and grown in a suitable culture medium that preferably contains one or more substances that inhibit the growth or survival of the unfused, parental immortalized cells. For example, if the parental immortalized cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Exemplary immortalized cells are those that fuse efficiently, support stable high-level production of antibody by the selected antibody-producing cells, and are sensitive to a medium such as HAT medium. For example, human myeloma and mouse-human heteromyeloma cell lines also have been described for the production of human monoclonal antibodies.

After hybridoma cells are identified that produce antibodies of the desired specificity, affinity, and/or activity, as described below, the clones may be subcloned by limiting dilution procedures; some embodiments of the invention are directed to combining multiple hybridomas to provide pooled hybridoma clones as detailed below. Hybridoma clones may be grown by standard methods. Suitable culture media for this purpose include, for example, D-MEM or RPMI-1640 medium. In addition, the hybridoma cells may be grown *in vivo* as ascites tumors in an animal using method well known in the art, for example when large amounts of antibodies are required.

Additional methods for screening and producing human antibodies are known in the art, for example using phage display libraries and recombinant antibody technology. For example, generation of antigen-specific human monoclonal antibodies by *in vitro* immunization and the phage display method is described e.g. in Matsumoto et al., 2008; generation of monoclonal antibodies from single human B cells by single cell RT-PCR and expression vector cloning is described e.g. by Tiller et al., 2008. The invention encompasses in some embodiments such alternative methods which may be recognized by the skilled artisan as equivalent to some of the techniques characterized in detail herein.

## Antibodies

Antibodies, or immunoglobulins, comprise two heavy chains linked together by disulfide bonds and two light chains, each light chain being linked to a respective heavy chain by disulfide bonds in a "Y" shaped configuration. Each heavy chain has at one end a variable domain (VH) followed by a number of constant domains (CH). Each light chain has a variable domain (VL) at one end and a constant domain (CL) at its other end, the light chain variable domain being aligned with the variable domain of the heavy chain and the light chain constant domain being aligned with the first constant domain of the heavy chain (CH1). The variable domains of each pair of light and heavy chains form the antigen binding site. The isotype of the heavy chain (gamma, alpha, delta, epsilon or mu) determines immunoglobulin class (IgG, IgA, IgD, IgE or IgM, respectively). The light chain is either of two isotypes (kappa, κ or lambda, λ) found in all antibody classes.

Various antibodies exist in the form of intact antibodies, such as polyclonal antibodies or monoclonal antibodies (mAbs) as well as proteolytic fragments thereof such as the Fab or F(ab')2 fragments. In addition, chimeric antibodies, recombinant and engineered antibodies and fragments thereof are available (and may be used for example as immunoassay reagents, as detailed herein).

An "antigen" is a molecule or a portion of a molecule capable of being bound by an antibody in a highly selective manner, and not with the multitude of other antibodies which may be evoked by other antigens. The antibody-binding portion of the antigen is called an epitope.

Hybridoma cells produce monoclonal antibodies, i.e. antibodies obtained from a population of substantially homogeneous antibodies (such that the individual antibodies comprising the population are identical except for possible naturally-occurring mutations that may be present in minor amounts). In contrast to polyclonal antibody preparations which typically include different antibodies directed against different antigenic determinants (epitopes), monoclonal antibodies are highly specific, being directed against a single epitope.

According to some embodiments, antibody preparations that are used to manufacture anti-cancer medicaments according to the invention are produced from pooled hybridoma (or other immortal antibody-producing cell) clones rather than from single hybridoma clones, as detailed herein. These pooled clones may contain enhanced variability compared to single hybridoma clones, as discussed in detail below, such that the antibody preparations may have greater variability compared to a monoclonal antibody preparation. However, these antibody preparations have specificity to a common tumor cell and are typically directed against a single antigen or epitope, and thus typically have a lower degree of variability compared to polyclonal antibodies.

The invention allows for antibodies to be produced from each clone separately, thus enabling the amount of each administered antibody to be determined and adjusted to the state of the tumor throughout the different stages of treatment. The antibodies secreted by the hybridoma clones or subclones are suitably separated from the culture medium, ascites fluid, or other sources by conventional immunoglobulin purification procedures such as, for example, protein A-Sepharose, hydroxylapatite chromatography, gel electrophoresis, dialysis, or affinity chromatography. For example, selected hybridomas can be grown in two-liter spinner-flasks for antibody purification. Supernatants can be filtered and concentrated before affinity chromatography with protein A-sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD280 using 1.43 extinction coefficient. The antibodies can be aliquoted and stored at - 80°C.

In various embodiments, antibodies may be produced and purified from hybridomas or clones at different stages, for example mAbs are purified from the supernatant of each hybridoma to be tested for reactivity with the tumor and lack of cross reactivity with normal tissue (operation (d) of the methods of the invention), and optionally also for other properties of the antibodies that may be relevant for assigning the clonal pools (step (e)). After the hybridomas are combined to produce the hybridoma clones, antibodies are produced from each clone and are subjected to quality assurance assays, to verify that they retain the desired properties *in vitro* and have adequate *in vivo* properties (operations (f)-(h)). Before administering the antibodies to the patient (operations (h)-(l)), antibody purification methods allowing production of antibodies at enhanced amounts and/or with enhanced purity, as known in the art. Quality assurance assays may be repeated throughout the process, for example before administering antibodies to the patient.

**Screening and Clonal Pooling**

According to further embodiments, the plurality of hybridomas (or antibody producing cells) are screened to identify cells producing antibodies which selectively bind the tumor cells obtained from said patient while not substantially binding non-malignant human cells or tissue in a selective manner.

The terms "selective binding", "specific binding" and/or "specifically bind" refer to two molecules (e.g. an antibody and an antigen) forming a complex that is relatively stable under physiologic conditions. Specific (or selective) binding is characterized by a high affinity and a low to moderate capacity as distinguished from nonspecific binding which usually has a low affinity with a moderate to high capacity. Typically, binding is considered specific when the association constant $K_A$ is higher than $10^6$ $M^{-1}$. The term "selectively bind" (or "bind in a selective manner") as used herein may further indicate that the binding of an antibody to an antigen is not competitively inhibited by the presence of non-related molecules.

Advantageously, the antibodies are screened against tumor cells isolated from the patient, which were not propagated in culture so as to produce *in vitro* mutated or genetically altered tumor cells, as specified above. Accordingly, in some embodiments, the screening is performed with tumor cells grown in culture for e.g. up to two or three days. In another embodiment the cells are not propagated in culture for more than one week prior to screening. In some embodiments, the screening is performed with tumor cells that were not substantially grown in culture, for example on fixed tumor cells (e.g. by formalin or paraformaldehyde).

In some embodiments, the antibody screening (e.g. in operations (d) or (g) of the methods of the invention) may be performed using various immonoassays known in the art, including, but not limited to, enzyme-linked immunosorbent assay (ELISA), Enzyme-linked immunosorbent spot (ELISPOT), techniques radioimmunoassay (RIA) and Fluorescence Activated Cell Sorting (FACS). In some embodiments, the methods of the invention are suitable for automated or semi-automated analysis, and may enable medium or high-throughput screening of multiple antibodies. For example, automated ELISA systems such as Biotest's Quickstep® ELISA Processor, Maxmat Automated microwell ELISA analyzer (Maxmat S.A., France), or DSX™ Four-Plate System (Dynex Technologies) may conveniently be used. In another particular embodiment, droplet-based micro fluidic device methods may be used for high throughput monoclonal antibody screening (Koster et al., 2008).

The antibodies produced are further tested according to some embodiments to identify those which are not cross-reactive with normal human antigens, namely bind the patient's tumor cells and do not substantially bind non-malignant human cells or tissue. This may conveniently be performed using tissue chips (that may be purchased from FDA approved manufacturers) according to e.g. FDA accepted protocols. Antibodies may be tested against the patient's tumor biopsy and healthy cell samples using e.g. immunoassays as detailed above.

According to some embodiments, the methods of the invention include a step of clonal pooling prior to antibody mass production, in which the individual (monoclonal) hybridomas (or antibody producing cells) are combined to obtain pooled clones, each clone being an oligo-clone consisting of one or more individual hybridomas having similar properties. In various embodiments, additional tests are performed on the hybridomas and/or their secreted antibodies in order to identify and combine suitable clones. Hybridomas which are determined substantially similar with respect to various tested parameters are combined to a single clone, used in subsequent propagation, screening and antibody production, thus providing efficient, cost effective and less time consuming methods.

According to some embodiments, the individual hybridomas within each pooled clone have substantially similar properties with respect to their genetic homology of the immunoglobulin variable domains. According to some embodiments the individual hybridomas within each pooled clone have substantially similar properties with respect to their antigen specificity, such that the antibodies secreted from said hybridomas specifically bind (co-localize to) the same tumor cells. In some embodiments, the individual hybridomas within each pooled clone have substantially similar properties with respect to their ability to co-localize to the same tumor cell. In some embodiments, the individual hybridomas within each pooled clone have substantially similar properties with respect to other parameters determining the antigenic target of the antibodies (belonging to group (i) parameters).

For example, determining the ability of the antibodies secreted from said hybridomas to bind the same tumor cells (e.g. a tumor clone or an antigen expressed by a selected population of tumor cells having common characteristics) may be performed by various immunoassays as described above. Conveniently, co-localization of the antibodies to a specific cancer cell may be determined e.g. by employing multi-color flow cytometry.

In some embodiments, genetic homology of the hybridomas is determined from the sequences of the variable region of the immunoglobulin (Ig) gene, using e.g. PCR to amplify hypervariable regions fragments and consequent sequencing. Hybridomas which are substantially homologous in the Ig variable region genes are combined to a single clone. DNA encoding the monoclonal antibodies is readily isolated and sequenced using conventional procedures (e.g., by using oligonucleotide probes that are capable of binding specifically to genes encoding the heavy and light chains of the monoclonal antibodies). For example, in various embodiments, substantial similarity in genetic homology of the Ig variable domains may be determined as at least 60%, at least 70%, at least 80%, at least 90%, at least 95%, at least 98% or 100% homology of the Ig variable domain. In other specific embodiments, genetic homology is determined with respect to the hypervariable regions. In other particular embodiments, genetic homology is determined with respect to the complementary determining regions (CDR).

According to some embodiments, the individual hybridomas within each pooled clone have substantially similar properties with respect to binding sensitivity and/or binding selectivity of the antibodies secreted from said hybridomas to their respective antigens (e.g. to the same tumor cells). In some embodiments, the individual hybridomas within each pooled clone have substantially similar properties with respect to other parameters determining the binding characteristics of the antibodies to their antigenic targets (belonging to group (ii) parameters).

In some embodiments, antibody binding selectivity (as defined above) may be conveniently determined by competitive binding assays, analyzed e.g. by flow cytometry or by fluorescent microscopy.

In some embodiments, antibody binding sensitivity (affinity or avidity) may be conveniently determined by measuring the minimal concentration of mAb detectable on a specific population of cancer cells, analyzed e.g. by flow cytometry or by fluorescent microscopy.

According to further embodiments, the individual hybridomas within each pooled clone have substantially similar properties with respect to the isotype of the antibodies secreted from said hybridomas, the growth rate of the hybridomas and the antibody secretion rate of said hybridomas. In some embodiments, the individual hybridomas within each pooled clone have substantially similar properties with respect to other parameters determining antibody production, pharmacokinetic/pharmacodynamic properties of the antibodies and/or their effector functions (belonging to group (iii) parameters).

These parameters may be measured by methods known in the art. Other assays could be employed as needed in the methods of the invention, for example histology, multiplex cytometry or in situ proximity ligation assays such as Duolink®.

In another embodiment, the clonal groups are determined such that the individual hybridomas within each clone are not substantially similar to hybridomas of other clones. In another embodiment, the clonal groups are determined such that the individual hybridomas within each clone are not substantially similar to hybridomas of other clones with respect to the parameters of group (i). In another embodiment, the clonal groups are determined such that the individual hybridomas within each clone are not substantially similar to hybridomas of other clones with respect to at least one of the parameters of group (ii). In another embodiment, the individual hybridomas within each clone are not substantially similar to hybridomas of other clones further with respect to the parameters of group (iii).

In some embodiments, the clonal groups are determined such that the individual hybridomas within each pooled clone are substantially similar to the hybridomas within the clone with respect to at least one of the parameters of group (i) and optionally further with respect to at least one of the parameters of groups (ii) and (iii).

In some embodiments the clonal groups are determined such that the individual hybridomas within each clone are not substantially similar to hybridomas of other clones with respect to the parameters of group (i) and optionally further with respect to at least one of the parameters of group (ii). In another embodiment, the individual hybridomas within each clone are not substantially similar to hybridomas of other clones further with respect to the parameters of group (iii).

In some embodiments, combining hybridomas to produce pooled clones results in the reduction of the number of clones used in further screening and manufacturing (e.g. from about 96 wells to about 20 wells). In certain embodiments, inherent variability within the antibodies produced by the pooled clone allows for an enhanced therapeutic effect of antibodies covering naturally occurring antigen variance in the tumor compared to monoclonal antibodies, while allowing tumor clone-specific adjustment to be maintained with sufficient reproducibility. In some embodiments, combining hybridomas allows generating enhanced selectivity differences between target cells and non-target somatic cells. For example, hybridoma immunoproducts potentially interact to produce a supra-linear effect on activity. Optionally, pooling criteria are chosen to maximize the lethal selectivity of the pool to target cells, and/or minimize supra-linear adverse effects on non-target somatic cells.

Determining whether the similarity between hybridomas with respect to the above-identified parameters is substantial, and the consequent selection of the clonal groups, may be effected by a skilled artisan so as to create clone groups (oligo-clones) from highly similar clones only, as detailed herein. In various particular embodiments, substantial similarity in a tested parameter may be e.g. of at least 50%, at least 60%, at least 70%, at least 80%, at least 90%, at least 95%, at least 98% or 100%. In certain particular embodiments, algorithmic approaches may conveniently be used for oligo-clone selection. For example, in certain embodiments, optimization-above-simulation algorithms may conveniently be used.

For example, in some embodiments, the different parameters are tested sequentially, such that only clones that are identical or determined substantially similar with respect to a first parameter (e.g. a parameter of group (i)) are tested for a second parameter (e.g. a parameter of group (ii) or (iii)). This process may be repeated for additional parameters to exclude hybridoma clones that are not substantially similar in one of the tested parameters and is concluded when the remaining tested clones are substantially similar in all the tested parameters. The hybridomas thus identified are pooled into an oligo-clone for subsequent propagation and testing. The process may be repeated on the non-pooled hybridomas to identify and produce additional oligo-clones.

In some exemplary embodiments, hybridoma clones are separately tested for the various parameters and virtually assigned into clone groups having maximal inter-group variability, e.g. with respect to parameters of group (i) and (ii). Individual hybridomas may be excluded from each group so as to obtain minimal intra-group variability, e.g. retaining in each such group only hybridomas that are substantially similar with respect to parameters of group (iii). The hybridomas thus identified are pooled into an oligo-clone for subsequent propagation and testing.

**Pharmaceutical Compositions and Treatment Optimization**

According to some embodiments, the identified antibodies are used for the preparation of a medicament for the treatment of cancer. According to further embodiments, additional assays are performed in order to determine a suitable dose for each antibody preparation in the composition according to the characteristics of the tumor, the antibodies and the patient.

Thus, according to some embodiments, the methods of the invention include determining the relative amount, and optionally malignancy, of individual tumor clones in said patient corresponding to each antibody preparation. In some embodiments, the methods include determining pharmacokinetic and/or pharmacodynamic properties of the antibody preparations. In some embodiments, the methods include determining patient-specific parameters.

In some embodiments, the methods of the invention include assaying the identified antibody preparations to select preparations of antibodies that localize to the tumor in the patient *in vivo,* which may be included in the composition. Optionally, the relative tumor-localizing fraction of each antibody preparation is determined, and the administered doses are adjusted accordingly.

For example, antibodies from each clone may be conjugated to imaging enhancement agents (e.g. magnetic, radioactive or fluorescent labels) and administered to the patient. The bio-distribution and accumulation of these labeled Abs may be monitored to identify tumor-localizing antibodies which may be suitable for treatment. In some embodiments, the labeled antibodies may be administered to the patient at low doses (e.g. subtherapeutic doses). Adverse effects may also be monitored so as to select antibody preparations that have sufficient in-vivo tumor localization and adequate *in vivo* safety.

In certain embodiments, the relative tumor localization fraction of each Ab preparation is calculated as the ratio of the tumor localizing amount (e.g. relative to the amount of its corresponding tumor clone within the tumor) to the amount that localizes to non tumor-related organs or tissues. In certain embodiments, the corresponding amount of the antibody preparations is determined to be inversely correlated to the relative tumor-localizing fraction of each antibody preparation.

In some embodiments, additional pharmacokinetic/pharmacodynamic properties of the antibody preparations may be measured and taken into consideration when determining the dose and administration regimen (for example the Ab half-life as indicated below).

In some embodiments, the Ab doses are determined and adjusted according to the amount, malignancy and/or other parameters characterizing each tumor clone.

In some embodiments, determining the relative amount of individual tumor clones in said patient corresponding to each antibody preparation may be performed by various *in vitro*, *ex vivo* and/or *in vivo* methods. For example, the identified antibodies may be used in a variety of immunoassays e.g. immunohistochemistry, flow cytometry or by using fluorescent microscopy. In some embodiments, determining the relative amounts of tumor clones may conveniently be performed using *in vivo* imaging methods as described above. In certain embodiments, the dose ratio of the different Ab preparations in the composition is correlated to the relative amounts of the individual tumor clones.

The malignancy of each tumor clone may optionally be evaluated based on the ability of cells of each tumor clone to proliferate and invade neighboring tissue. Thus, in certain embodiments, cells of various tumor clones may be isolated and tested for viability (e.g. by staining with trypan blue or propidium iodide or using Annexin V kits), proliferation rate (e.g. by measuring [3H]thymidine incorporation), migration (e.g. using transwell assays) and/or by determining tumor stage or grade for each clone according to accepted classification. In a particular embodiment, malignancy is determined by measuring tumor viability and migration. According to some embodiments, the dose ratio of the different Ab preparations in the composition is correlated with the relative malignancy of each tumor clone.

In some embodiments, the amount of antibody preparations may be determined according to patient parameters including but not limited to the patient's size, weight, age, gender, livelihood (e.g. by measuring vital signs such as blood pressure, heart rate or temperature, weight loss/gain), disease severity and the nature and/or dose of other medical treatments that are concurrently administered to or performed on the patient. For example, in some embodiments, the Ab dose correlates to the patient's weight. These parameters, individually and/or in whole or partial aggregate are used, in some embodiments, for evaluation of patient performance status. In some embodiments, patient performance status includes measures of performance status according to a scale of observed and/or patient-reported criteria such as mood, energy level, and/or level of activity. In some embodiments, performance status relates to patient well-being and/or an evaluation of patient risk with respect to potential treatment side-effects.

In some embodiments, the methods of the invention include providing a pharmaceutical composition comprising the antibody preparations identified as described herein at predetermined doses, wherein the dose ratio of the different preparations in the composition is correlated to the relative amounts of the individual tumor clones in said patient, optionally further correlated to the relative malignancy of each tumor clone, and optionally further inversely correlated to the relative tumor-localizing fraction of each antibody preparation. Conveniently, determination of the Ab doses based on these parameters may be done using adaptive control algorithms, as detailed herein. In a particular embodiment, said doses are determined such that said composition reacts with substantially all of said individual tumor clones *in vivo*.

A pharmaceutical composition can contain a pharmaceutically acceptable carrier for administration of the antibodies. The carrier should not itself induce the production of antibodies harmful to the individual receiving the composition and should not be toxic. The term "carrier" refers to a diluent, adjuvant or excipient, with which an Ab preparation of the disclosure is administered. Such pharmaceutical carriers can be liquids, such as water and oils, including those of petroleum, animal, vegetable or synthetic origin, such as peanut oil, soybean oil, mineral oil, sesame oil and the like. The carriers can be saline, gum acacia, gelatin, starch paste, talc, keratin, colloidal silica, urea, and the like. In addition, auxiliary, stabilizing, thickening, lubricating and coloring agents can be used. In one embodiment, when administered to a subject, the Ab preparation of the disclosure and pharmaceutically acceptable carriers are sterile. Water is a preferred carrier when the Ab preparation of the disclosure is administered intravenously. Saline solutions and aqueous dextrose and glycerol solutions can also be employed as liquid carriers, particularly for injectable solutions. Suitable pharmaceutical carriers also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents. Suitable carriers can in some embodiments be selected from large, slowly metabolized macromolecules such as proteins, polypeptides, liposomes, polysaccharides, polylactic acids, polyglycolic acids, polymeric amino acids, amino acid copolymers and inactive virus particles, although suitable carriers are not limited to these examples.

Preferred forms for administration include forms suitable for parenteral administration, e.g. by injection or infusion, for example by bolus injection or continuous infusion. Where the product is for injection or infusion, it can take the form of a suspension, solution or emulsion in an oily or aqueous vehicle and it can contain formulatory agents, such as suspending, preservative, stabilizing and/or dispersing agents. Alternatively, the antibody molecule can be in dry form, for reconstitution before use with an appropriate sterile liquid. Once formulated, the compositions of the disclosure can be administered directly to the patient. According to embodiments of the invention it is preferred that the compositions are adapted for administration to human patients.

A pharmaceutical composition of this disclosure can be administered by any number of routes accepted in the art for administration of antibodies. Direct delivery of the compositions will generally be accomplished by injection, subcutaneously, intraperitoneally, intravenously or intramuscularly, or delivered to the interstitial space of a tissue. Dosage treatment can be a single dose schedule or a multiple dose schedule. According to a currently preferred embodiment, the antibodies are administered intravenously.

When an antibody or antibody fragment composition is to be administered by a route using the gastrointestinal tract, the composition can to contain additional agents which protect the antibody from degradation but which release the antibody once it has been absorbed from the gastrointestinal tract. Such additional agents are well-known to those skilled in the art.

In another embodiment, the antibodies are optionally conjugated to an anti-cancer substance prior to administration to said patient. For example, the conjugation may be performed on antibodies identified in operation (h) or operation (i) if applicable, or following operation (j) (operations referenced are with respect to the list of operations presented herein below). In various embodiments, the antibodies may be conjugated to chemotherapeutic drugs, toxins, radioisotopes, magnetic nanoparticles and/or other anti-cancer substances that are suitable for the treated tumor and patient. Methods for attaching antibodies to such effectors are known in the art.

For example, toxins (or cytotoxic proteins) can include, but are not limited to, Ricin-A, Pseudomonas toxin, Diphtheria toxin, and tumor necrosis factor.

Chemotherapeutic drugs can interface with critical cellular processes including DNA, RNA, and protein synthesis. Examples of such drugs include, but are not limited to, daunorubicin, doxorubicin, methotrexate, paclitaxel, melphalan, vinca alkaloids, etoposide and Mitomycin C.

In a particular embodiment, the anti-cancer substance may further comprise means for monitoring the localization and distribution of the administered antibodies (e.g. using radioisotopes or magnetic nanoparticles).

Examples of radioisotopes include e.g. Bi, I, Re, and Y, among others. Radioisotopes can exert their cytotoxic effect by locally irradiating the cells, leading to various intracellular lesions, as is known in the art of radiotherapy.

Magnetic nanoparticles, such as nano-magnetic beads can be both visualized by computerized tomography (CT) and magnetic resonance imaging (MRI) or activated for hyperthermia treatment. During such treatment, after the composition is administered and given sufficient time for the Abs to bind the tumor cells, the patient is placed in an alternating magnetic field of suitable amplitude and frequency, such that the tumor temperature rises. This could kill the tumor cells by necrosis if the temperature is above 45 °C, or could improve the efficiency of chemotherapy if the temperature is raised around 42 °C. For example, metallic particles or iron oxide particles are known in the art and may be used in the methods of the invention according to accepted protocols.

In some embodiments the Abs may be tested and selected to exert direct antitumor effects, as known in the art.

In some embodiments, the hybridomas themselves are optionally provided. This provides a potential advantage for allowing continuous immunocompound protection during the inter-treatment intervals. The problem of unbounded multiplication of the hybridomas is optionally overcome by using trans-specific hybridomas, for example, murine hybridomas, which are potentially cleared over time by the patient's own immune system-the auxiliary immune system being itself more vulnerable to the host immune system than the cancer it itself is provided to overcome.

According to some embodiments, the disclosure provides methods of treating a patient in need thereof comprising administering a pharmaceutical composition of the disclosure to the subject. As used herein, the term "treatment" or "therapy" refers to administering an active agent with the purpose to cure, heal, alleviate, relieve, alter, remedy, ameliorate, improve, or affect a condition (e.g., a disease), the symptoms of the condition, or to prevent or delay the onset of the symptoms, complications, biochemical indicia of a disease, or otherwise arrest or inhibit further development of the disease, condition, or disorder in a statistically significant manner. In some embodiments the methods of the disclosure are used for treating a patient afflicted with cancer.

Generally, an effective dose of an antibody preparation administered e.g. intravenously, is from 0.01 mg/kg to 50 mg/kg, preferably 0.1 mg/kg to 20 mg/kg, more preferably from about 1 mg/kg to about 15 mg/kg. Compositions can be administered individually to a patient or can be administered in combination with other agents, drugs or hormones. According to some aspects, antibodies can be conjugated with these agents.

In some embodiments, if an antibody molecule has a short half-life (e.g. 2 to 10 hours) it can be necessary to give one or more doses per day. Alternatively, if an antibody molecule has a long half-life (e.g. 2 to 15 days) it can only be necessary to give a dosage once per day, per week or even once every 1 or 2 months.

**Exemplary Operations in Methods of the Invention**

In some embodiments, the methods of the disclosure include one or more of:

a. obtaining a sample of tumor cells from said patient;
b. obtaining B cells from the patient;
c. fusing the B cells with immortal cells to produce a plurality of hybridomas;
d. screening the plurality of hybridomas to identify hybridomas producing antibodies which selectively bind the tumor cells obtained from said patient while not substantially binding non-malignant human cells or tissue in a selective manner;
e. combining the individual hybridomas identified in (d) to obtain pooled clones, each clone consisting of one or more individual hybridomas that are substantially similar to the hybridomas within the clone with respect to at least one of the parameters of group (i) and optionally further with respect to at least one of the parameters of groups (ii) and (iii);
f. producing antibodies from each clone separately, to obtain a plurality of antibody preparations corresponding to the plurality of clones;
g. assaying the plurality of antibody preparations to identify preparations of antibodies that do not substantially bind non-malignant human cells or tissue in a selective manner while selectively binding to tumor cells obtained from said patient;
h. assaying the antibody preparations identified in (g) to select preparations of antibodies that localize to the tumor in said patient *in vivo,* and optionally to determine the relative tumor-localizing fraction of each antibody preparation;
i. determining the relative amount and optionally malignancy of individual tumor clones in said patient corresponding to each antibody preparation; and
j. providing a pharmaceutical composition comprising the antibody preparations identified in (h) at predetermined doses, wherein the dose ratio of the different preparations in the composition is correlated to the relative amounts of the individual tumor clones in said patient, optionally further correlated to the relative malignancy of each tumor clone, and optionally further inversely correlated to the relative tumor-localizing fraction of each antibody preparation, wherein the antibodies are optionally conjugated to an anti-cancer substance prior to administration to said patient;
k. administering said composition to said patient;
l. determining the amount and optionally malignancy of said individual tumor clones in said patient; and/or
m. repeating at least (k) to (1) at predetermined intervals, to adjust the administered dose of each antibody preparation according to a change in the amount and/or malignancy of said individual tumor clones.

Optionally, the methods of the invention include operations of identifying additional tumor-specific antibodies during treatment adjustment. For example, following administration of the pharmaceutical composition to the patient, operations (b)-(d) may be repeated and the antigenic specificities of the newly identified Abs are determined and compared to those of the previously administered antibodies. If one or more antibodies are identified that selectively bind the tumor cells while not substantially binding non-malignant human cells or tissue in a selective manner, but have distinct antigenic specificities, operations (e)-(j) may be repeated.

It is expected that during the life of a patent maturing from this application many relevant modeling, predicting, immunocompounds, pharmaceuticals, immune assessment, disease assessment and antibody production technologies will be developed and the scope of the related terms are intended to include all such new technologies a *priori*.

As used herein, the term "about" refers to within ±10%.

The terms "comprises", "comprising", "includes", "including", "having" and their conjugates mean: "including but not limited to".

The term "consisting of' means: "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

The words "example" and "exemplary" are used herein to mean "serving as an example, instance or illustration". Any embodiment described as an "example or "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features except insofar as such features conflict.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, *etc.*, as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the present invention. To the extent that section headings are used, they should not be construed as necessarily limiting.

When reference is made to particular sequence listings, such reference is to be understood to also encompass sequences that substantially correspond to its complementary sequence as including minor sequence variations resulting from, for example: sequencing errors, cloning errors, or other alterations resulting in base substitution, base deletion or base addition; provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is appreciated that certain features of the invention, which are, for clarity, described in the context of separate embodiments, may also be provided in combination in a single embodiment. Conversely, various features of the invention,

**EP 3 052 933 B1**

which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

REFERENCES

Aoki M, Saikawa Y, Hosokawa S, Fukuda K, Kumai K, Kubota T, et al. A novel human monoclonal antibody derived from tumor-infiltrating lymphocytes in lung cancer inhibits cancer cell growth with morphological changes. Anticancer Res. 2005 Nov-Dec;25(6B):3791-8.

Brandlein S, Lorenz J, Ruoff N, Hensel F, Beyer I, Muller J, et al. Human monoclonal IgM antibodies with apoptotic activity isolated from cancer patients. Hum Antibodies. 2002;11(4):107-19.

Disis ML, Lyerly HK. Global role of the immune system in identifying cancer initiation and limiting disease progression. J Clin Oncol. 2005 Dec 10;23(35):8923-5.

Drake CG, Jaffee E, Pardoll DM. Mechanisms of immune evasion by tumors. Adv Immunol. 2006;90:51-81.

Dudley ME, Rosenberg SA. Adoptive-cell-transfer therapy for the treatment of patients with cancer. Nat Rev Cancer. 2003 Sep;3(9):666-75.

Finn OJ. Cancer vaccines: between the idea and the reality. Nat Rev Immunol. 2003 Aug;3(8):630-41.

Imahayashi S, Ichiyoshi Y, Yoshino I, Eifuku R, Takenoyama M, Yasumoto K. Tumor infiltrating B-cell-derived IgG recognizes tumor components in human lung cancer. Cancer Invest. 2000;18(6):530-6.

Koster et al., Drop-based microfluidic devices for encapsulation of single cells. Lab Chip, 2008, 8, 1110-1115.

Marantz Henig. In business to treat cancer. The New York Times, November 23, 1986.

Matsumoto et al., A rapid and efficient strategy to generate antigen-specific human monoclonal antibody by in vitro immunization and the phage display method. Journal of Immunological Methods 332 (2008) 2-9.

McIntosh. Providing experimental therapies as a commercial venture fails. Journal of the National Cancer Institute. 1990:84-87.

Rabinovich GA, Gabrilovich D, Sotomayor EM. Immunosuppressive strategies that are mediated by tumor cells. Annu Rev Immunol. 2007;25:267-96.

Rothe A, Klimka A, Tur MK, Pfitzner T, Huhn M, Sasse S, et al. Construction of phage display libraries from reactive lymph nodes of breast carcinoma patients and selection for specifically binding human single chain Fv on cell lines. Int J Mol Med. 2004 Oct;14(4):729-35.

Schwartz-Albiez et al. Natural antibodies, intravenous immunoglobulin and their role in autoimmunity, cancer and inflammation. Clinical and Experimental Immunology, 2009;158 (Suppl. 1): 43-50.

Schwartz-Albiez et al., Cytotoxic natural antibodies against human tumours: An option for anti-cancer immunotherapy? Autoimmunity Reviews 7 (2008) 491-495.

Tiller et al., Efficient generation of monoclonal antibodies from single human B cells by single cell RT-PCR and expression vector cloning. J Immunol Methods. 2008 January 1; 329(1-2): 112-124.

Vollmers and Brändlein. Natural antibodies and cancer. Journal of Autoimmunity 29 (2007) 295-302.

Vollmers and Brändlein. Natural antibodies and cancer. New Biotechnology 2009 Volume 25, Number 5: 294-298.

Vollmers and Brändlein. Natural Monoclonal Antibodies and Cancer. Recent Patents on Anti-Cancer Drug Discovery, 2008, 3, 84-87.

Vollmers and Brändlein. Nature's best weapons to fight cancer. Revival of human monoclonal IgM antibodies. Human Antibodies 11 (2002) 131-142.

**Claims**

1. A method of adapting a pharmaceutical composition comprising immunocompound pharmaceutical components for the treatment of a cancerous disease in a patient in need thereof, comprising:

   determining a model including: (1) a current disease state including as factors determinations of cell count prevalences of a plurality of clonal cell types in the cancerous disease of the patient, and (2) immunocompound pharmaceutical components having treatment effects on the disease state based on the measured partitioning of the immunocompound pharmaceutical components among the clonal cell types;
   calculating a composition of said immunocompound pharmaceutical components acting on the current disease state according to their measured partitioning to produce a target disease state model including as factors determinations of cell count prevalences of the plurality of clonal cell types;
formulating a treatment composition for said patient based on the calculated composition;
receiving results providing information about cell count prevalences of the plurality of clonal cell types in the patient after the treatment composition was administered;
producing a new disease state model based on the received results;
adjusting the model based on differences between the target disease state model and the new disease state model of the patient; and
repeating said calculating and formulating.

2. The method according to claim 1, wherein the immunocompound pharmaceutical components comprise immunocompounds; and wherein their measured partitioning is determined based on their binding activities associated with binding to respective clonal cell types of the plurality of clonal cell types.

3. A method according to claim 1, wherein said repeating comprises repeating said calculating and formulating for at least four iterations of formulation and subsequent treatment with the formulation.

4. A method according to claim 1, wherein said repeating comprises repeating said adjusting.

5. A method according to claim 1, wherein said calculating takes into account a tradeoff between a change in patient health state and a change in disease state caused by treatment with said composition.

6. A method according to claim 1, wherein said current disease state is stored as a vector, and wherein said calculating comprises matrix-vector or matrix-matrix multiplications on said stored vector.

7. A method according to claim 1, wherein said calculating uses a mimo model with multiple inputs generating multiple interdependent outputs.

8. A method according to claim 1, wherein said calculating comprises search a state-space for a state at least approaching said target state.

9. A method according to claim 1, wherein said adjusting comprises at least one of the group consisting of:

adjusting a model of the interaction between disease and treatment, and
changing a disease state portion of the model.

10. The method of claim 2, comprising determining the susceptibility of said cancer; the determining of susceptibility comprising assaying identified preparations of said immunocompounds to select preparations of immunocompounds that localize to the clonal cell types in a cancerous tumor of said patient in vivo.

11. The method of claim 2, wherein said formulating comprises providing a pharmaceutical composition comprising identified immunocompound preparations at predetermined doses, wherein the dose ratio of the different preparations in the composition is correlated to relative vital states of said clonal cell types.

12. The method according to claim 1, the model comprising an adaptively controlled model wherein:

said calculating a composition comprises calculating control to exercise on the adaptively controlled model to produce a state at least approaching the target disease state, based on which control component the calculated composition is determined;
the adjusting the model based on differences between the target disease state model and the new disease state model of the patient adjusts modelling of the interaction between disease and treatment;
the adjusting the model further comprises adjusting the current disease state of the model to correspond to the new disease state of the patient; and
the repeating of the calculating calculates a new composition which is different from the composition based on the adjusting of the model.

13. The method according to claim 12, the model comprising an adaptive linearly controlled model, wherein the calculating a composition comprises calculating a linear combination of the modeled treatment effects of the immunocompound pharmaceutical components on the disease state.

14. The method of claim 2, wherein the results providing information identify a distribution of a clonal cell type in the patient from diagnostic images of a distribution of at least one of the immunocompounds in vivo.

15. A system consisting of a computer, configured by programming to implement processing to perform the determining, calculating, receiving, producing, and adjusting of claim 1.

16. A method of adapting a pharmaceutical composition comprising immunocompound pharmaceutical components for the treatment of a cancerous disease in a patient in need thereof, comprising:

receiving results providing information about cell count prevalences of a plurality of clonal cell types in the patient after a pharmaceutical composition was administered;
producing a disease state model based on the received results, wherein the disease state includes as factors determinations of cell count prevalences of the plurality of clonal cell types in the cancerous disease of the patient;
adjusting a model including: (1) the disease state, and (2) immunocompound pharmaceutical components having treatment effects on the disease state based on the measured partitioning of the immunocompound pharmaceutical components among the clonal cell types, wherein the model is adjusted based on differences between disease states of the patient prior to and after administration of the pharmaceutical composition;
calculating a composition of said immunocompound pharmaceutical components acting on the adjusted disease state model according to their measured partitioning to produce a target disease state model; and
formulating a treatment composition for said patient based on the calculated composition.

**Patentansprüche**

1. Ein Verfahren zum Adaptieren einer pharmazeutischen Zusammensetzung, die pharmazeutische Immunverbindungskomponenten beinhaltet, zur Behandlung einer Krebskrankheit bei einem Patienten, der diese benötigt, beinhaltend:

Bestimmen eines Modells, das Folgendes umfasst: (1) einen derzeitigen Krankheitsstatus, der als Faktoren Bestimmungen von Zellzahlprävalenzen einer Vielzahl von klonalen Zelltypen in der Krebskrankheit des Patienten umfasst, und (2) pharmazeutische Immunverbindungskomponenten mit Behandlungswirkungen auf den Krankheitsstatus auf der Grundlage der gemessenen Aufteilung der pharmazeutischen Immunverbindungskomponenten unter den klonalen Zelltypen;
Berechnen einer Zusammensetzung der genannten pharmazeutischen Immunverbindungskomponenten, die sich auf den derzeitigen Krankheitsstatus auswirken, gemäß ihrer gemessenen Aufteilung, um ein Zielkrankheitsstatusmodell zu produzieren, das als Faktoren Bestimmungen von Zellzahlprävalenzen einer Vielzahl von klonalen Zelltypen umfasst;
Formulieren einer Behandlungszusammensetzung für den genannten Patienten auf der Grundlage der berechneten Zusammensetzung;
Erhalten von Ergebnissen, die Informationen über Zellzahlprävalenzen der Vielzahl von klonalen Zelltypen bei dem Patienten bereitstellen, nachdem die Behandlungszusammensetzung verabreicht wurde;
Produzieren eines neuen Krankheitsstatusmodells auf der Grundlage der erhaltenen Ergebnisse;
Anpassen des Modells auf der Grundlage von Unterschieden zwischen dem Zielkrankheitsstatusmodell und dem neuen Krankheitsstatusmodell des Patienten; und
Wiederholen des genannten Berechnens und Formulierens.

2. Verfahren nach Anspruch 1, wobei die pharmazeutischen Immunverbindungskomponenten Immunverbindungen beinhalten; und wobei ihre gemessene Aufteilung auf der Grundlage ihrer Bindungsaktivitäten bestimmt wird, die mit der Bindung an jeweilige klonale Zelltypen der Vielzahl von klonalen Zelltypen assoziiert sind.

3. Verfahren nach Anspruch 1, wobei das genannte Wiederholen das Wiederholen des genannten Berechnens und Formulierens für mindestens vier Iterationen des Formulierens und der nachfolgenden Behandlung mit der Formulierung beinhaltet.

4. Verfahren nach Anspruch 1, wobei das genannte Wiederholen das Wiederholen des genannten Anpassens beinhaltet.

5. Verfahren nach Anspruch 1, wobei das genannte Berechnen einen Abgleich zwischen einer Änderung des Gesundheitsstatus des Patienten und einer Änderung des Krankheitsstatus, der durch die Behandlung mit der genannten Zusammensetzung verursacht wird, berücksichtigt.

6. Verfahren nach Anspruch 1, wobei der genannte derzeitige Krankheitsstatus als ein Vektor gespeichert wird, und wobei das genannte Berechnen Matrix-Vektor- oder Matrix-Matrix-Multiplikationen an dem gespeicherten Vektor beinhaltet.

7. Verfahren nach Anspruch 1, wobei das genannte Berechnen ein Mimo-Modell mit mehreren Eingaben, das mehrere voneinander abhängige Ausgaben erzeugt, verwendet.

8. Verfahren nach Anspruch 1, wobei das genannte Berechnen Statussuch-Raum für einen Status beinhaltet, der mindestens eine Annäherung des genannten Zielstatus ist.

9. Verfahren nach Anspruch 1, wobei das genannte Anpassen mindestens eines von der Gruppe beinhaltet, die aus Folgendem besteht:

   Anpassen eines Modells der Interaktion zwischen Krankheit und Behandlung, und
   Ändern eines Krankheitsstatusanteils des Modells.

10. Verfahren nach Anspruch 2, das Folgendes beinhaltet: Bestimmen der Anfälligkeit des genannten Krebses; wobei das Bestimmen der Anfälligkeit das Analysieren identifizierter Präparate der genannten Immunverbindungen beinhaltet, um Präparate von Immunverbindungen auszuwählen, die an den klonalen Zelltypen in einem Krebstumor des genannten Patienten in vivo lokalisieren.

11. Verfahren nach Anspruch 2, wobei das genannte Formulieren das Bereitstellen einer pharmazeutischen Zusammensetzung beinhaltet, die identifizierte Immunverbindungspräparate in vorbestimmten Dosen beinhaltet, wobei das Dosisverhältnis der verschiedenen Präparate in der Zusammensetzung mit dem relativen Vitalstatus der genannten klonalen Zelltypen korreliert ist.

12. Verfahren nach Anspruch 1, wobei das Modell ein adaptiv kontrolliertes Modell beinhaltet, wobei:

   das genannte Berechnen einer Zusammensetzung das Berechnen der Kontrolle beinhaltet, die auf das adaptiv kontrollierte Modell ausgeübt werden soll, um einen Status zu produzieren, der mindestens eine Annäherung des Zielkrankheitsstatus ist, basierend darauf, welche Kontrollkomponente der berechneten Zusammensetzung bestimmt wird;
   das Anpassen des Modells auf der Grundlage von Unterschieden zwischen dem Zielkrankheitsstatusmodell und dem neuen Krankheitsstatusmodell des Patienten, das Modellieren der Interaktion zwischen Krankheit und Behandlung anpasst;
   das Anpassen des Modells ferner das Anpassen des derzeitigen Krankheitsstatus des Modells beinhaltet, sodass es dem neuen Krankheitsstatus des Patienten entspricht; und
   das Wiederholen des Berechnens eine neue Zusammensetzung berechnet, die verschieden von der Zusammensetzung ist, die auf dem Anpassen des Modells basiert.

13. Verfahren nach Anspruch 12, wobei das Modell ein adaptiv linear kontrolliertes Modell beinhaltet, wobei das Berechnen einer Zusammensetzung das Berechnen einer linearen Kombination der modellierten Behandlungswirkungen der pharmazeutischen Immunverbindungskomponenten auf den Krankheitsstatus beinhaltet.

14. Verfahren nach Anspruch 2, wobei die Ergebnisse, die Informationen bereitstellen, eine Verteilung eines klonalen Zelltyps bei dem Patienten aus diagnostischen Bildern einer Verteilung von mindestens einer der Immunverbindungen in vivo identifizieren.

15. Ein System, das aus einem Computer besteht, der durch Programmieren dazu konfiguriert ist, die Verarbeitung zu implementieren, um das Bestimmen, Berechnen, Erhalten, Produzieren, und Anpassen nach Anspruch 1 durchzuführen.

16. Ein Verfahren zum Adaptieren einer pharmazeutischen Zusammensetzung, die pharmazeutische Immunverbindungskomponenten beinhaltet, zur Behandlung einer Krebskrankheit bei einem Patienten, der diese benötigt, beinhaltend:

Erhalten von Ergebnissen, die Informationen über Zellzahlprävalenzen einer Vielzahl von klonalen Zelltypen bei dem Patienten bereitstellen, nachdem eine pharmazeutische Zusammensetzung verabreicht wurde;

Produzieren eines Krankheitsstatusmodells auf der Grundlage der erhaltenen Ergebnisse, wobei der Krankheitsstatus als Faktoren Bestimmungen von Zellzahlprävalenzen der Vielzahl von klonalen Zelltypen in der Krebserkrankung des Patienten umfasst;

Anpassen eines Modells, das Folgendes umfasst: (1) den Krankheitsstatus, und (2) pharmazeutische Immunverbindungskomponenten mit Behandlungswirkungen auf den Krankheitsstatus auf der Grundlage der gemessenen Aufteilung der pharmazeutischen Immunverbindungskomponenten unter den klonalen Zelltypen, wobei das Modell auf der Grundlage von Unterschieden zwischen den Krankheitsstatus des Patienten vor und nach der Verabreichung der pharmazeutischen Zusammensetzung angepasst wird;

Berechnen einer Zusammensetzung der genannten pharmazeutischen Immunverbindungskomponenten, die sich auf das angepasste Krankheitsstatusmodell auswirken, gemäß ihrer gemessenen Aufteilung, um ein Zielkrankheitsstatusmodell zu produzieren; und

Formulieren einer Behandlungszusammensetzung für den genannten Patienten auf der Grundlage der berechneten Zusammensetzung.

## Revendications

1. Procédé d'adaptation d'une composition pharmaceutique comprenant des constituants pharmaceutiques de type composés immunologiques pour le traitement d'une maladie cancéreuse chez un patient qui en a besoin, comprenant :

   la détermination d'un modèle comprenant : (1) un état pathologique présent comprenant comme facteurs des déterminations de prévalences de numérations cellulaires d'une pluralité de types de cellules clonales dans la maladie cancéreuse du patient, et (2) des constituants pharmaceutiques de type composés immunologiques ayant des effets thérapeutiques sur l'état pathologique basés sur le partitionnement mesuré des constituants pharmaceutiques de type composés immunologiques parmi les types de cellules clonales ;

   le calcul d'une composition constituée desdits constituants pharmaceutiques de type composés immunologiques agissant sur l'état pathologique présent en fonction de leur partitionnement mesuré pour produire un modèle d'état pathologique cible comprenant comme facteurs des déterminations de prévalences de numérations cellulaires de la pluralité de types de cellules clonales ;

   la formulation d'une composition de traitement pour ledit patient basée sur la composition calculée ;

   la réception de résultats apportant des informations sur les prévalences de numérations cellulaires de la pluralité de types de cellules clonales chez le patient après l'administration de la composition de traitement ;

   la production d'un nouveau modèle d'état pathologique basé sur les résultats reçus ;

   l'ajustement du modèle en fonction des différences entre le modèle d'état pathologique cible et le nouveau modèle d'état pathologique du patient ; et

   la répétition dudit calcul et de ladite formulation.

2. Procédé selon la revendication 1, dans lequel les constituants pharmaceutiques de type composés immunologiques comprennent des composés immunologiques ; et dans lequel leur partitionnement mesuré est déterminé sur la base de leurs activités de liaison associées à une liaison aux types de cellules clonales respectifs de la pluralité de types de cellules clonales.

3. Procédé selon la revendication 1, dans lequel ladite répétition comprend une répétition dudit calcul et de ladite formulation pour au moins quatre itérations de formulation et un traitement ultérieur avec la formulation.

4. Procédé selon la revendication 1, dans lequel ladite répétition comprend une répétition dudit ajustement.

5. Procédé selon la revendication 1, dans lequel ledit calcul prend en compte un compromis entre un changement dans l'état de santé du patient et un changement dans l'état pathologique causé par le traitement avec ladite composition.

6. Procédé selon la revendication 1, dans lequel ledit état pathologique présent est mémorisé comme vecteur, et dans lequel ledit calcul comprend des multiplications matrice-vecteur ou matrice-matrice sur ledit vecteur mémorisé.

7. Procédé selon la revendication 1, dans lequel ledit calcul utilise un modèle MIMO comportant de multiples entrées générant de multiples sorties interdépendantes.

8. Procédé selon la revendication 1, dans lequel ledit calcul comprend la recherche d'un espace d'état pour un état qui au moins se rapproche dudit état cible.

9. Procédé selon la revendication 1, dans lequel ledit ajustement comprend au moins l'un du groupe constitué de :

    l'ajustement d'un modèle de l'interaction entre la maladie et le traitement, et
    la modification d'une partie du modèle correspondant à l'état pathologique.

10. Procédé selon la revendication 2, comprenant une détermination de la sensibilité dudit cancer ; la détermination de la sensibilité comprenant un essai de préparations identifiées desdits composés immunologiques pour sélectionner des préparations de composés immunologiques qui se localisent sur les types de cellules clonales dans une tumeur cancéreuse dudit patient in vivo.

11. Procédé selon la revendication 2, dans lequel ladite formulation comprend la fourniture d'une composition pharmaceutique comprenant des préparations de composés immunologiques identifiées à des doses prédéterminées, dans lequel la proportion des doses des différentes préparations dans la composition est corrélée à des états vitaux relatifs desdits types de cellules clonales.

12. Procédé selon la revendication 1, le modèle comprenant un modèle contrôlé de façon adaptative dans lequel :

    ledit calcul d'une composition comprend le calcul d'un contrôle à exercer sur le modèle contrôlé de façon adaptative pour produire un état qui au moins se rapproche de l'état pathologique cible, la composition calculée étant déterminée à partir de cette composante de contrôle ;
    l'ajustement du modèle en fonction des différences entre le modèle d'état pathologique cible et le nouveau modèle d'état pathologique du patient ajuste la modélisation de l'interaction entre la maladie et le traitement ;
    l'ajustement du modèle comprend en outre un ajustement de l'état pathologique présent du modèle pour le faire correspondre au nouvel état pathologique du patient ; et
    lors de la répétition du calcul est calculée une nouvelle composition qui est différente de la composition basée sur l'ajustement du modèle.

13. Procédé selon la revendication 12, le modèle comprenant un modèle adaptatif contrôlé de façon linéaire, dans lequel le calcul d'une composition comprend le calcul d'une combinaison linéaire des effets thérapeutiques modélisés des constituants pharmaceutiques de type composés immunologiques sur l'état pathologique.

14. Procédé selon la revendication 2, dans lequel les résultats apportant des informations identifient une distribution d'un type de cellule clonale chez le patient à partir d'images diagnostiques d'une distribution d'au moins l'un des composés immunologiques in vivo.

15. Système constitué d'un ordinateur, configuré par programmation pour exécuter un traitement afin d'effectuer la détermination, le calcul, la réception, la production et l'ajustement selon la revendication 1.

16. Procédé d'adaptation d'une composition pharmaceutique comprenant des constituants pharmaceutiques de type composés immunologiques pour le traitement d'une maladie cancéreuse chez un patient qui en a besoin, comprenant :

    la réception de résultats apportant des informations sur les prévalences de numérations cellulaires d'une pluralité de types de cellules clonales chez le patient après l'administration d'une composition pharmaceutique ;
    la production d'un modèle d'état pathologique basé sur les résultats reçus, dans lequel l'état pathologique comprend comme facteurs des déterminations de prévalences de numérations cellulaires de la pluralité de types de cellules clonales dans la maladie cancéreuse du patient ;
    l'ajustement d'un modèle comprenant : (1) l'état pathologique, et (2) des constituants pharmaceutiques de type composés immunologiques ayant des effets thérapeutiques sur l'état pathologique basés sur le partitionnement mesuré des constituants pharmaceutiques de type composés immunologiques parmi les types de cellules clonales, le modèle étant ajusté en fonction des différences entre les états pathologiques du patient avant et après l'administration de la composition pharmaceutique ;
    le calcul d'une composition constituée desdits constituants pharmaceutiques de type composés immunologiques agissant sur le modèle d'état pathologique ajusté en fonction de leur partitionnement mesuré pour produire un modèle d'état pathologique cible ; et
la formulation d'une composition de traitement pour ledit patient basée sur la composition calculée.

FIG. 1A

FIG. 1B

$$\vec{x}_{n+1} = A \cdot \vec{x}_n - B \cdot \vec{u}_n$$

200

| Size, cell counts | Performance status, vitality |
|---|---|

232

236

| To be reduced by treatment | To be preserved above risk levels |
|---|---|

234

238

**A**

| $^+C_0$ Estimate cancer dynamics C | $^-P_0$ Estimate patient dynamics P |
|---|---|

212

214

204

| Assay treatments vs. somatic cells | Assay treatments vs. cancer clones | Assay treatments vs. support cells |
|---|---|---|

220

224

228

| Weaken host; impair patient dynamics P | Damage cancer; impair cancer dynamics C | Damage support; impair cancer dynamics C |
|---|---|---|

222

226

230

**B**

| $^+Z_0$ Estimate adverse effects Z | $^-T_0$ Estimate treatment T | $^-S_0$ Estimate support effects S |
|---|---|---|

206

208

210

202

FIG. 2

FIG. 3

FIG. 4B

FIG. 4A

**FIG. 5**

Treatment vs. Disease State

FIG. 6

## Treatment vs. Disease State

701

**Treatment Intensity**

712  713  716  707
705A
703  719A  717
719  705B
718

**Clone Prevelence**

705  712
703  713  716
710  718
715  717
714
707

702

**Treatment Time**

703 — – – – – Clone A/Treatment targeting Clone A
705 —————— Clone B/Treatment targeting Clone B
707 —·········· Clone C/Treatment targeting Clone C

## FIG. 7

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0222360 A
- US 6180357 B
- WO 2013011479 A2
- WO 201413569 A1

### Non-patent literature cited in the description

- **JIAN-HUA YU et al.** Individualized leukemia cell-population profiles in common B-cell acute lymphoblastic leukemia patients. *Chinese Journal of Cancer,* 05 April 2013, vol. 32 (4), 213-223
- **ANDERSON AR ; WEAVER AM ; CUMMINGS PT ; QUARANTA V.** Tumor morphology and phenotypic evolution driven by selective pressure from the microenvironment. *Cell,* 01 December 2006, vol. 127 (5), 905-15
- **GONZALEZ-GARCIA I ; SOLE RV ; COSTA J.** Metapopulation dynamics and spatial heterogeneity in cancer. *Proc Natl Acad Sci USA.,* 01 October 2002, vol. 99 (20), 13085-9
- **MALEY CC ; GALIPEAU PC ; FINLEY JC ; WONG-SURAWAT VJ ; LI X ; SANCHEZ CA et al.** Genetic clonal diversity predicts progression to esophageal adenocarcinoma. *Nat Genet.,* April 2006, vol. 38 (4), 468-73
- **SHIBATA D.** Clonal diversity in tumor progression. *Nat Genet.,* April 2006, vol. 38 (4), 402-3
- **AOKI M ; SAIKAWA Y ; HOSOKAWA S ; FUKUDA K ; KUMAI K ; KUBOTA T et al.** A novel human monoclonal antibody derived from tumor-infiltrating lymphocytes in lung cancer inhibits cancer cell growth with morphological changes. *Anticancer Res.,* November 2005, vol. 25 (6B), 3791-8
- **BRANDLEIN S ; LORENZ J ; RUOFF N ; HENSEL F ; BEYER I ; MULLER J et al.** Human monoclonal IgM antibodies with apoptotic activity isolated from cancer patients. *Hum Antibodies.,* 2002, vol. 11 (4), 107-19
- **DISIS ML ; LYERLY HK.** Global role of the immune system in identifying cancer initiation and limiting disease progression. *J Clin Oncol.,* 10 December 2005, vol. 23 (35), 8923-5
- **DRAKE CG ; JAFFEE E ; PARDOLL DM.** Mechanisms of immune evasion by tumors. *Adv Immunol.,* 2006, vol. 90, 51-81
- **DUDLEY ME ; ROSENBERG SA.** Adoptive-cell-transfer therapy for the treatment of patients with cancer*. Nat Rev Cancer,* September 2003, vol. 3 (9), 666-75

- **FINN OJ.** Cancer vaccines: between the idea and the reality. *Nat Rev Immunol.,* August 2003, vol. 3 (8), 630-41
- **IMAHAYASHI S ; ICHIYOSHI Y ; YOSHINO I ; EIFUKU R ; TAKENOYAMA M ; YASUMOTO K.** Tumor infiltrating B-cell-derived IgG recognizes tumor components in human lung cancer. *Cancer Invest.,* 2000, vol. 18 (6), 530-6
- **KOSTER et al.** Drop-based microfluidic devices for encapsulation of single cells. *Lab Chip,* 2008, vol. 8, 1110-1115
- **MARANTZ HENIG.** In business to treat cancer. *The New York Times,* 23 November 1986
- **MATSUMOTO et al.** A rapid and efficient strategy to generate antigen-specific human monoclonal antibody by in vitro immunization and the phage display method. *Journal of Immunological Methods,* 2008, vol. 332, 2-9
- **MCINTOSH.** Providing experimental therapies as a commercial venture fails. *Journal of the National Cancer Institute,* 1990, 84-87
- **RABINOVICH GA ; GABRILOVICH D ; SOTOMAYOR EM.** Immunosuppressive strategies that are mediated by tumor cells. *Annu Rev Immunol.,* 2007, vol. 25, 267-96
- **ROTHE A ; KLIMKA A ; TUR MK ; PFITZNER T ; HUHN M ; SASSE S et al.** Construction of phage display libraries from reactive lymph nodes of breast carcinoma patients and selection for specifically binding human single chain Fv on cell lines. *Int J Mol Med.,* October 2004, vol. 14 (4), 729-35
- **SCHWARTZ-ALBIEZ et al.** Natural antibodies, intravenous immunoglobulin and their role in autoimmunity, cancer and inflammation. *Clinical and Experimental Immunology,* 2009, vol. 158 (1), 43-50
- **SCHWARTZ-ALBIEZ et al.** Cytotoxic natural antibodies against human tumours: An option for anti-cancer immunotherapy?. *Autoimmunity Reviews,* 2008, vol. 7, 491-495

- **TILLER et al.** Efficient generation of monoclonal antibodies from single human B cells by single cell RT-PCR and expression vector cloning. *J Immunol Methods,* 01 January 2008, vol. 329 (1-2), 112-124
- **VOLLMERS ; BRÄNDLEIN.** Natural antibodies and cancer. *Journal of Autoimmunity,* 2007, vol. 29, 295-302
- **VOLLMERS ; BRÄNDLEIN.** Natural antibodies and cancer. *New Biotechnology,* 2009, vol. 25 (5), 294-298
- **VOLLMERS ; BRÄNDLEIN.** Natural Monoclonal Antibodies and Cancer. *Recent Patents on Anti-Cancer Drug Discovery,* 2008, vol. 3, 84-87
- **VOLLMERS ; BRÄNDLEIN.** Nature's best weapons to fight cancer. Revival of human monoclonal IgM antibodies. *Human Antibodies,* 2002, vol. 11, 131-142